# EUROPEAN PATENT SPECIFICATION

(11) **EP 4 048 807 B1**
(45) Date of publication and mention of the grant of the patent: **15.07.2026**
(21) Application number: 20786662.5
(22) Date of filing: 23.09.2020
(51) Int. Cl.: C12N 15/113

(54) **COMPOSITIONS AND METHODS FOR MODULATING APOLIPOPROTEIN B (APOB) GENE EXPRESSION**
ZUSAMMENSETZUNGEN UND VERFAHREN ZUR MODULATION VON APOLIPOPROTEIN-B(APOB)-GENEXPRESSION
COMPOSITIONS ET PROCÉDÉS POUR MODULER L'EXPRESSION GÉNIQUE DE L'APOLIPOPROTÉINE B (APOB)

(30) Priority: 23.09.2019 US 201962904325 P
(43) Date of publication of application: 31.08.2022
(73) Proprietor: Omega Therapeutics, Inc., Cambridge, MA 02140 (US)
(72) Inventor: PATIL, Vishwesh Ashok, Arlington, MA 02474 (US); FARELLI, Jeremiah D., Marblehead, MA 01945 (US); KARNIK, Rahul, Cambridge, MA 02138 (US); SMITH, Jesse Jerome, Waltham, MA 02453 (US); SARISOZEN, Can, Westford, MA 01886 (US); SCHEIDEGGER, Adam Walter, Somerville, MA 02145 (US); BENNETT, Barbara, Wakefield, MA 01880 (US)
(74) Representative: Hoffmann Eitle
(86) International application number: PCT/US2020/052119
(87) International publication number: WO 2021/061707

(56) References cited:
- WO-A1-2006/053430
- WO-A1-2014/204726
- WO-A1-2018/049079
- CATHERINE MCLAUGHLIN ET AL: "Development of novel therapeutic approaches for the reduction of apolipoprotein B expression", 1 January 2014 (2014-01-01), XP055755300, Retrieved from the Internet <URL:https://www.researchgate.net/profile/Catherine_Mclaughlin/publication/278524013_Development_of_novel_therapeutic_approaches_for_the_reduction_of_apolipoprotein_B_expression/links/5581468908ae607ddc3243e3/Development-of-novel-therapeutic-approaches-for-the-reduction-of-apolipoprotein-B-expression.pdf> DOI: 10.13140/rg.2.1.3104.7840
- KELSEY E. JARRETT ET AL: "Somatic genome editing with CRISPR/Cas9 generates and corrects a metabolic disease", SCIENTIFIC REPORTS, vol. 7, no. 1, 16 March 2017 (2017-03-16), pages 1 - 12, XP055566917, DOI: 10.1038/srep44624

## Description

### Background of the Invention

Hyperlipidemias are abnormally elevated levels of any or all lipids or lipoproteins in the blood. One hyperlipidemia, hypercholesterolemia, also referred to as high cholesterol, is the presence of high levels of cholesterol in the blood. Hyperlipidemias, such as hypercholesterolemia may be a consequence of diet, obesity, inherited (genetic) diseases (such as LDL receptor mutations in familial hypercholesterolemia), or the presence of other diseases, such as type 2 diabetes and an underactive thyroid.

Apolipoprotein B (ApoB) is the primary apolipoprotein of chylomicrons, VLDL, IDL, and LDL particles, which are responsible for transporting lipids, including cholesterol, around the body within lipid particles to all cells within all tissues. It is the primary organizing protein component of the particles and is important for the formation of these particles. In particular, ApoB on LDL particle acts as a ligand for LDL receptors in various cells throughout the body. Overexpression studies of ApoB suggest that elevated levels of ApoB may cause hypobetalipoproteinemia, normotriglyceridemic hypobetalipoproteinemia, and hypercholesterolemia, diseases affecting plasma cholesterol.

While hypercholesterolemia itself is asymptomatic, longstanding elevation of serum cholesterol can lead to atherosclerosis which can, over time, lead to progressive stenosis or even complete occlusion of the involved arteries. In addition, smaller plaques may rupture and cause a clot to form and obstruct blood flow resulting in, for example, myocardial infarction and/or stroke.

Avoiding trans-fats and replacing saturated fats with polyunsaturated fats are recommended dietary measures to reduce total blood cholesterol and LDL in adults. In subjects with very high cholesterol (e.g., hypercholesterolemia), however, diet modifications are often not sufficient to achieve the desired lowering of LDL, and lipid-lowering medications are usually required. However, many subjects have adverse reactions to lipid lowering medications.

Accordingly, there is a need in the art for compositions and methods that treat an ApoB associated disease, such as hypercholesterolemia.

PCT publication No. WO 2006/053430 relates to siRNA silencing of ApoB. PCT publication No. WO 2014/204726 relates to delivery and use of the CRISPR-Cas systems, vectors and compositions for hepatic targeting and therapy. PCT publication No. WO 2018/049079 relates to methods and compositions for modulating gene expression.

### Summary of the Invention

The present invention provides agents and compositions for modulating the expression (*e.g.*, enhancing or reducing expression) of Apolipoprotein B (APOB ) gene by targeting an APOB expression control region. The APOB gene may be in a cell, *e.g.,* a mammalian cell, such as a mammalian somatic cell, *e.g.,* a mouse or human somatic cell. The present disclosure also provides methods of using the agents and compositions of the invention for modulating the expression of an APOB gene or APOB disrupting agents for use in treating a subject who would benefit from modulating the expression of an APOB gene, *e.g.,* a subject suffering or prone to suffering from an APOB-associated disease.

Accordingly, in one aspect, the present invention provides a site-specific apoliporpotein B (APOB) disrupting agent. The disrupting agent comprises a site-specific APOB targeting moiety which targets an APOB expression control region. In one embodiment, the site-specific APOB targeting moiety comprises a polymeric molecule. In another embodiment, the polymeric molecule comprises a polyamide. In still another embodiment, the polymeric molecule comprises a polynucleotide.

In one embodiment, the expression control region comprises an APOB-associated anchor sequence. In another embodiment, the APOB-associated anchor sequence comprises a CCCTC-binding factor (CTCF) binding motif. In still another embodiment, the APOB-associated CTCF binding motif comprises the nucleotide sequence of APOB CTCF site 3.

In another embodiment, the expression control region comprises an APOB-associated anchor sequence-mediated conjunction. In another embodiment, the APOB-associated anchor sequence-mediated conjunction comprises one or more transcriptional control elements internal to the conjunction. In still another embodiment, the APOB-associated anchor sequence-mediated conjunction comprises one or more transcriptional control elements external to the conjunction.

In still another embodiment, the APOB-associated anchor sequence is located within about 300 kb of the transcriptional control element. In another embodiment, the anchor sequence is located within 10 kb of the transcriptional control element.

In yet another embodiment, the expression control region comprises an APOB-specific transcriptional control element. In another embodiment, the transcriptional control element comprises an APOB promoter. In still another embodiment, the transcriptional control element comprises a transcriptional enhancer. In yet another embodiment, the transcriptional control element comprises a transcriptional repressor.

In one embodiment, the site-specific APOB disrupting agent comprises a nucleotide sequence having at least 85% nucleotide identity to the entire nucleotide sequence of any of the nucleotide sequences in Table 2.

In another embodiment the site-specific APOB disrupting agent comprises a polynucleotide encoding a DNA-binding domain, or fragment thereof, of a zinc finger polypeptide (ZNF) or a transcription activator-like effector (TALE) polypeptide that specifically binds to the APOB expression control region.

In still another embodiment, the site-specific APOB disrupting agent comprises a nucleotide modification.

In yet another embodiment, the site-specific APOB disrupting agent comprises a peptide nucleic acid (PNA).

In one aspect, the present invention provides a vector comprises the site-specific APOB disrupting agent of any aspect and various embodiments. In one embodiment, the vector is a viral expression vector.

In another aspect, the present invention provides a cell comprises the site-specific APOB disrupting agent or the vactor of any aspect and various embodiments.

In one embodiment, the site-specific APOB disrupting agent is present in a composition. In another embodiment, the composition comprises a pharmaceutical composition. In still another embodiment, the pharmaceutical composition comprises a lipid formulation. In yet another embodiment, the lipid formulation comprises one or more cationic lipids, one or more non-cationic lipids, one or more cholesterol-based lipids, or one or more PEG-modified lipids, or combinations of any of the foregoing. In one embodiment, the pharmaceutical composition comprises a lipid nanoparticle.

In one aspect, the present invention provides a site-specific APOB disrupting agent. The disrupting agent comprises a nucleic acid molecule encoding a fusion protein, the fusion protein comprising a site-specific APOB targeting moiety which targets an APOB expression control region and an effector molecule. In one embodiment, the site-specific APOB targeting moiety comprises a polynucleotide encoding a DNA-binding domain, or fragment thereof, of a zinc finger polypeptide (ZNF) or a transcription activator-like effector (TALE) polypeptide that specifically binds to the APOB expression control region. In another embodiment, the effector molecule comprises a nucleic acid molecule encoding a polypeptide. In still another embodiment the fusion protein comprises a peptide-nucleic acid fusion.

In one embodiment, the effector is selected from the group consisting of a nuclease, a physical blocker, an epigenetic recruiter, and an epigenetic CpG modifier, and combinations of any of the foregoing. In another embodiment, the effector comprises a CRISPR associated protein (Cas) polypeptide or nucleic acid molecule encoding the Cas polypeptide. In still another embodiment, the Cas polypeptide is an enzymatically inactive Cas polypeptide. In yet another embodiment, the site-specific APOB disrupting agent further comprises a catalytically active domain of human exonuclease 1 (hEXO1). In one embodiment, the epigenetic recruiter comprises a transcriptional enhancer or a transcriptional repressor. In another embodiment, wherein the epigenetic CpG modifier comprises a DNA methylase, a DNA demethylase, a histone modifying agent, or a histone deacetylase.

In one embodiment, the effector molecule comprises a zinc finger polypeptide. In another embodiment, the effector molecule comprises a transcription activator-like effector nuclease (TALEN) polypeptide.

In one aspect, the present invention provides a vector. The vector comprises a nucleic acid molecule encoding the site-specific APOB disrupting agent of any of the aspects and various embodiments of the invention. In one embodiment, the vector is a viral expression vector.

In another aspect, the present invention provides a cell, the cell comprises the site-specific APOB disrupting agent or the vector of any aspects and various embodiment of the invention.

In one embodiment, the site-specific APOB disrupting agent is present in a composition. In another embodiment, the composition comprises a pharmaceutical composition. In still another embodiment, the pharmaceutical composition comprises a lipid formulation. In yet another embodiment, the lipid formulation comprises one or more cationic lipids, one or more non-cationic lipids, one or more cholesterol-based lipids, or one or more PEG-modified lipids, or combinations of any of the foregoing. In one embodiment, the pharmaceutical composition comprises a lipid nanoparticle.

In one aspect, the present disclosure provides a method of modulating expression of Apolipoprotein B (APOB ) in a cell. The method comprises contacting the cell with a site-specific APOB disrupting agent, the disrupting agent comprising a site-specific APOB targeting moiety which targets an APOB expression control region, and an effector molecule, thereby modulating expression of APOB in the cell. In one embodiment, the modulation of expression is enhanced expression of APOB in the cell. In another embodiment, the modulation of expression is reduced expression of APOB in the cell. In still another embodiment, the site-specific APOB targeting moiety comprises a polymeric molecule. In yet another embodiment, the polymeric molecule comprises a polyamide. In one embodiment, the polymeric molecule comprises a polynucleotide.

In one embodiment, the expression control region comprises an APOB-associated anchor sequence. In another embodiment, the APOB-associated anchor sequence comprises a CCCTC-binding factor (CTCF) binding motif. In still another embodment, the APOB-associated CTCF binding motif comprises the nucleotide sequence of APOB CTCF site 3.

In one embodiment, the expression control region comprises an APOB-associated anchor sequence-mediated conjunction. In another embodiment, the APOB-associated anchor sequence-mediated conjunction comprises one or more transcriptional control elements internal to the conjunction. In still another embodiment, the APOB-associated anchor sequence-mediated conjunction comprises one or more transcriptional control elements external to the conjunction.

In one embodiment, the anchor sequence is located within about 300 kb of the transcriptional control element. In another embodiment, the anchor sequence is located within 10 kb of the transcriptional control element.

In another embodiment, the expression control region comprises an APOB-specific transcriptional element. In one embodiment, the transcriptional element comprises an APOB promoter. In another embodiment, the transcriptional control element comprises transcriptional enhancer. In still another embodiment, the transcriptional control element comprises a transcriptional repressor.

In still another embodiment, the site-specific APOB disrupting agent comprises a nucleotide sequence having at least 85% nucleotide identity to the entire nucleotide sequence of any of the nucleotide sequences in Table 2.

In yet another embodiment, the site-specific APOB disrupting agent comprises a polynucleotide encoding a DNA-binding domain, or fragment thereof, of a zinc finger polypeptide (ZNF) or a transcription activator-like effector (TALE) polypeptide that specifically binds to the APOB expression control region.

In one embodiment, the site-specific APOB disrupting agent comprises a nucleotide modification.

In another embodiment, the polymeric molecule comprises a peptide nucleic acid (PNA).

In still another embodiment, the effector molecule comprises a polypeptide. In one embodiment, the polypeptide comprises a fusion protein comprising the site-specific APOB targeting moiety which targets an APOB expression regulatory region, and the effector molecule. In another embodiment, the fusion protein comprises a peptide-nucleic acid fusion molecule.

In yet another embodiment, the effector is selected from the group consisting of a nuclease, a physical blocker, an epigenetic recruiter, and an epigenetic CpG modifier, and combinations of any of the foregoing. In one embodiment, the effector comprises a CRISPR associated protein (Cas) polypeptide or nucleic acid molecule encoding the Cas polypeptide. In another embodiment, the Cas polypeptide is an enzymatically inactive Cas polypeptide. In still another embodiment, the effector molecule further comprises a catalytically active domain of human exonuclease 1 (hEXO1). In yet another embodiment, the epigenetic recruiter comprises a transcriptional enhancer or a transcriptional repressor. In one embodiment, the epigenetic CpG modifier comprises a DNA methylase, a DNA demethylase, a histone modifying agent, or a histone deacetylase.

In one embodiment, the effector molecule comprises a zinc finger polypeptide. In another embodiment, the effector molecule comprises a Transcription activator-like effector nuclease (TALEN) polypeptide.

In another embodiment, the fusion protein comprises an enzymatically inactive Cas polypeptide and an epigenetic recruiter polypeptide. In one embodiment, the fusion protein comprises an enzymatically Cas polypeptide and an epigenetic CpG modifier polypeptide.

In one embodiment, the site-specific disrupting agent, the effector, or both the site-specific disrupting agent and the effector are present in a vector. In another embodiment, the site-specific disrupting agent and the effector are present in the same vector. In still another embodiment, the site-specific disrupting agent and the effector are present in different vectors. In yet another embodiment, the vector is a viral expression vector.

In another embodiment, the site-specific disrupting agent, the effector, or both the site-specific disrupting agent and the effector are present in a composition. In one embodiment, the site-specific disrupting agent and the effector are present in the same composition. In another embodiment, the site-specific disrupting agent and the effector are present in different compositions. In still another embodiment, the composition comprises a pharmaceutical composition. In yet another embodiment, the pharmaceutical composition comprises a lipid formulation. In one embodiment, the lipid formulation comprises one or more cationic lipids, one or more non-cationic lipids, one or more cholesterol-based lipids, or one or more PEG-modified lipids, or combinations of any of the foregoing. In another embodiment, the pharmaceutical composition comprises a lipid nanoparticle.

In one aspect, the cell is a mammalian cell. In one embodiment, the mammalian cell is a somatic cell. In another embodiment, the mammalian cell is a primary cell.

In one embodiment, the contacting is performed *in vitro.* In another embodiment, the contacting is performed *in vivo.* In still another embodiment, the contacting is performed *ex vivo.* In yet another embodiment, the method further comprises administering the cell to a subject. In one embodiment, the cell is within a subject. In another embodiment, the subject has an APOB-associated disease. In still another embodiment, the APOB-associated disease is selected from the group consisting of a hyperlipidemia, a hypercholesterolemia, high LDL cholesterol, low HDL cholesterol, hypertriglyceridemia, postprandial hypertriglyceridemia, insulin resistance not related to an immune response to insulin, type 2 diabetes, hypertension, endothelial cell dysfunction, heart disease, and atherosclerosis.

In one aspect, the present invention provide APOB disrupting agents for use in a method for treating a subject having an APOB-associated disease, comprising administering to the subject a therapeutically effective amount of the site-specific APOB disrupting agent, the disrupting agent comprising a site-specific APOB targeting moiety which targets an APOB expression control region, and an effector molecule, thereby treating the subject. In one embodiment, the APOB-associated disease is hypercholesterolemia and the site-specific APOB disrupting agent reduces expression of APOB in the subject. In another embodiment, the site-specific APOB disrupting agent and the effector molecule are administered to the subject concurrently. In still another embodiment, the site-specific APOB disrupting agent and the effector molecule are administered to the subject sequentially. In yet another embodiment, the effector molecule is administered to the subject prior to administration of the site-specific APOB disrupting agent. In one embodiment, the site-specific APOB disrupting agent is administered to the subject prior to administration of the effector molecule.

The present invention is defined in the appended claims.

### Brief Description of the Drawings

Figure 1 depicts an APOB genome neighborhood showing a portion of the upstream region and coding region of an APOB gene and the positions of various regulatory elements of an APOB gene, *e.g.,* CTCF site 3.
Figure 2 schematically depicts the location of CTCF site 3 of an APOB gene and the relative positions of the guide RNAs described herein.
Figure 3A is a graph depicting the percentage of editing in PMH cells 72 hours after contacting the cells with the indicated APOB disrupting agents comprising a site-specific targeting moieties and an effector molecule comprising Cas9 at the indicated concentrations as measured by T7E1 assay.
Figure 3B is an image of an electrophoresis gel depicting the results of the T7E1 assay described in Figure 3A. A negative result, *i.e*., no DNA band on the gel, indicates that the genomic DNA of the corresponding cells was disrupted by Cas9.
Figure 3C is a graph depicting the percentage of productive editing in PMH cells 72 hours after contacting the cells with the indicated APOB disrupting agents comprising a site-specific targeting moieties (*i.e.,* sgRNA) and an effector molecule comprising Cas9 at the indicated concentrations . "Productive editing" refers to the Cas9-induced genome editing that resulted in a functional change in the ApoB expression control region, *e.g.,* loss of the ability of CTCF3 site to form DNA topological configuration, *e.g.*, a loop.
Figure 3D is a graph depicting the productive editing ratio in PMH cells 72 hours after contacting the cells with the indicated APOB disrupting agents comprising a site-specific targeting moieties and an effector molecule comprising Cas9 at indicated concentrations.

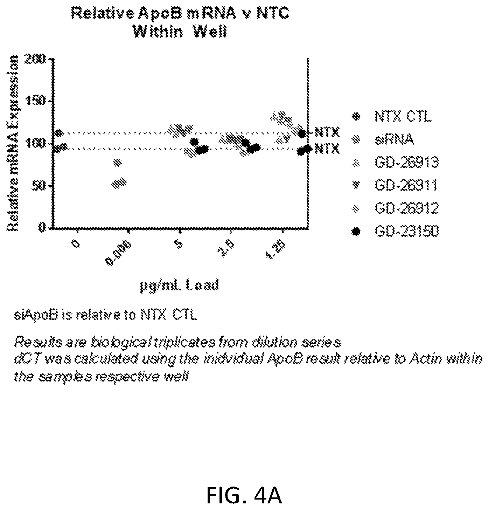

Figures 4A-4D are graphs demonstrating that the site-specific APOB targeting moieties (*i.e.*, sgRNA) in combination with Cas9 coding mRNA reduced APOB mRNA levels. Two sgRNAs, GD-26911 and GD-26912,reduced ApoB expression more than 50% at a concentration of 5µg/ml of the combination of sgRNA and Cas9 coding mRNA. The graphs show APOB mRNA level at 72 hours after contacting the cells wit htheindicated sgRNA and an effector comprising Cas9. Results are from experimental triplicates. The level of siRNA targeting APOB gene (siApoB) is relative to NTX control. The ApoB mRNA levels in sgRNA treatment groups are graphed versus the non-targeting guide RNA formulation (GD-23150 group) (non-target control). NTX CTL indicates the no-treatment group and ApoB levels in the NTX CTL indicate the baseline PMH. GD-23150 group indicates the PMH treated with LNPs that contain non-targeting sgRNA. siRNA group indicates the group that was treated with ApoB siRNA containing LNPs. Relative ApoB levels were graphed against the total RNA concentrations (*i.e*., the combination of sgRNA and Cas9 coding mRNA) in the LNP treatments. NTX CTL: no treatment control; NTC: non-target control.

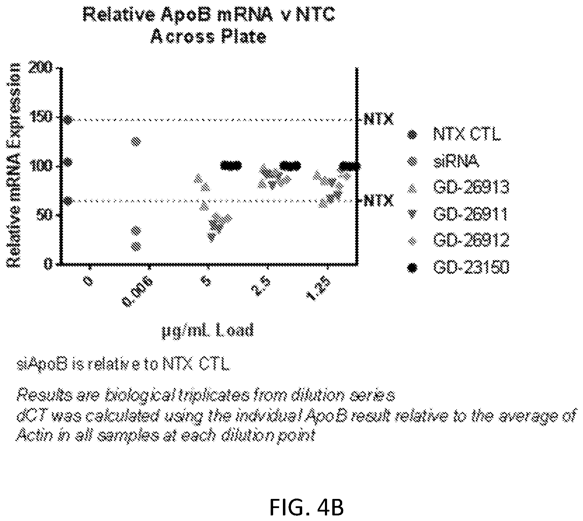

Figures 4A and 4B depict relative APOB mRNA levels as compared to NTC. Figure 4A is a graph depicting within well results. Delta cycle threshold (dCT) values were calculated using the individual APOB results relative to actin within the sample's respective well. Figure 4B is a graph depicting across plate results. dCT values werecalculated using the individual APOB results relative to the average of actin in all samples at each dilution point.

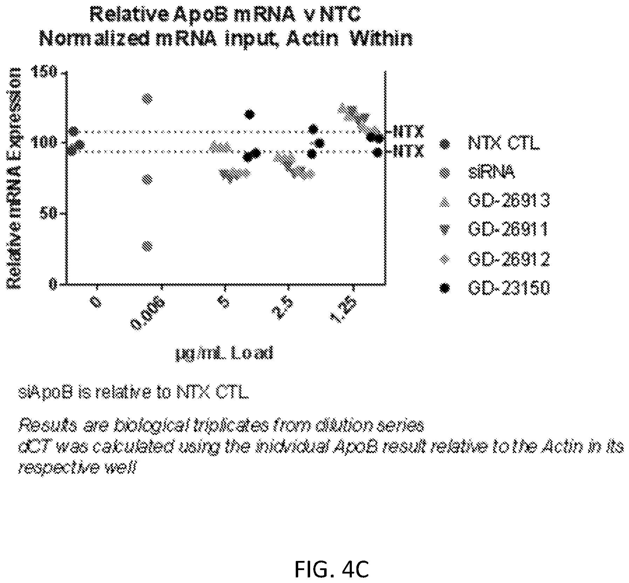

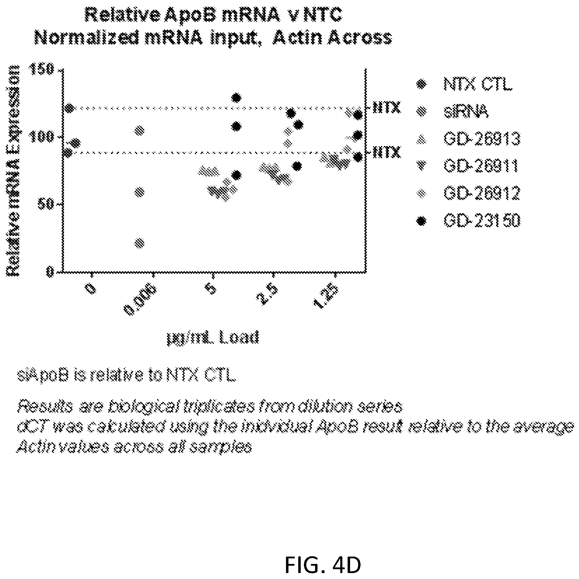

Figures 4C and 4D depict relative APOB mRNA levels as compared to NTC with normalized mRNA inputs. The same amount of total RNA across the groups from the PMH samples were used in the qPCR analysis. Figure 4C is a graph depicting within well results. dCT value was calculated using the individual APOB results relative to actin within the sample's respective well. Figure 4D is a graph depicting across plate results. dCT values were calculated using the individual APOB results relative to the average of actin in all samples.

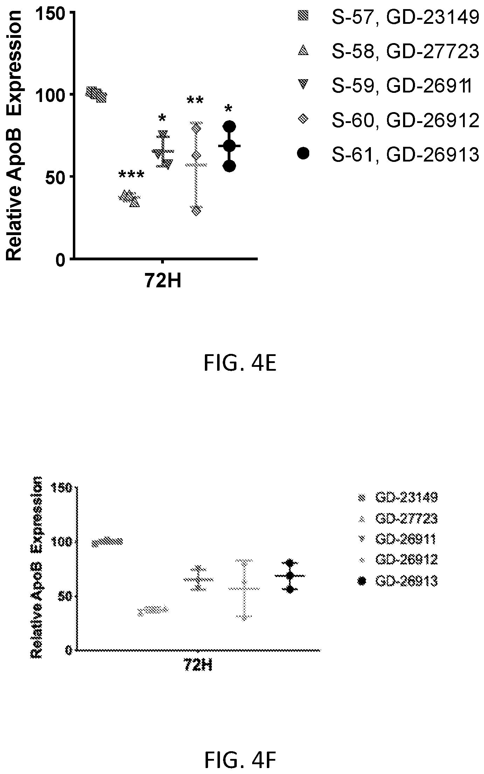

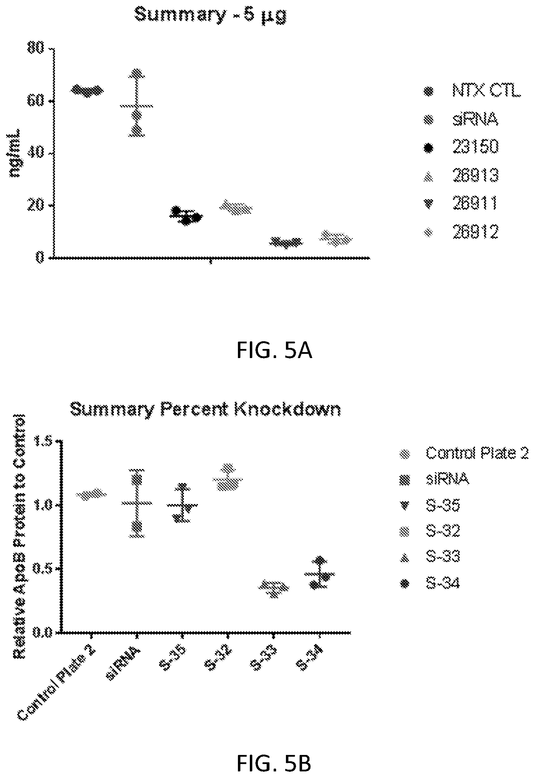

Figures 4E and 4F depict APOB mRNA level in cells transfected with the indicated sgRNA in combination with mRNA coding Cas9. In Figures 4E and 4F, 5 µg/ml of total RNA, *i.e.*, the combination of sgRNA and Cas9 coding mRNA, were transfected into the cells. In addition, a sgRNA, GD-27723, which targets an exon of the APOB gene, was used as a positive control, while a sgRNA, GD-23149, which targets an unrelated gene was used as a negative control.
Figures 5A and 5B are graphs depicting the expression level of ApoB protein. Figure 5A shows the protein concentration of ApoB in the supernatant of the cultured cells transfected with the indicated sgRNA in combination with Cas9 coding mRNA. Figure 5B shows the percentage of reduction in protein levels induced by the indicated sgRNA in combination with Cas9 coding mRNA.

### Detailed Description of the Invention

The present invention provides agents and compositions for modulating expression (e.g., enhanced or reduced expression) of an Apolipoprotein B (APOB ) gene by targeting an APOB expression control region. The APOB gene may be in a cell, *e.g.,* a mammalian cell, such as a mammalian somatic cell, *e.g.,* a mouse or human somatic cell. The present disclosure also provides methods of using the agents and compositions of the invention for modulating the expression of an APOB gene or APOB disrupting agents for use in treating a subject who would benefit from modulating the expression of an APOB gene, *e.g.,* a subject suffering or prone to suffering from an APOB-associated disease.

The agents of the invention are referenced to herein as site-specific APOB disrupting agents and are described in Section II, below.

### I. Definitions

In order that the present invention may be more readily understood, certain terms are first defined. In addition, it should be noted that whenever a value or range of values of a parameter are recited, it is intended that values and ranges intermediate to the recited values are also intended to be part of this invention.

The articles "a" and "an" are used herein to refer to one or to more than one (*i.e*., to at least one) of the grammatical object of the article. By way of example, "an element" means one element or more than one element, *e.g*., a plurality of elements.

The term "including" is used herein to mean, and is used interchangeably with, the phrase "including but not limited to". The term "or" is used herein to mean, and is used interchangeably with, the term "and/or," unless context clearly indicates otherwise.

The term "about" is used herein to mean within the typical ranges of tolerances in the art. For example, "about" can be understood as about 2 standard deviations from the mean. In certain embodiments, about means ±10%. In certain embodiments, about means ±5%. When about is present before a series of numbers or a range, it is understood that "about" can modify each of the numbers in the series or range.

The term "at least" prior to a number or series of numbers is understood to include the number adjacent to the term "at least", and all subsequent numbers or integers that could logically be included, as clear from context. For example, the number of nucleotides in a nucleic acid molecule must be an integer. For example, "at least 18 nucleotides of a 21 nucleotide nucleic acid molecule" means that 18, 19, 20, or 21 nucleotides have the indicated property. When at least is present before a series of numbers or a range, it is understood that "at least" can modify each of the numbers in the series or range.

As used herein, "no more than" or "less than" is understood as the value adjacent to the phrase and logical lower values or integers, as logical from context, to zero. When "no more than" is present before a series of numbers or a range, it is understood that "no more than" can modify each of the numbers in the series or range.

As used herein, the term "substantially" refers to the qualitative condition of exhibiting total or near-total extent or degree of a characteristic or property of interest. One of ordinary skill in the art will understand that biological and chemical phenomena rarely, if ever, go to completion and/or proceed to completeness or achieve or avoid an absolute result. The term "substantially" may therefore be used in some embodiments herein to capture potential lack of completeness inherent in many biological and chemical phenomena.

As used herein, the term "apolipoprotein B" or "APOB " refers to the gene that encodes the well known apolipoprotein of chylomicrons, VLDL, IDL, and LDL particles. The encoded protein is the primary organizing protein component of the particles and is important for the formation of these particles. APOB on the LDL particle also acts as a ligand for LDL receptors in various cells throughout the body. High levels of APOB are related to heart disease. Hypobetalipoproteinemia is a genetic disorder that can be caused by a mutation in the APOB gene, APOB. Mutations in gene APOB 100 can also cause familial hypercholesterolemia, a hereditary (autosomal dominant) form of metabolic disorder hypercholesterolemia. Overproduction of apolipoprotein B can result in lipid-induced endoplasmic reticulum stress and insulin resistance in the liver. The nucleotide and amino acid sequence of APOB is known and may be found in, for example, GenBank Accession Nos. NM_009693, XM_001000646, XM_0010000659, XM_001000667, XM_137955, XM_894981, XM-906759, NP_033823.2, XP_001000646, XP_001000659, XP_001000667, XP_137955, XP_900074, XP_911852, NM_000384.3, NP_000375.3, the entire contents of each of which are incorported herein by reference. The nucleotide sequence of the genomic region of Chromosome 2 in human, or chromosome 12 in mouse, which includes the endogenous promoters of APOB and the APOB coding sequence is also known and may be found in: Mouse - mm10 genome build: chr12:7968110-8023150, Human - hg19 genome build: chr2:21160333-21330910.

The term "site-specific APOB disrupting agent," as used herein, refers to any agent that specifically binds to a target APOB expression control region and, *e.g*., modulates expression of an APOB gene. Site-specific APOB disruption agents of the invention may comprise a "site-specific APOB targeting moiety."

As used herein, the term "site-specific APOB targeting moiety" refers to a moiety that specifically binds to an APOB expression control region, *e.g.,* a transcriptional control region of an APOB gene, such as a promoter, an enhancer, or a repressor; or an APOB-associated anchor sequence, such as, for example within an APOB-associated anchor sequence-mediated conjunction. Exemplary "site-specific APOB targeting moieties" include, but are not limited to, polyamides, nucleic acid molecules, such as RNA, DNA, or modified RNA or DNA, polypeptides, protein nucleic acid molecules, and fusion proteins.

As used herein, the terms "specific binding" or "specifically binds" refer to an ability to discriminate between possible binding partners in the environment in which binding is to occur. In some embodiments, a disrupting agent that interacts, *e.g*., preferentially interacts, with one particular target when other potential disrupting agents are present is said to "bind specifically" to the target (*i.e.*, the expression control region) with which it interacts. In some embodiments, specific binding is assessed by detecting or determining the degree of association between the disrupting agent and its target; in some embodiments, specific binding is assessed by detecting or determining degree of dissociation of a disrupting agent-target complex. In some embodiments, specific binding is assessed by detecting or determining ability of the disrupting agent to compete with an alternative interaction between its target and another entity. In some embodiments, specific binding is assessed by performing such detections or determinations across a range of concentrations.

As used herein, the term "expression control region" or expression control domain' refers to a region or domain present in a genomic DNA that modulates the expression of a target gene in a cell. A functionality associated with an expression control region may directly affect expression of a target gene, *e.g*., by recruiting or blocking recruitment of a transcription factor that would stimulate expression of the gene. A functionality associated with an expression control region may indirectly affect expression of a target gene, *e.g*., by introducing epigenetic modifications or recruiting other factors that introduce epigenetic modifications that induce a change in chromosomal topology that modulates expression of a target gene. Expression control regions may be upstream and/or downstream of the protein coding sequence of a gene and include, for example, transcriptional control elements, *e.g*., promoters, ehnacers, or repressors; and anchor sequences, and anchor sequence-mediated conjunctions.

The term "transcriptional control element," as used herein, refers to a nucleic acid sequence that controls transcription of a gene. Transcriptional control elements include, for example, anchor sequences, anchor sequence-mediated conjunctions, promoters, transcriptional enhancers, and transcriptional repressors.

A promoter is a region of DNA recognized by an RNA polymerase to initiate transcription of a particular gene and is generally located upstream of the 5'-end of the transcription start site of the gene.

A "transcriptional enhancer" increases gene transcription. A "transcriptional silencer" or "transcriptional repressor" decreases gene transcription. Enhancing and silencing sequences may be about 50-3500 base pairs in length and may influence gene transcription up to about 1 megabases away.

The term "gene," as used herein, refers to a sequence of nucleotides that encode a molecule, such as a protein, that has a function. A gene contains sequences that are transcribed (*e.g*., a 3'UTR), sequences that are not transcribed (*e.g*., a promoter), sequences that are translated (*e.g*., an exon), and sequences that are not translated (*e.g*., intron).

As used herein, the term "target gene" means an APOB gene that is targeted for modulation, *e.g*., increase or decrease, of expression. In some embodiments, an APOB target gene is part of a targeted genomic complex (*e.g*. an APOB gene that has at least part of its genomic sequence as part of a target genomic complex, *e.g*. inside an anchor sequence-mediated conjunction), which genomic complex is targeted by one or more site-specific disrupting agents as described herein. In some embodiments, modulation comprises inhibition of expression of the target gene. In some embodiments, an APOB gene is modulated by contacting the APOB gene or a transcription control element operably linked to the APOB gene with one or more site-specific disrupting agents as described herein. In some embodiments, an APOB gene is aberrantly expressed (*e.g*., over-expressed) in a cell, *e.g.,* a cell in a subject (*e.g*., a subject having an APOB-associated disease). In some embodiments, an APOB gene is aberrantly expressed (*e.g*., under-expressed) in a cell, *e.g.,* a cell in a subject (*e.g*., a subject having an APOB-associated disease).

The term "anchor sequence" as used herein, refers to a nucleic acid sequence recognized by a nucleating agent that binds sufficiently to form an anchor sequence-mediated conjunction, *e.g*., a complex. In some embodiments, an anchor sequence comprises one or more CTCF binding motifs. In some embodiments, an anchor sequence is not located within a gene coding region. In some embodiments, an anchor sequence is located within an intergenic region. In some embodiments, an anchor sequence is not located within either of an enhancer or a promoter. In some embodiments, an anchor sequence is located at least 400 bp, at least 450 bp, at least 500 bp, at least 550 bp, at least 600 bp, at least 650 bp, at least 700 bp, at least 750 bp, at least 800 bp, at least 850 bp, at least 900 bp, at least 950 bp, or at least 1kb away from any transcription start site. In some embodiments, an anchor sequence is located within a region that is not associated with genomic imprinting, monoallelic expression, and/or monoallelic epigenetic marks. In some embodiments, the anchor sequence has one or more functions selected from binding an endogenous nucleating polypeptide (*e.g*., CTCF), interacting with a second anchor sequence to form an anchor sequence mediated conjunction, or insulating against an enhancer that is outside the anchor sequence mediated conjunction. In some embodiments of the present invention, technologies are provided that may specifically target a particular anchor sequence or anchor sequences, without targeting other anchor sequences (*e.g.*, sequences that may contain a nucleating agent (*e.g*., CTCF) binding motif in a different context); such targeted anchor sequences may be referred to as the "target anchor sequence". In some embodiments, sequence and/or activity of a target anchor sequence is modulated while sequence and/or activity of one or more other anchor sequences that may be present in the same system (*e.g*., in the same cell and/or in some embodiments on the same nucleic acid molecule, *e.g*., the same chromosome) as the other targeted anchor sequence is not modulated. In some embodiments, the anchor sequence comprises or is a nucleating polypeptide binding motif. In some embodiments, the anchor sequence is adjacent to a nucleating polypeptide binding motif.

The term "anchor sequence-mediated conjunction" as used herein, refers to a DNA structure, in some cases, a complex, that occurs and/or is maintained *via* physical interaction or binding of at least two anchor sequences in the DNA by one or more polypeptides, such as nucleating polypeptides, or one or more proteins and/or a nucleic acid entity (such as RNA or DNA), that bind the anchor sequences to enable spatial proximity and functional linkage between the anchor sequences.

As used herein, the term "genomic complex" is a complex that brings together two genomic sequence elements that are spaced apart from one another on one or more chromosomes, via interactions between and among a plurality of protein and/or other components (potentially including, the genomic sequence elements). In some embodiments, the genomic sequence elements are anchor sequences to which one or more protein components of the complex bind. In some embodiments, a genomic complex may comprise an anchor sequence-mediated conjunction. In some embodiments, a genomic sequence element may be or comprise a CTCF binding motif, a promoter and/or an enhancer. In some embodiments, a genomic sequence element includes at least one or both of a promoter and/or regulatory region (*e.g*., an enhancer). In some embodiments, complex formation is nucleated at the genomic sequence element(s) and/or by binding of one or more of the protein component(s) to the genomic sequence element(s). As will be understood by those skilled in the art, in some embodiments, co-localization (*e.g*., conjunction) of the genomic sites *via* formation of the complex alters DNA topology at or near the genomic sequence element(s), including, in some embodiments, between them. In some embodiments, a genomic complex comprises an anchor sequence-mediated conjunction, which comprises one or more loops. In some embodiments, a genomic complex as described herein is nucleated by a nucleating polypeptide such as, for example, CTCF and/or Cohesin. In some embodiments, a genomic complex as described herein may include, for example, one or more of CTCF, Cohesin, non-coding RNA (*e.g*., eRNA), transcriptional machinery proteins (*e.g*., RNA polymerase, one or more transcription factors, for example selected from the group consisting of TFIIA, TFIIB, TFIID, TFIIE, TFIIF, TFIIH, *etc.*), transcriptional regulators (*e.g*., Mediator, P300, enhancer-binding proteins, repressor-binding proteins, histone modifiers, *etc*.), *etc.* In some embodiments, a genomic complex as described herein includes one or more polypeptide components and/or one or more nucleic acid components (*e.g*., one or more RNA components), which may, in some embodiments, be interacting with one another and/or with one or more genomic sequence elements (*e.g*., anchor sequences, promoter sequences, regulatory sequences (*e.g*., enhancer sequences)) so as to constrain a stretch of genomic DNA into a topological configuration (*e.g.*, a loop) that the stretch of genomic DNA does not adopt when the complex is not formed.

An "effector molecule," as used herein, refers to a molecule that is able to regulate a biological activity, such as enzymatic activity, gene expression, anchor sequence-mediated conjunction or cell signaling. Exemplary effectors are described in Section II, below, and in some embodiment include, for example, nucleases, physical blockers, epigenetic recruiters, *e.g*., a transcriptional enhancer or a transcriptional repressor, and epigenetic CpG modifiers, *e.g*., a DNA methylase, a DNA demethylase, a histone modifying agent, or a histone deacetylase, and combinations of any of the foregoing.

### II. Site-Specific APOB Disrupting Agents of the Invention

The present invention provides site-specific APOB disrupting agents which, in one aspect of the invention, include a site-specific APOB targeting moiety which targets an APOB expression control region. In another aspect, the site-specific disrupting agents of the invention include a site-specific APOB targeting moiety which targets an APOB expression control region and an effector molecule. As will be appreciated by one of ordinary skill in the art, such disrupting agents are site-specific and, thus, specifically bind to an APOB expression control region (*e.g.*, one or more transcriptional control elements and/or one or more target anchor sequences), *e.g*., within a cell and not to non-targeted expression control regions (*e.g*., within the same cell).

The site-specific APOB disrupting agents of the invention comprise a site-specific APOB targeting moiety targeting an APOB expression control region. The expression control region targeted by the site-specific targeting moiety may be, for example, a transcriptional control element or an anchor sequence, such as an anchor sequence within an anchor-mediated conjunction.

Thus, site-specific APOB disrupting agents of the invention may modulate expression of a gene, *i.e*., APOB , *e.g.,* by modulating expression of the gene from an endogenous promoter, an enhancer, or a repressor, may alter methylation of the control region, may alter at least one anchor sequence; may alter at least one conjunction nucleating molecule binding site, such as by altering binding affinity for the conjunction nucleating molecule; may alter an orientation of at least one common nucleotide sequence, such as a CTCF binding motif by, *e.g*., substitution, addition or deletion in at least one anchor sequence, such as a CTCF binding motif.

In certain embodiments, the site-specific disrupting agents and compositions described herein target an expression control region comprising one or more APOB-specific transcriptional control elements to modulate expression in a cell. APOB-specific transcriptional control elements that can be targeted include APOB-specific promoter, APOB-specific enhancers, APOB-specific repressors, and APOB-associated anchor sequence, *e.g*., CTCF sites. In one embodiment, APOB-associated CTCF site regulates expression in cells of the liver, *e.g.,* CTCF site 3. The nucleotide sequences of APOB CTCF sites 3 may be found in, for example, GenBank Accession No. M96151.

For example, a site-specific disrupting agent may include a site-specific targeting moiety, *e.g*., a nucleic acid molecule, such as a guide RNA targeting an APOB endogenous CTCF sites, *e.g.,* CTCF site 3, and an effector molecule, such as an effector molecule that includes a transcriptional enhancer or transcriptional repressor that modulates, *e.g*., enhances or represses, expression of a target gene from an endogenous promoter to modulate gene expression.

In certain embodiments of the invention, the site-specific disrupting agents and compositions described herein target an expression control region comprising one or more APOB-associated anchor sequences, *e.g*., within an anchor sequence-mediated conjunction, comprising a first and a second APOB-associated anchor sequence to alter a two-dimensional chromatin structure (*e.g*., anchor sequence-mediated conjunctions in order to modulate expression in a cell, *e.g.,* a cell within a subject, *e.g.,* by modifying anchor sequence-mediated conjunctions in DNA, *e.g.,* genomic DNA.

In one aspect, the invention includes a site-specific APOB disrupting agent comprising a site-specific APOB targeting moiety which targets an APOB expression control region comprising one or more APOB-associated anchor sequences within an anchor sequence-mediated conjunction. The disrupting agent binds, *e.g*., specifically binds, a specific anchor sequence-mediated conjunction to alter a topology of the anchor sequence-mediated conjunction, *e.g*., an anchor sequence-mediated conjunction having a physical interaction of two or more DNA loci bound by a conjunction nucleating molecule.

The formation of an anchor sequence-mediated conjunction may force transcriptional control elements to interact with an APOB gene or spatially constrain the activity of the transcriptional control elements. Altering anchor sequence- mediated conjunctions, therefore, allows for modulating APOB expression without altering the coding sequences of the APOB gene being modulated.

In some embodiments, the site-specific disrupting agents and compositions of the invention modulate expression of anAPOB gene associated with an anchor sequence-mediated conjunction by physically interfering between one or more anchor sequences and a conjunction nucleating molecule. For example, a DNA binding small molecule (*e.g.*, minor or major groove binders), peptide (*e.g*., zinc finger, TALE, novel or modified peptide), protein (*e.g*., CTCF, modified CTCF with impaired CTCF binding and/or cohesion binding affinity), or nucleic acids (*e.g*., ssDNA, modified DNA or RNA, peptide oligonucleotide conjugates, locked nucleic acids, bridged nucleic acids, polyamides, and/or triplex forming oligonucleotides) may physically prevent a conjunction nucleating molecule from interacting with one or more anchor sequences to modulate APOB gene expression.

In some embodiments, the site-specific disrupting agents and compositions of the invention modulate expression of an APOB gene associated with an anchor sequence-mediated conjunction by modification of an anchor sequence, *e.g.,* epigenetic modifications, *e.g.,* histone protein modifications, or genomic editing modifications. For example, one or more anchor sequences associated with an anchor sequence-mediated conjunction comprising an APOB gene may be targeted for genome editing, *e.g*., Cas9-mediated genome editing.

In some embodiments, the site-specific disrupting agents and compositions of the invention modulate expression of an APOB gene associated with an anchor sequence-mediated conjunction, *e.g.*, activate or represses transcription, *e.g*., induces epigenetic changes to chromatin or genome editing.

In some embodiments, an anchor sequence-mediated conjunction includes one or more anchor sequences, an APOB gene, and one or more transcriptional control elements, such as an enhancing or silencing element. In some embodiments, the transcriptional control element is within, partially within, or outside the anchor sequence-mediated conjunction.

In one embodiment, the anchor sequence-mediated conjunction comprises a loop, such as an intra-chromosomal loop. In certain embodiments, the anchor sequence-mediated conjunction has a plurality of loops. One or more loops may include a first anchor sequence, a nucleic acid sequence, a transcriptional control element, and a second anchor sequence. In another embodiment, at least one loop includes, in order, a first anchor sequence, a transcriptional control element, and a second anchor sequence; or a first anchor sequence, a nucleic acid sequence, and a second anchor sequence. In yet another embodiment, either one or both of the nucleic acid sequences and the transcriptional control element is located within or outside the loop. In still another embodiment, one or more of the loops comprises a transcriptional control element.

In some embodiments, the anchor sequence-mediated conjunction includes a TATA box, a CAAT box, a GC box, or a CAP site.

In some embodiments, the anchor sequence-mediated conjunction comprises a plurality of loops, and where the anchor sequence-mediated conjunction comprises at least one of an anchor sequence, a nucleic acid sequence, and a transcriptional control element in one or more of the loops.

In one aspect, the site-specific disrupting agents and compositions of the invention may introduce a targeted alteration to an anchor sequence-mediated conjunction to modulate expression of a nucleic acid sequence with a disrupting agent that binds the anchor sequence. In some embodiments, the anchor sequence-mediated conjunction is altered by targeting one or more nucleotides within the anchor sequence-mediated conjunction for substitution, addition or deletion.

In some embodiments, expression, *e.g*., transcription, is activated by inclusion of an activating loop or exclusion of a repressive loop. In one such embodiment, the anchor sequence-mediated conjunction comprises a transcriptional control sequence that increases transcription of a nucleic acid sequence, *e.g*., such an APOB encoding nucleic acid. In another such embodiment, the anchor sequence-mediated conjunction excludes a transcriptional control element that decreases expression, *e.g.,* transcription, of a nucleic acid sequence, *e.g.,* such an APOB encoding nucleic acid.

In some embodiments, expression, *e.g*., transcription, is repressed by inclusion of a repressive loop or exclusion of an activating loop. In one such embodiment, the anchor sequence-mediated conjunction includes a transcriptional control element that decreases expression, *e.g*., transcription, of a nucleic acid sequence, *e.g*., such an APOB encoding nucleic acid sequence. In another such embodiment, the anchor sequence-mediated conjunction excludes a transcriptional control sequence that increases transcription of a nucleic acid sequence, *e.g*., such an APOB encoding nucleic acid.

Each anchor sequence-mediated conjunction comprises one or more anchor sequences, *e.g*., a plurality. Anchor sequences can be manipulated or altered to disrupt naturally occurring loops or form new loops (*e.g*., to form exogenous loops or to form non-naturally occurring loops with exogenous or altered anchor sequences). Such alterations modulate APOB gene expression by changing the 2-dimensional structure of DNA containing all or a portion of an APOB gene, *e.g*., by thereby modulating the ability of the APOB gene to interact with transcriptional control elements (e.g., enhancing and silencing/repressive sequences). In some embodiments, the chromatin structure is modified by substituting, adding or deleting one or more nucleotides within an anchor sequence of the anchor sequence-mediated conjunction.

The anchor sequences may be non-contiguous with one another. In embodiments with noncontiguous anchor sequences, the first anchor sequence may be separated from the second anchor sequence by about 500bp to about 500Mb, about 750bp to about 200Mb, about lkb to about 100Mb, about 25kb to about 50Mb, about 50kb to about 1Mb, about 100kb to about 750kb, about 150kb to about 500kb, or about 175kb to about 500kb. In some embodiments, the first anchor sequence is separated from the second anchor sequence by about 500bp, 600bp, 700bp, 800bp, 900bp, lkb, 5kb, 10kb, 15kb, 20kb, 25kb, 30kb, 35kb, 40kb, 45kb, 50kb, 55kb, 60kb, 65kb, 70kb, 75kb, 80kb, 85kb, 90kb, 95kb, 100kb, 125kb, 150kb, 175kb, 200kb, 225kb, 250kb, 275kb, 300kb, 350kb, 400kb, 500kb, 600kb, 700kb, 800kb, 900kb, 1Mb, 2Mb, 3Mb, 4Mb, 5Mb, 6Mb, 7Mb, 8Mb, 9Mb, 10Mb, 15Mb, 20Mb, 25Mb, 50Mb, 75Mb, 100Mb, 200Mb, 300Mb, 400Mb, 500Mb, or any size therebetween.

In one embodiment, the anchor sequence comprises a common nucleotide sequence, *e.g*., a CTCF-binding motif:
N(T/C/G)N(G/A/T)CC(A/T/G)(C/G)(C/T/A)AG(G/A)(G/T)GG(C/A/T)(G/A)(C/G)(C/T/A)(G/A/C) (SEQ ID NO: 1), where N is any nucleotide.

A CTCF-binding motif may also be in the opposite orientation, *e.g.*, (G/A/C)(C/T/A)(C/G)(G/A)(C/A/T)GG(G/T)(G/A)GA(C/T/A)(C/G)(A/T/G)CC(G/A/T)N(T/C/G)N (SEQ ID NO:2).

In one embodiment, the anchor sequence comprises SEQ ID NO: 1 or SEQ ID NO:2 or a nucleotide sequence at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% identical to either SEQ ID NO: 1 or SEQ ID NO:2.

In some embodiments, the anchor sequence-mediated conjunction comprises at least a first anchor sequence and a second anchor sequence. The first anchor sequence and second anchor sequence may each comprise a common nucleotide sequence, *e.g*., each comprises a CTCF binding motif. In some embodiments, the first anchor sequence and second anchor sequence comprise different sequences, *e.g*., the first anchor sequence comprises a CTCF binding motif and the second anchor sequence comprises an anchor sequence other than a CTCF binding motif. In some embodiments, each anchor sequence comprises a common nucleotide sequence and one or more flanking nucleotides on one or both sides of the common nucleotide sequence.

Two CTCF-binding motifs (*e.g*., contiguous or non-contiguous CTCF binding motifs) that can form a conjunction may be present in the genome in any orientation, *e.g.*, in the same orientation (tandem) either 5'->3' (left tandem, *e.g.,* the two CTCF-binding motifs that comprise SEQ ID NO: 1) or 3'-> 5' (right tandem, *e.g*., the two CTCF-binding motifs comprise SEQ ID NO:2), or convergent orientation, where one CTCF-binding motif comprises SEQ ID NO: 1 and the other comprises SEQ ID NO:2. CTCFBSDB 2.0: Database For CTCF binding motifs And Genome Organization (http ://insulatordb.uthsc.edu/) can be used to identify CTCF binding motifs associated with a target gene, *e.g.,* APOB .

In some embodiments, the anchor sequence-mediated conjunction is altered by changing an orientation of at least one common nucleotide sequence, *e.g*., a conjunction nucleating molecule binding site.

In some embodiments, the anchor sequence comprises a conjunction nucleating molecule binding site, *e.g.,* CTCF binding motif, and site-specific disrupting agent of the invention introduces an alteration in at least one conjunction nucleating molecule binding site, *e.g*. altering binding affinity for the conjunction nucleating molecule.

In some embodiments, the anchor sequence-mediated conjunction is altered by introducing an exogenous anchor sequence. Addition of a non-naturally occurring or exogenous anchor sequence to form or disrupt a naturally occurring anchor sequence-mediated conjunction, *e.g*., by inducing a non-naturally occurring loop to form that alters transcription of the nucleic acid sequence.

In some embodiments, the anchor sequence-mediated conjunction comprises an APOB gene, and one or more, *e.g.,* 2, 3, 4, 5, or other genes other than the APOB gene.

In some embodiments, the anchor sequence-mediated conjunction is associated with one or more, *e.g.,* 2, 3, 4, 5, or more, transcriptional control elements. In some embodiments, the APOB gene is noncontiguous with one or more of the transcriptional control elements. In some embodiments where the APOB gene is non-contiguous with the transcriptional control element, the gene may be separated from one or more transcriptional control elements by about 100bp to about 500Mb, about 500bp to about 200Mb, about lkb to about 100Mb, about 25kb to about 50Mb, about 50kb to about 1Mb, about 100kb to about 750kb, about 150kb to about 500kb, or about 175kb to about 500kb. In some embodiments, the gene is separated from the transcriptional control element by about 100bp, 300bp, 500bp, 600bp, 700bp, 800bp, 900bp, lkb, 5kb, 10kb, 15kb, 20kb, 25kb, 30kb, 35kb, 40kb, 45kb, 50kb, 55kb, 60kb, 65kb, 70kb, 75kb, 80kb, 85kb, 90kb, 95kb, 100kb, 125kb, 150kb, 175kb, 200kb, 225kb, 250kb, 275kb, 300kb, 350kb, 400kb, 500kb, 600kb, 700kb, 800kb, 900kb, 1Mb, 2Mb, 3Mb, 4Mb, 5Mb, 6Mb, 7Mb, 8Mb, 9Mb, 10Mb, 15Mb, 20Mb, 25Mb, 50Mb, 75Mb, 100Mb, 200Mb, 300Mb, 400Mb, 500Mb, or any size therebetween.

In some embodiments, the type of anchor sequence-mediated conjunction may help to determine how to modulate gene expression, *e.g*., choice of site-specific targeting moiety, by altering the anchor sequence- mediated conjunction. For example, some types of anchor sequence-mediated conjunctions comprise one or more transcription control elements within the anchor sequence - mediated conjunction. Disruption of such an anchor sequence-mediated conjunction by disrupting the formation of the anchor sequence- mediated conjunction, *e.g*., altering one or more anchor sequences, is likely to decrease transcription of an APOB gene within the anchor sequence-mediated conjunction.

In some embodiments, expression of the APOB gene is regulated, modulated, or influenced by one or more transcriptional control elements associated with the anchor sequence-mediated conjunction. In some embodiments, the anchor sequence-mediated conjunction comprises an APOB gene and one or more transcriptional control elements. For example, the APOB gene and one or more transcriptional control sequences are located within, at least partially, an anchor sequence-mediated conjunction, *e.g*., a Type 1 anchor sequence-mediated conjunction. The anchor sequence-mediated conjunction may also be referred to as a "Type 1, EP subtype." In some embodiments, the APOB gene has a defined state of expression, *e.g.*, in its native state, *e.g.,* in a diseased state. For example, the APOB gene may have a high level of expression. By disrupting the anchor sequence-mediated conjunction, expression of the APOB gene may be decreased, *e.g*., decreased transcription due to conformational changes of the DNA previously open to transcription within the anchor sequence-mediated conjunction, *e.g*., decreased transcription due to conformational changes of the DNA creating additional distance between the APOB gene and the enhancing sequences. In one embodiment, both theAPOB gene associated and one or more transcriptional control sequences, *e.g*., enhancing sequences, reside inside the anchor sequence-mediated conjunction. Disruption of the anchor sequence-mediated conjunction decreases expression of the APOB gene. In one embodiment, the APOB gene associated with the anchor sequence-mediated conjunction is accessible to one or more transcriptional control elements that reside inside, at least partially, the anchor sequence-mediated conjunction.

In some embodiments, expression of the APOB gene is regulated, modulated, or influenced by one or more transcriptional control elements associated with, but inaccessible due to the anchor sequence- mediated conjunction. For example, the anchor sequence-mediated conjunction associated with an APOB gene disrupts the ability of one or more transcriptional control elements to regulate, modulate, or influence expression of the APOB gene. The transcriptional control sequences may be separated from the APOB gene, *e.g.,* reside on the opposite side, at least partially, *e.g.,* inside or outside, of the anchor sequence-mediated conjunction as the APOB gene, *e.g.,* the APOB gene is inaccessible to the transcriptional control elements due to proximity of the anchor sequence-mediated conjunction. In some embodiments, one or more enhancing sequences are separated from the APOB gene by the anchor sequence-mediated conjunction, *e.g.*, a Type 2 anchor sequence-mediated conjunction.

In some embodiments, the APOB gene is inaccessible to one or more transcriptional control elements due to the anchor sequence-mediated conjunction, and disruption of the anchor sequence-mediated conjunction allows the transcriptional control element to regulate, modulate, or influence expression of the APOB gene. In one embodiment, the APOB gene is inside and outside the anchor sequence-mediated conjunction and inaccessible to the one or more transcriptional control elements. Disruption of the anchor sequence- mediated conjunction increases access of the transcriptional control elements to regulate, modulate, or influence expression of the APOB gene, *e.g.,* the transcriptional control elements increase expression of the APOB gene. In one embodiment, the APOB gene is inside the anchor sequence-mediated conjunction and inaccessible to the one or more transcriptional control elements residing outside, at least partially, the anchor sequence-mediated conjunction. Disruption of the anchor sequence-mediated conjunction increases expression of the APOB gene. In one embodiment, the APOB gene is outside, at least partially, the anchor sequence-mediated conjunction and inaccessible to the one or more transcriptional control elements residing inside the anchor sequence-mediated conjunction. Disruption of the anchor sequence -mediated conjunction increases expression of the APOB gene.

### A. APOB Site-Specific Targeting Moieties

The site-specific APOB targeting moieties of the invention target an APOB expression control region and may comprise a polymer or polymeric molecule, such as a polyamide (*i.e*., a molecule of repeating units linked by amide binds, *e.g.,* a polypeptide), a polymer of nucleotides (such as a guide RNA), a peptide nucleic acid (PNA), or a polymer of amino acids, such as a peptide or polypeptide, *e.g.,* a fusion protein, *etc.* Suitable site-specific APOB targeting moieties, compositions, and methods of use of such agents and compositions are described below and in PCT Publication WO 2018/049073.

In one embodiment, a site-specific disrupting agent of the invention comprises a site-specific APOB targeting moiety comprising a nucleic acid molecule, such as a guide RNA (or gRNA) or a guide RNA and an effector, or fragment thereof, or nucleic acid molecule encoding an effector, or fragment thereof.

In another embodiment, a site specific disrupting agent of the invention comprises a site-specific APOB targeting moiety comprising a nucleic acid molecule encoding a polypeptide, such as a DNA-binding domain, or fragment thereof, of a zinc finger polypeptide (ZNF) or a transcription activator-like effector (TALE) polypeptide, that is engineered to specifically target an APOB expression control region to modulate expression of an APOB gene.

In another embodiment, a site-specific disrupting agent of the invention comprises a site-specific APOB targeting moiety comprising a polynucleotide, such as a PNA, *e.g.,* a nucleic acid gRNA linked to an effector polypeptide, or fragment thereof.

In another embodiment, a site-specific disrupting agent of the invention comprises a site-specific APOB targeting moiety comprising a fusion molecule, such as a nucleic acid molecule encoding a fusion protein comprising a Cas polypeptide and, *e.g.,* an epigenetic recruiter or an epigenetic CpG modifier.

In yet, another embodiment, a site-specific disrupting agent of the invention comprises a site-specific APOB targeting moiety comprising a fusion molecule, such as fusion protein comprising a Cas polypeptide and, *e.g.,* an epigenetic recruiter or an epigenetic CpG modifier.

As used herein, in its broadest sense, the term "nucleic acid" refers to any compound and/or substance that is or can be incorporated into an oligonucleotide chain. In some embodiments, a nucleic acid is a compound and/or substance that is or can be incorporated into a polynucleotide chain *via* a phosphodiester linkage. As will be clear from context, in some embodiments, "nucleic acid" refers to an individual nucleic acid residue (*e.g*., a nucleotide and/or nucleoside); in some embodiments, "nucleic acid" refers to a polynucleotide chain comprising individual nucleic acid residues. In some embodiments, a "nucleic acid" is or comprises RNA; in some embodiments, a "nucleic acid" is or comprises DNA. In some embodiments, a "nucleic acid" is a "mixmer" comprising locked nucleic acid molecules and deoxynucleic acid molecules. In some embodiments, a nucleic acid is, comprises, or consists of one or more natural nucleic acid residues. In some embodiments, a nucleic acid is, comprises, or consists of one or more nucleic acid analogs. In some embodiments, a nucleic acid analog differs from a nucleic acid in that it does not utilize a phosphodiester backbone. For example, in some embodiments, a nucleic acid is, comprises, or consists of one or more "peptide nucleic acids", which are known in the art and have peptide bonds instead of phosphodiester bonds in the backbone, are considered within the scope of the present invention. Alternatively or additionally, in some embodiments, a nucleic acid has one or more phosphorothioate and/or 5'-N-phosphoramidite linkages rather than phosphodiester bonds. In some embodiments, a nucleic acid is, comprises, or consists of one or more natural nucleosides (*e.g*., adenosine, thymidine, guanosine, cytidine, uridine, deoxyadenosine, deoxythymidine, deoxy guanosine, and deoxycytidine). In some embodiments, a nucleic acid is, comprises, or consists of one or more nucleoside analogs (*e.g*., 2-aminoadenosine, 2-thiothymidine, inosine, pyrrolo-pyrimidine, 3 - methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5 -propynyl-cytidine, C5-methylcytidine, 2-aminoadenosine, 7-deazaadenosine, 7-deazaguanosine, 8-oxoadenosine, 8-oxoguanosine, 0(6)-methylguanine, 2-thiocytidine, methylated bases, intercalated bases, and combinations thereof). In some embodiments, a nucleic acid comprises one or more modified sugars (*e.g*., 2'-fluororibose, ribose, 2'-deoxyribose, arabinose, and hexose) as compared with those in natural nucleic acids. In some embodiments, a nucleic acid has a nucleotide sequence that encodes a functional gene product such as an RNA or protein. In some embodiments, a nucleic acid includes one or more introns. In some embodiments, nucleic acids are prepared by one or more of isolation from a natural source, enzymatic synthesis by polymerization based on a complementary template (*in vivo* or *in vitro*), reproduction in a recombinant cell or system, and chemical synthesis. In some embodiments, a nucleic acid is at least 3, 4, 5, 6, 7, 8, 9, 10, 15, 20, 25, 30, 35, 40, 45, 50, 55, 60, 65, 70, 75, 80, 85, 90, 95, 100, 1 10, 120, 130, 140, 150, 160, 170, 180, 190, 20, 225, 250, 275, 300, 325, 350, 375, 400, 425, 450, 475, 500, 600, 700, 800, 900, 1000, 1500, 2000, 2500, 3000, 3500, 4000, 4500, 5000 or more residues long. In some embodiments, a nucleic acid is partly or wholly single stranded; in some embodiments, a nucleic acid is partly or wholly double stranded. In some embodiments a nucleic acid has a nucleotide sequence comprising at least one element that encodes, or is the complement of a sequence that encodes, a polypeptide. In some embodiments, a nucleic acid has enzymatic activity.

As used herein, the terms "peptide," "polypeptide," and "protein" refer to a compound comprised of amino acid residues covalently linked by peptide bonds, or by means other than peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds or by means other than peptide bonds. As used herein, the term refers to both short chains, which also commonly are referred to in the art as peptides, oligopeptides and oligomers, for example, and to longer chains, which generally are referred to in the art as proteins, of which there are many types.

In certain embodiments, a polypeptide is or may comprise a chimeric or "fusion protein." As used herein, a "chimeric protein" or "fusion protein" comprises all or part (preferably a biologically active part) of a first protein operably linked to a heterologous second polypeptide (*i.e*., a polypeptide other than the first protein). Within the fusion protein, the term "operably linked" is intended to indicate that the first protein or segment thereof and the heterologous polypeptide are fused in-frame to each other. The heterologous polypeptide can be fused to the amino-terminus or the carboxyl-terminus of the first protein or segment.

A "polyamide" is a polymeric molecule with repeating units linked by amide binds. Proteins are examples of naturally occurring polyamides. In some embodiments, a polyamide comprises a peptide nucleic acid (PNA).

A "peptide nucleic acid" ("PNA") is a molecule in which one or more amino acid units in the PNA have an amide containing backbone, *e.g*., aminoethyl-glycine, similar to a peptide backbone, with a nucleic acid side chain in place of the amino acid side chain. Peptide nucleic acids (PNA) are known to hybridize complementary DNA and RNA with higher affinity than their oligonucleotide counterparts. This character of PNA not only makes them a stable hybrid with the nucleic acid side chains, but at the same time, the neutral backbone and hydrophobic side chains result in a hydrophobic unit within the polypeptide. The nucleic acid side chain includes, but is not limited to, a purine or a pyrimidine side chain such as adenine, cytosine, guanine, thymine and uracil. In one embodiment, the nucleic acid side chain includes a nucleoside analog as described herein.

In one embodiment, a site-specific APOB targeting moiety of the invention comprises a polyamide. Suitable polyamides for use in the agents and compositions of the invention are known in the art.

In one embodiment, a site-specific APOB targeting moiety of the invention comprises a polynucleotide. In some embodiments, the nucleotide sequence of the polynucleotide encodes an APOB gene or an APOB expression product. In some embodiments, the nucleotide sequence of the polynucleotide does not include an APOB coding sequence or an APOB expression product. For example, in some embodiments, a site-specific APOB targeting moiety of the invention comprises a polynucleotide that hybridizes to a target expression control region, *e.g.,* a promoter or an anchor sequence. In some embodiments, the nucleotide sequence of the polynucleotide is a complement of a target anchor sequence, or has a sequence that is at least 80%, at least 85%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% identical to a complement of the target sequence.

The polynucleotides of the invention may include deoxynucleotides, ribonucleotides, modified deoxynucleotides, modified ribonucleotides (*e.g*., chemical modifications, such as modifications that alter the backbone linkages, sugar molecules, and/or nucleic acid bases), and artificial nucleic acids. In some embodiments, the polynucleotide includes, but is not limited to, genomic DNA, cDNA, peptide nucleic acids (PNA) or peptide oligonucleotide conjugates, locked nucleic acids (LNA), bridged nucleic acids (BNA), polyamides, triplex forming oligonucleotides, modified DNA, antisense DNA oligonucleotides, tRNA, mPvNA, rPvNA, modified RNA, miRNA, gRNA, and siRNA or other RNA or DNA molecules.

In some embodiments, the polynucleotides of the invention have a length from about 2 to about 5000 nts, about 10 to about 100 nts, about 50 to about 150 nts, about 100 to about 200 nts, about 150 to about 250 nts, about 200 to about 300 nts, about 250 to about 350 nts, about 300 to about 500 nts, about 10 to about 1000 nts, about 50 to about 1000 nts, about 100 to about 1000 nts, about 1000 to about 2000 nts, about 2000 to about 3000 nts, about 3000 to about 4000 nts, about 4000 to about 5000 nts, or any range therebetween.

The polynucleotides of the invention may include nucleosides, *e.g*., purines or pyrimidines, *e.g*., adenine, cytosine, guanine, thymine and uracil. In some embodiments, the polynucleotides includes one or more nucleoside analogs. The nucleoside analog includes, but is not limited to, a nucleoside analog, such as 5-fluorouracil; 5-bromouracil, 5-chlorouracil, 5-iodouracil, hypoxanthine, xanthine, 4-acetylcytosine, 4- methylbenzimidazole, 5-(carboxyhydroxylmethyl) uracil, 5-carboxymethylaminomethyl-2-thiouridine, 5- carboxymethylaminomethyluracil, dihydrouracil, dihydrouridine, beta-D-galactosylqueosine, inosine, N6-isopentenyladenine, 1-methylguanine, 1-methylinosine, 2,2-dimethylguanine, 2-methyladenine, 2- methylguanine, 3-methylcytosine, 5-methylcytosine, N6-adenine, 7-methylguanine, 5- methylaminomethyluracil, 5-methoxyaminomethyl-2-thiouracil, beta-D-mannosylqueosine, 5'- methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4- thiouracil, 5-methyluracil, uracil-5-oxyacetic acid methylester, uracil-5-oxyacetic acid (v), 5-methyl-2- thiouracil, 3-(3-amino-3-N-2-carboxypropyl) uracil, (acp3)w, 2,6-diaminopurine, 3-nitropyrrole, inosine, thiouridine, queuosine, wyosine, diaminopurine, isoguanine, isocytosine, diaminopyrimidine, 2,4- difluorotoluene, isoquinoline, pyrrolo[2,3- ]pyridine, and any others that can base pair with a purine or a pyrimidine side chain.

In some embodiments, the site-specific APOB targeting moieties of the invention comprising a polynucleotide encoding a polypeptide that comprises a DNA-binding domain, or fragment thereof, of a zinc finger polypeptide (ZNF) or a transcription activator-like effector (TALE) polypeptide, that is engineered to specifically target an APOB expression control region to modulate expression of an APOB gene.

The design and preparation of such zinc finger polynucleotides which specifically bind to a DNA target region of interest, such as an APOB expression control region, is well known in the art. For example, zinc finger (ZNF) proteins contain a DNA binding motif that specifically binds a triplet of nucleotides. Thus to design and prepare the site-specific APOB targeting moieties of the invention, a modular assembly process which includes combining separate zinc finger DNA binding domains that can each recognize a specific 3-basepair DNA sequence to generate 3-finger, 4-, 5-, or 6-finger arrays that recognize target sites ranging from 9 basepairs to 18 basepairs in length may be used. Another suitable method may include 2-finger modules to generate ZNF polynucleotides with up to six individual zinc fingers. See, *e.g.,* Shukla VK, et al., Nature. 459 (7245) 2009: 437-41; Dreier B, et al., JBC. 280 (42) 2005: 35588-97; Dreier B, et al, JBC 276 (31) 2001: 29466-78; Bae KH, et al., Nature Biotechnology. 21 (3) 2003: 275-80.

In some embodiments, a site-specific APOB targeting moiety of the invention comprises a polynucleotide encoding a polypeptide that comprises a DNA-binding domain (DBD), or fragment thereof, of a zinc finger, that is engineered to specifically target an ApoB expression control region to modulate expression of an ApoB gene. Exemplary amino acid sequences encoding a zinc finger that binds to a nucleotide triplet suitable for use in the present invention are provided in Table 1 below. (See, *e.g.,* Gersbach *et al.,* Synthetic Zinc Finger Proteins: The Advent of Targeted Gene Regulation and Genome Modification Technologies).

**Table 1.**

| Amino Acid Sequence of Zing Finger DNA Binding Domain (Finger) | Nucleotide Triplet | SEQ ID NO. |
|---|---|---|
| RKDALRG | TTG | 1 |
| TTGALTE | CTT | 2 |
| QRHHLVE | CTC | 3 |
| QNSTLTE | CTA | 4 |
| RNDALTE | CTG | 5 |
| HKNALQN | ATT | 6 |
| RRSACRR | ATC | 7 |
| QKSSLIA | ATA | 8 |
| RRDELNV | ATG | 9 |
| TSGSLVR | GTT | 10 |
| DPGALVR | GTC | 11 |
| QSSSLVR | GTA | 12 |
| RSDELVR | GTG | 13 |
| RLRDIQF | TCT | 14 |
| RSDERKR | TCC | 15 |
| RSDHLTT | TCA | 16 |
| RLRALDR | TCG | 17 |
| TKNSLTE | CCT | 18 |
| SKKHLAE | CCC | 19 |
| TSHSLTE | CCA | 20 |
| RNDTLTE | CCG | 21 |
| THLDLIR | ACT | 22 |
| DKKDLTR | ACC | 23 |
| SPADLTR | ACA | 24 |
| RTDTLRD | ACG | 25 |
| TSGELVR | GCT | 26 |
| DCRDLAR | GCC | 27 |
| QSGDLRR | GCA | 28 |
| RSDDLVR | GCG | 29 |
| ARGNLRT | TAT | 30 |
| SRGNLKS | TAC | 31 |
| QASNLIS | TAA | 32 |
| REDNLHT | TAG | 33 |
| TSGNLTE | CAT | 34 |
| SKKALTE | CAC | 35 |
| QSGNLTE | CAA | 36 |
| RADNLTE | CAG | 37 |
| TTGNLTV | AAT | 38 |
| DSGNLRV | AAC | 39 |
| QRANLRA | AAA | 40 |
| RKDNLKN | AAG | 41 |
| TSGNLVR | GAT | 42 |
| DPGNLVR | GAC | 43 |
| QSSNLVR | GAA | 44 |
| RSDNLVR | GAG | 45 |
| APKALGW | TGC | 46 |
| QAGHLAS | TGA | 47 |
| RSDHLTT | TGG | 48 |
| SRRTCRA | CGT | 49 |
| HTGHLLE | CGC | 50 |
| QSGHLTE | CGA | 51 |
| RSDKLTE | CGG | 52 |
| HRTTLTN | AGT | 53 |
| ERSHLRE | AGC | 54 |
| QLAHLRA | AGA | 55 |
| RSDHLTN | AGG | 56 |
| TSGHLVR | GGT | 57 |
| DPGHLVR | GGC | 58 |
| QRAHLER | GGA | 59 |
| RSDKLVR | GGG | 60 |

A zinc finger DNA binding domain comrpises an N-terminal region and a C-terminal region with the "fingers" that bind to the target DNA sequence in between. The N-terminal region generally is 7 amino acids in length. The C-terminal region is generally 6 amino acids in length. Thus, the N-terminal region generally comprises the amino acid sequence of X₁X₂X₃X₄X₅X₆X₇ (SEQ ID NO:). "X" can be any amino acid. In some embodiments, the N-terminal region comprises the exemplary amino acid sequence of LEPGEKP (SEQ ID NO:). "X" can be any amino acid. The C-terminal region generally comprises the amino acid sequence of X₂₅X₂₆X₂₇X₂₈X₂₉X₃₀ (SEQ ID NO:). In certain embodiments, the C-terminal region comprises the exemplary amino acid sequence of TGKKTS (SEQ ID NO:)

Each finger in the DNA binding domain is flanked by a N-terminal backbone located to the N-terminus of the finger and a C-terminal backbone located to the C-terminus of the finger. The N-terminal backbone of the finger generally is 11 amino acids long with two conservative cysteines (C) locate at 3rd and 6th positions. Thus, the N-terminal backbone of the finger generally comprises the amino acid sequence of X₈X₉CX₁₀X₁₁CX₁₂X₁₃X₁₄X₁₅X₁₆ (SEQ ID NO:). "X" can be any amino acid. The C-terminal backbone of the finger generally is 5 amino acids long with two conservative histines (H) located at 1st and 5th positions. Thus, the C-terminal backbone of the finger generally comprises the amino acid sequence of HX₁₇X₁₈X₁₉H (SEQ ID NO: ). "X" can be any amino acid. In some embodiments, the N-terminal backbone comprises the exemplary amino acid sequence of YKCPECGKSFS (SEQ ID No. 61) and the C-terminal backbone comprises the exemplary amino acid sequence of HQRTH (SEQ ID No. 62). Two "fingers" are linked through a linker. A linker generally is 5 amino acids in length and comprises the amino acidsequence of X₂₀X₂₁X₂₂X₂₃X₂₄ (SEQ ID NO: ). "X" can be any amino acid. In certain embodiments, the linker comprises the exemplary amino acid sequence of TGEKP (SEQ ID No. 63). Thus, the zinc finger of a site specific ApoB site-specific disrupting agent has a structure as follows: (N-terminal backbone - finger - C-terminal backbone - linker)n and the zinc finger DNA binding domain of a site specific ApoB site-specific disrupting agent has a structure as follows: [N-terminal region (N-terminal backbone - finger - C-terminal backbone - linker)n- C-terminal region]. "N" represents the number of triplets of nucleotides to which the zinc finger DNA binding domain and, thus, to which the ApoB site-specific disrupting agent binds.

The "finger" amino acid sequences of four nucleotide triplets are unknown, however, if such a triplet is identified in a target area of interest, two "linker span sequences" - linker span 1 and linker span 2 - are useful to circumvent the issue. Linker span 1 is used to skip one base pair if a "finger" amino acid sequence of a triplet is not available. Linker span 2 is used to skip 2 base pairs if a "finger" amino acid sequence of a triplet is not available. Linker span 1 is generally 12 amino acids long. Linker span 2 is generally 16 amino acids long. Thus, linker span 1 generally comprises the amino acid sequence of X₃₁X₃₂X₃₃X₃₄X₃₅X₃₆X₃₇X₃₈X₃₉X₄₀X₄₁X₄₂ (SEQ ID NO: ). Linker span 2 generally comprises the amino acid sequence of X₄₃X₄₄X₄₃X₄₆X₄₇X₄₈X₄₉X₃ₒX₃₁X₃₂X₅₃X₃₄X₃₃X₃₆X₃₇X₃₈ (SEQ ID NO: ). In some embodiments, linker span 1 comprises the amino acid sequence of THPRAPIPKPFQ (SEQ ID NO: ). In certain embodiments, linker span 2 comprises the amino acid sequence of TPNPHRRTDPSHKPFQ (SEQ ID NO: ). When linker span 1 and/or linker span 2 is used, the finger - linker span 1/ span 2 - finger comprises the structure as follows: N-terminal back bone - finger - C-terminal backbone - linker span 1 /span 2 - N-terminal backbone - finger - C-terminal backbone - linker. Similarly, the design and preparation of such TALE polypeptides which specifically bind to a DNA target region of interest, such as an APOB expression control region, is well known in the art. For example, the DNA binding domain of TALE contains a repeated highly conserved 33-34 amino acid sequence with divergent 12th and 13th amino acids. These two positions, referred to as the Repeat Variable Diresidue (RVD), are highly variable and show a strong correlation with specific nucleotide recognition. This straightforward relationship between amino acid sequence and DNA recognition has allowed for the engineering of specific DNA-binding domains by selecting a combination of repeat segments containing the appropriate RVDs. See, *e.g.,* Boch J Nature Biotechnology. 29 (2) 2011: 135-6; Boch J, et al., Science. 326 (5959) 2009: 1509-12; Moscou MJ & Bogdanove AJ Science. 326 (5959) 2009: 1501.

In some embodiments, the site-specific APOB targeting moieties of the invention comprising a polynucleotide comprise a guide RNA (or gRNA) or nucleic acid encoding a guide RNA. A gRNA is a short synthetic RNA molecule comprising a "scaffold" sequence necessary for, *e.g*., directing an effector to an APOB expression control element which may, *e.g*., include an about 20 nucleotide site-specific sequence targeting a genomic target sequence comprising the APOB expression control element.

Generally, guide RNA sequences are designed to have a length of between about 17 to about 24 nucleotides (*e.g*., 19, 20, or 21 nucleotides) and are complementary to the target sequence. Custom gRNA generators and algorithms are available commercially for use in the design of effective guide RNAs. Gene editing has also been achieved using a chimeric "single guide RNA" ("sgRNA"), an engineered (synthetic) single RNA molecule that mimics a naturally occurring crRNA-tracrRNA complex and contains both a tracrRNA (for binding the nuclease) and at least one crRNA (to guide the nuclease to the sequence targeted for editing). Chemically modified sgNAs have also been demonstrated to be effective in genome editing; see, for example, Hendel et al. (2015) Nature Biotechnol., 985 - 991.

Exemplary site-specific APOB targeting moieties comprising a polynucleotide, *e.g*., gRNA, are provided in Table 2, below. In some embodiments, the polynucleotide comprises a nucleotide sequence at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% identical to the entire nucleotide sequence of any one of the nucleotide sequences in Table 2.

It will be understood that, although the sequences in Table 2 are described as modified (or unmodified), the nucleic acid molecule encompassed by the of the invention, *e.g*., a site-specific disrupting agent, may comprise any one of the sequences set forth in Table 2 that is un-modified or modified differently than described therein.

In some embodiments, a site-specific APOB targeting moiety comprising a polynucleotide, *e.g.,* gRNA, comprises a nucleotide sequence complementary to an anchor sequence. In one embodiment, the anchor sequence comprises a CTCF-binding motif or consensus sequence: N(T/C/G)N(G/A/T)CC(A/T/G)(C/G)(C/T/A)AG(G/A)(G/T)GG(C/A/T)(G/A)(C/G)(C/T/A)(G/A/C) (SEQ ID NO: 1), where N is any nucleotide. A CTCF-binding motif or consensus sequence may also be in the opposite orientation, *e.g*., (G/A/C)(C/T/A)(C/G)(G/A)(C/A/T)GG(G/T)(G/A)GA(C/T/A)(C/G)(A/T/G)CC(G/A/T)N(T/C/G)N (SEQ ID NO: 2). In some embodiments, the nucleic acid sequence comprises a sequence complementary to a CTCF-binding motif or consensus sequence.

In some embodiments, the polynucleotide comprises a nucleotide sequence at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% complementary to an anchor sequence.

In some embodiments, the polynucleotide comprises a nucleotide sequence at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% complementary to a CTCF-binding motif or consensus sequence. In some embodiments, the polynucleotide is selected from the group consisting of a gRNA, and a sequence complementary or a sequence comprising at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% complementary sequence to an anchor sequence.

In some embodiments, a site-specific APOB targeting moiety comprising a polynucleotide of the invention is an RNAi molecule. RNAi molecules comprise RNA or RNA-like structures typically containing 15-50 base pairs (such as aboutl 8-25 base pairs) and having a nucleobase sequence identical (complementary) or nearly identical (substantially complementary) to a coding sequence in an expressed target gene within the cell. RNAi molecules include, but are not limited to: short interfering RNAs (siRNAs), double-strand RNAs (dsRNA), micro RNAs (miRNAs), short hairpin RNAs (shRNA), meroduplexes, and dicer substrates (U.S. Patent Nos. 8,084,599, 8,349,809, and 8,513,207). In one embodiment, the invention includes a composition to inhibit expression of a gene encoding a polypeptide described herein, *e.g.,* a conjunction nucleating molecule.

RNAi molecules comprise a sequence substantially complementary, or fully complementary, to all or a fragment of a target gene. RNAi molecules may complement sequences at the boundary between introns and exons to prevent the maturation of newly-generated nuclear RNA transcripts of specific genes into mRNA for transcription. RNAi molecules complementary to specific genes can hybridize with the mRNA for that gene and prevent its translation. The antisense molecule can be DNA, RNA, or a derivative or hybrid thereof. Examples of such derivative molecules include, but are not limited to, peptide nucleic acid (PNA) and phosphorothioate-based molecules such as deoxyribonucleic guanidine (DNG) or ribonucleic guanidine (R G).

RNAi molecules can be provided to the cell as "ready-to-use" RNA synthesized in vitro or as an antisense gene transfected into cells which will yield RNAi molecules upon transcription. Hybridization with mRNA results in degradation of the hybridized molecule by RNAse H and/or inhibition of the formation of translation complexes. Both result in a failure to produce the product of the original gene.

The length of the RNAi molecule that hybridizes to the transcript of interest should be around 10 nucleotides, between about 15 or 30 nucleotides, or about 15, 16, 17, 18, 19, 20, 21, 22, 23, 24, 25, 26, 27, 28, 29, 30 or more nucleotides. The degree of identity of the antisense sequence to the targeted transcript should be at least 75%, at least 80%, at least 85%, at least 90%, or at least 95.

RNAi molecules may also comprise overhangs, *i.e.* typically unpaired, overhanging nucleotides which are not directly involved in the double helical structure normally formed by the core sequences of the herein defined pair of sense strand and antisense strand. RNAi molecules may contain 3' and/or 5' overhangs of about 1-5 bases independently on each of the sense strands and antisense strands. In one embodiment, both the sense strand and the antisense strand contain 3' and 5' overhangs. In one embodiment, one or more of the 3' overhang nucleotides of one strand base pairs with one or more 5' overhang nucleotides of the other strand. In another embodiment, the one or more of the 3' overhang nucleotides of one strand base do not pair with the one or more 5' overhang nucleotides of the other strand. The sense and antisense strands of an RNAi molecule may or may not contain the same number of nucleotide bases. The antisense and sense strands may form a duplex wherein the 5' end only has a blunt end, the 3' end only has a blunt end, both the 5' and 3' ends are blunt ended, or neither the 5' end nor the 3' end are blunt ended. In another embodiment, one or more of the nucleotides in the overhang contains a thiophosphate, phosphorothioate, deoxynucleotide inverted (3' to 3' linked) nucleotide or is a modified ribonucleotide or deoxynucleotide.

Small interfering RNA (siRNA) molecules comprise a nucleotide sequence that is identical to about 15 to about 25 contiguous nucleotides of the target mRNA. In some embodiments, the siRNA sequence commences with the dinucleotide AA, comprises a GC -content of about 30-70% (about 50-60%, about 40-60%, or about 45%-55%), and does not have a high percentage identity to any nucleotide sequence other than the target in the genome of the mammal in which it is to be introduced, for example as determined by standard BLAST search.

siRNAs and shRNAs resemble intermediates in the processing pathway of the endogenous microRNA (miRNA) genes (Bartel, Cell 116:281-297, 2004). In some embodiments, siRNAs can function as miRNAs and vice versa (Zeng et al., Mol Cell 9: 1327-1333, 2002; Doench et al., Genes Dev 17:438-442, 2003). MicroRNAs, like siRNAs, use RISC to downregulate target genes, but unlike siRNAs, most animal miRNAs do not cleave the mRNA. Instead, miRNAs reduce protein output through translational suppression or polyA removal and mRNA degradation (Wu et al., Proc Natl Acad Sci USA 103 :4034-4039, 2006). Known miRNA binding sites are within mRNA 3' UTRs; miRNAs seem to target sites with near-perfect complementarity to nucleotides 2-8 from the miRNA's 5' end (Rajewsky, Nat Genet 38 Suppl: S8- 13, 2006; Lim et al, Nature 433 :769-773, 2005). This region is known as the seed region. Because siRNAs and miRNAs are interchangeable, exogenous siRNAs downregulate mRNAs with seed complementarity to the siRNA (Birmingham et al., Nat Methods 3 : 199-204, 2006. Multiple target sites within a 3' UTR give stronger downregulation (Doench et al., Genes Dev 17:438-442, 2003).

Lists of known miRNA sequences can be found in databases maintained by research organizations, such as Wellcome Trust Sanger Institute, Perm Center for Bioinformatics, Memorial Sloan Kettering Cancer Center, and European Molecule Biology Laboratory, among others. Known effective siRNA sequences and cognate binding sites are also well represented in the relevant literature. RNAi molecules are readily designed and produced by technologies known in the art. In addition, there are computational tools that increase the chance of finding effective and specific sequence motifs (Pei et al. 2006, Reynolds et al. 2004, Khvorova et al. 2003, Schwarz et al. 2003, Ui-Tei et al. 2004, Heale et al. 2005, Chalk et al. 2004, Amarzguioui et al. 2004).

An RNAi molecule modulates expression of RNA encoded by a gene. Because multiple genes can share some degree of sequence homology with each other, in some embodiments, the RNAi molecule can be designed to target a class of genes with sufficient sequence homology. In some embodiments, the RNAi molecule can contain a sequence that has complementarity to sequences that are shared amongst different gene targets or are unique for a specific gene target. In some embodiments, the RNAi molecule can be designed to target conserved regions of an RNA sequence having homology between several genes thereby targeting several genes in a gene family (*e.g*., different gene isoforms, splice variants, mutant genes, *etc.*)*.* In some embodiments, the RNAi molecule can be designed to target a sequence that is unique to a specific RNA sequence of a single gene.

In some embodiments, the RNAi molecule targets a sequence in a conjunction nucleating molecule, *e.g.,* CTCF, cohesin, USF 1, YY1, TATA-box binding protein associated factor 3 (TAF3), ZNF 143, or another polypeptide that promotes the formation of an anchor sequence-mediated conjunction, or an epigenetic modifying agent, *e.g*., an enzyme involved in post-translational modifications including, but are not limited to, DNA methylases (*e.g.*, DNMT3a, DNMT3b, DNMTL), DNA demethylation (*e.g*., the TET family enzymes catalyze oxidation of 5-methylcytosine to 5-hydroxymethylcytosine and higher oxidative derivatives), histone methyltransferases, histone deacetylase (*e.g*., HDACl, HDAC2, HDAC3), sirtuin 1, 2, 3, 4, 5, 6, or 7, lysine-specific histone demethylase 1 (LSD1), histone-lysine-N-methyltransferase (Setdbl), euchromatic histone-lysine N-methyltransferase 2 (G9a), histone-lysine N-methyltransferase (SUV39H1), enhancer of zeste homolog 2 (EZH2), viral lysine methyltransferase (vSET), histone methyltransferase (SET2), protein-lysine N-methyltransferase (SMYD2), and others. In one embodiment, the RNAi molecule targets a protein deacetylase, *e.g*., sirtuin 1, 2, 3, 4, 5, 6, or 7. In one embodiment, the invention includes a composition comprising an RNAi that targets a conjunction nucleating molecule, *e.g*., CTCF.

In some embodiments, the site-specific APOB targeting moiety comprises a peptide or protein moiety. In some embodiments, a site-specific disrupting agent comprises a fusion protein. In some embodiments, an effector is ca peptide or protein moiety. The peptide or protein moieties may include, but is not limited to, a peptide ligand, antibody fragment, or targeting aptamer that binds a receptor such as an extracellular receptor, neuropeptide, hormone peptide, peptide drug, toxic peptide, viral or microbial peptide, synthetic peptide, and agonist or antagonist peptide.

Exemplary peptides or protein include a DNA- binding protein, a CRISPR component protein, a conjunction nucleating molecule, a dominant negative conjunction nucleating molecule, an epigenetic modifying agent, or any combination thereof. In some embodiments, the peptide comprises a nuclease, a physical blocker, an epigenetic recruiter, and an epigenetic CpG modifier, and fragments and combinations of any of the foregoing. In some embodiments, the peptide comprises a DNA-binding domain of a protein, such as a helix-turn-helix motif, a leucine zipper, a Zn-finger, a TATA box binding proteins, a transcription factor.

Peptides or proteins may be linear or branched. The peptide or protein moiety may have a length from about 5 to about 200 amino acids, about 15 to about 150 amino acids, about 20 to about 125 amino acids, about 25 to about 100 amino acids, or any range therebetween.

As indicated above, in some embodiments, the site-specific APOB targeting moieties of the invention comprise a fusion protein.

In some embodiments, the fusion proteins of the invention include a site-specific APOB targeting moiety which targets an APOB expression control region and an effector molecule. In other embodiments, a fusion protein of the invention comprises an effector molecule. Exemplary effector molecules include are described below and in some embodiments include, for example, nucleases, physical blockers, epigenetic recruiters, *e.g*., a transcriptional enhancer or a transcriptional repressor, and epigenetic CpG modifiers, *e.g*., a DNA methylase, a DNA demethylase, a histone modifying agent, or a histone deacetylase, and combinations of any of the foregoing.

For example, a site-specific targeting moiety may comprise a gRNA and an effector, such as a nuclease, *e.g.,* a Cas9, *e.g.,* a wild type Cas9, a nickase Cas9 (*e.g.,* Cas9 D10A), a dead Cas9 (dCas9), eSpCas9, Cpfl, C2C1, or C2C3, or a nucleic acid encoding such a nuclease. The choice of nuclease and gRNA(s) is determined by whether the targeted mutation is a deletion, substitution, or addition of nucleotides, *e.g*., a deletion, substitution, or addition of nucleotides to a targeted sequence. Fusions of a catalytically inactive endonuclease *e.g.,* a dead Cas9 (dCas9, *e.g.,* D10A; H840A) tethered with all or a portion of (*e.g.,* biologically active portion of) an (one or more) effector domain create chimeric proteins that can be linked to the polypeptide to guide the composition to specific DNA sites by one or more RNA sequences (e.g., DNA recognition elements including, but not restricted to zinc finger arrays, sgRNA, TAL arrays, peptide nucleic acids described herein) to modulate activity and/or expression of one or more target nucleic acids sequences (*e.g*., to methylate or demethylate a DNA sequence).

In one embodiment, a fusion protein of the invention may comprise an effector molecule comprising, for example, a CRISPR associated protein (Cas) polypeptide, or fragment thereof, (*e.g.,* a Cas9 polypeptide, or fragment thereof) and an epigenetic recruiter or an epigenetic CpG modifier.

In one embodiment, a suitable Cas polypeptide is an enzymatically inactive Cas polypeptide, *e.g.,* a "dead Cas polypeptide" or "dCas" polypeptide

Exemplary Cas polypeptides that are adaptable to the methods and compositions described herein are described below. Using methods known in the art, a Cas polypeptide can be fused to any of a variety of agents and/or molecules as described herein; such resulting fusion molecules can be useful in various disclosed methods.

In one aspect, the invention includes a composition comprising a protein comprising a domain, *e.g.,* an effector, that acts on DNA (*e.g.,* a nuclease domain, *e.g.,* a Cas9 domain, *e.g.,* a dCas9 domain; a DNA methyltransferase, a demethylase, a deaminase), in combination with at least one guide RNA (gRNA) or antisense DNA oligonucleotide that targets the protein to site-specific target sequence, wherein the composition is effective to alter, in a human cell, the expression of a target gene. In some embodiments, the enzyme domain is a Cas9 or a dCas9. In some embodiments, the protein comprises two enzyme domains, *e.g*., a dCas9 and a methylase or demethylase domain.

In one aspect, the invention includes a composition comprising a protein comprising a domain, *e.g.,* an effector, that comprises a transcriptional control element (*e.g.,* a nuclease domain, *e.g.,* a Cas9 domain, *e.g*., a dCas9 domain; a transcriptional enhancer; a transcriptional repressor), in combination with at least one guide RNA (gRNA) or antisense DNA oligonucleotide that targets the protein to a site-specific target seqeunce, wherein the composition is effective to alter, in a human cell, the expression of a target gene. In some embodiments, the enzyme domain is a Cas9 or a dCas9. In some embodiments, the protein comprises two enzyme domains, *e.g*., a dCas9 and a transcriptional enhancer or transcriptional repressor domain.

As used herein, a "biologically active portion of an effector domain" is a portion that maintains the function (*e.g.* completely, partially, minimally) of an effector domain (*e.g.,* a "minimal" or "core" domain).

The chimeric proteins described herein may also comprise a linker, *e.g.*, an amino acid linker. In some aspects, a linker comprises 2 or more amino acids, *e.g.,* one or more GS sequences. In some aspects, fusion of Cas9 (*e.g.,* dCas9) with two or more effector domains (*e.g.,* of a DNA methylase or enzyme with a role in DNA demethylation or protein acetyl transferase or deacetylase) comprises one or more interspersed linkers (*e.g*., GS linkers) between the domains. In some aspects, dCas9 is fused with 2- 5 effector domains with interspersed linkers.

In some embodiments, a site-specific APOB targeting moiety comprises a conjunction nucleating molecule, a nucleic acid encoding a conjunction nucleating molecule, or a combination thereof. In some embodiments, an anchor sequence-mediated conjunction is mediated by a first conjunction nucleating molecule bound to the first anchor sequence, a second conjunction nucleating molecule bound to the noncontiguous second anchor sequence, and an association between the first and second conjunction nucleating molecules. In some embodiments, a conjunction nucleating molecule may disrupt, *e.g*., by competitive binding, the binding of an endogenous conjunction nucleating molecule to its binding site.

The conjunction nucleating molecule may be, *e.g.,* CTCF, cohesin, USF1, YY1, TATA-box binding protein associated factor 3 (TAF3), ZNF143 binding motif, or another polypeptide that promotes the formation of an anchor sequence-mediated conjunction. The conjunction nucleating molecule may be an endogenous polypeptide or other protein, such as a transcription factor, *e.g.,* autoimmune regulator (AIRE), another factor, *e.g*., X-inactivation specific transcript (XIST), or an engineered polypeptide that is engineered to recognize a specific DNA sequence of interest, *e.g*., having a zinc finger, leucine zipper or bHLH domain for sequence recognition. The conjunction nucleating molecule may modulate DNA interactions within or around the anchor sequence -mediated conjunction. For example, the conjunction nucleating molecule can recruit other factors to the anchor sequence that alters an anchor sequence- mediated conjunction formation or disruption.

The conjunction nucleating molecule may also have a dimerization domain for homo- or heterodimerization. One or more conjunction nucleating molecules, *e.g*., endogenous and engineered, may interact to form the anchor sequence-mediated conjunction. In some embodiments, the conjunction nucleating molecule is engineered to further include a stabilization domain, *e.g.*, cohesion interaction domain, to stabilize the anchor sequence-mediated conjunction. In some embodiments, the conjunction nucleating molecule is engineered to bind a target sequence, *e.g*., target sequence binding affinity is modulated. In some embodiments, the conjunction nucleating molecule is selected or engineered with a selected binding affinity for an anchor sequence within the anchor sequence-mediated conjunction. Conjunction nucleating molecules and their corresponding anchor sequences may be identified through the use of cells that harbor inactivating mutations in CTCF and Chromosome Conformation Capture or 3C-based methods, *e.g*., Hi-C or high-throughput sequencing, to examine topologically associated domains, *e.g*., topological interactions between distal DNA regions or loci, in the absence of CTCF. Long-range DNA interactions may also be identified. Additional analyses may include ChlA- PET analysis using a bait, such as Cohesin, YY1 or USF1, ZNF143 binding motif, and MS to identify complexes that are associated with the bait.

### B. Effector Molecules

Effector molecules for use in the compositions and methods of the invention include those that modulate a biological activity, for example increasing or decreasing enzymatic activity, gene expression, cell signalling, and cellular or organ function. Preferred effector molecules of the invention are nucleases, physical blockers, epigenetic recruiters, *e.g*., a transcriptional enhancer or a transcriptional repressor, and epigenetic CpG modifiers, *e.g*., a DNA methylase, a DNA demethylase, a histone modifying agent, or a histone deacetylase, and combinations of any of the foregoing.

Additional effector activities may also include binding regulatory proteins to modulate activity of the regulator, such as transcription or translation. Effector molecules also may include activator or inhibitor (or "negative effector") functions as described herein. In another example, the effector molecule may inhibit substrate binding to a receptor and inhibit its activation, *e.g.*, naltrexone and naloxone bind opioid receptors without activating them and block the receptors' ability to bind opioids. Effector molecules may also modulate protein stability/degradation and/or transcript stability /degradation. For example, proteins may be targeted for degradation by the polypeptide co-factor, ubiquitin, onto proteins to mark them for degradation. In another example, an effector molecule inhibits enzymatic activity by blocking the enzyme's active site, *e.g*., methotrexate is a structural analog of tetrahydrofolate, a coenzyme for the enzyme dihydrofolate reductase that binds to dihydrofolate reductase 1000-fold more tightly than the natural substrate and inhibits nucleotide base synthesis.

In some embodiments, the effector molecule is a chemical, *e.g*., a chemical that modulates a cytosine (C) or an adenine(A) (*e.g*., Na bisulfite, ammonium bisulfite). In some embodiments, the effector molecule has enzymatic activity (methyl transferase, demethylase, nuclease (*e.g*., Cas9), a deaminase). In some embodiments, the effector molecule sterically hinders formation of an anchor sequence-mediated conjunction or binding of an RNA polymerase to a promoter.

The effector molecule with effector activity may be any one of the small molecules, peptides, fusion proteins, nucleic acids, nanoparticle, aptamers, or pharmacoagents with poor PK/PD described herein.

In some embodiments, the effector molecule is an inhibitor or "negative effector molecule". In the context of a negative effector molecule that modulates formation of an anchor sequence-mediated conjunction, in some embodiments, the negative effector molecule is characterized in that dimerization of an endogenous nucleating polypeptide is reduced when the negative effector molecule is present as compared with when it is absent. For example, in some embodiments, the negative effector molecule is or comprises a variant of the endogenous nucleating polypeptide's dimerization domain, or a dimerizing portion thereof.

For example, in certain embodiments, an anchor sequence-mediated conjunction is altered (*e.g.,* disrupted) by use of a dominant negative effector, *e.g.,* a protein that recognizes and binds an anchor sequence, (*e.g.,* a CTCF binding motif), but with an inactive (*e.g.,* mutated) dimerization domain, *e.g*., a dimerization domain that is unable to form a functional anchor sequence-mediated conjunction. For example, the Zinc Finger domain of CTCF can be altered so that it binds a specific anchor sequence (by adding zinc fingers that recognize flanking nucleic acids), while the homo-dimerization domain is altered to prevent the interaction between the engineered CTCF and endogenous forms of CTCF.

In some embodiments, the effector molecule comprises a synthetic conjunction nucleating molecule with a selected binding affinity for an anchor sequence within a target anchor sequence-mediated conjunction, (the binding affinity may be at least 10%, 15%, 20%, 25%, 30%, 35%, 40%, 45%, 50%, 55%, 60%, 65%, 70%, 75%, 80%, 85%, 90%, 95%, or higher or lower than the affinity of an endogenous conjunction nucleating molecule that associates with the target anchor sequence. The synthetic conjunction nucleating molecule may have between 30-90%, 30-85%, 30-80%, 30-70%, 50-80%, 50-90% amino acid sequence identity to the endogenous conjunction nucleating molecule). The conjunction nucleating molecule may disrupt, such as through competitive binding, the binding of an endogenous conjunction nucleating molecule to its anchor sequence. In some more embodiments, the conjunction nucleating molecule is engineered to bind a novel anchor sequence within the anchor sequence-mediated conjunction.

In some embodiments, a dominant negative effector molecule has a domain that recognizes specific DNA sequences (*e.g.,* an anchor sequence, a CTCF anchor sequence, flanked by sequences that confer sequence specificity), and a second domain that provides a steric presence in the vicinity of the anchoring sequence. The second domain may include a dominant negative conjunction nucleating molecule or fragment thereof, a polypeptide that interferes with conjunction nucleating molecule sequence recognition (*e.g*., the amino acid backbone of a peptide/nucleic acid or PNA), a nucleic acid sequence ligated to a small molecule that imparts steric interference, or any other combination of DNA recognition elements and a steric blocker.

In some embodiments, the effector molecule is an epigenetic modifying agent. Epigenetic modifying agents useful in the methods and compositions described herein include agents that affect, *e.g.,* DNA methylation, histone acetylation, and RNA-associated silencing. In some embodiments, the effectors sequence-specifically target an epigenetic enzyme (*e.g*., an enzyme that generates or removes epigenetic marks, *e.g*., acetylation and/or methylation). Exemplary epigenetic effectors may target an expression control region comprising, *e.g*., a transcriptional control element or an anchor sequence, by a site-specific disrupting agent comprising a site-specific targeting moiety.

In some embodiments, an effector molecule comprises one or more components of a gene editing system. Components of gene editing systems may be used in a variety of contexts including but not limited to gene editing. For example, such components may be used to target agents that physically modify, genetically modify, and/or epigenetically modify APOB sequences.

Exemplary gene editing systems include the clustered regulatory interspaced short palindromic repeat (CRISPR) system, zinc finger nucleases (ZFNs), and Transcription Activator-Like Effector-based Nucleases (TALEN). ZFNs, TALENs, and CRISPR-based methods are described, *e.g*., in Gaj et al. Trends Biotechnol. 31.7(2013):397-405; CRISPR methods of gene editing are described, *e.g.,* in Guan et al, Application of CRISPR-Cas system in gene therapy: Pre-clinical progress in animal model. DNA Repair 2016 July 30 [Epub ahead of print]; Zheng et al, Precise gene deletion and replacement using the CRISPR/Cas9 system in human cells. BioTechniques, Vol. 57, No. 3, September 2014, pp. 115-124; .

CRISPR systems are adaptive defense systems originally discovered in bacteria and archaea. CRISPR systems use RNA-guided nucleases termed CRISPR-associated or "Cas" endonucleases (e. g., Cas9 or Cpfl) to cleave foreign DNA. In a typical CRISPR/Cas system, an endonuclease is directed to a target nucleotide sequence (*e.g*., a site in the genome that is to be sequence-edited) by sequence-specific, non-coding "guide RNAs" that target single- or double-stranded DNA sequences. Three classes (I-III) of CRISPR systems have been identified. The class II CRISPR systems use a single Cas endonuclease (rather than multiple Cas proteins). One class II CRISPR system includes a type II Cas endonuclease such as Cas9, a CRISPR RNA ("crRNA"), and a trans-activating crRNA ("tracrRNA"). The crRNA contains a "guide RNA", typically about 20- nucleotide RNA sequence that corresponds to a target DNA sequence. The crRNA also contains a region that binds to the tracrRNA to form a partially double-stranded structure which is cleaved by RNase III, resulting in a crRNA/tracrRNA hybrid. The crRNA/tracrRNA hybrid then directs the Cas9 endonuclease to recognize and cleave the target DNA sequence. The target DNA sequence must generally be adjacent to a "protospacer adjacent motif ("PAM") that is specific for a given Cas endonuclease; however, PAM sequences appear throughout a given genome. CRISPR endonucleases identified from various prokaryotic species have unique PAM sequence requirements; examples of PAM sequences include 5'- NGG (Streptococcus pyogenes), 5'-NNAGAA (Streptococcus thermophilus CRISPR1), 5'-NGGNG (Streptococcus thermophilus CRISPR3), and 5'-NNNGATT (Neisseria meningiditis). Some endonucleases, e. g., Cas9 endonucleases, are associated with G-rich PAM sites, e. g., 5'-NGG, and perform blunt-end cleaving of the target DNA at a location 3 nucleotides upstream from (5 ' from) the PAM site. Another class II CRISPR system includes the type V endonuclease Cpfl, which is smaller than Cas9; examples include AsCpfl (from Acidaminococcus sp.) and LbCpfl (from Lachnospiraceae sp.). Cpf 1 -associated CRISPR arrays are processed into mature crRNAs without the requirement of a tracrRNA; in other words a Cpfl system requires only the Cpfl nuclease and a crRNA to cleave the target DNA sequence. Cpfl endonucleases, are associated with T-rich PAM sites, e. g., 5'-TTN. Cpfl can also recognize a 5'-CTA PAM motif. Cpfl cleaves the target DNA by introducing an offset or staggered double-strand break with a 4- or 5-nucleotide 5' overhang, for example, cleaving a target DNA with a 5- nucleotide offset or staggered cut located 18 nucleotides downstream from (3 ' from) from the PAM site on the coding strand and 23 nucleotides downstream from the PAM site on the complimentary strand; the 5-nucleotide overhang that results from such offset cleavage allows more precise genome editing by DNA insertion by homologous recombination than by insertion at blunt-end cleaved DNA. See, e. g., Zetsche et al. (2015) Cell, 163:759 - 771.

A variety of CRISPR associated (Cas) genes or proteins can be used in the present invention and the choice of Cas protein will depend upon the particular conditions of the method.

Specific examples of Cas proteins include class II systems including Cas1, Cas2, Cas3, Cas4, Cas5, Cas6, Cas7, Cas8, Cas9, CaslO, Cpfl, C2C1, or C2C3. In some embodiments, a Cas protein, *e.g.,* a Cas9 protein, may be from any of a variety of prokaryotic species. In some embodiments a particular Cas protein, *e.g.,* a particular Cas9 protein, is selected to recognize a particular protospacer-adjacent motif (PAM) sequence. In some embodiments, the site-specific targeting moiety includes a sequence targeting polypeptide, such as an enzyme, *e.g*., Cas9. In certain embodiments a Cas protein, *e.g.,* a Cas9 protein, may be obtained from a bacteria or archaea or synthesized using known methods. In certain embodiments, a Cas protein may be from a gram positive bacteria or a gram negative bacteria. In certain embodiments, a Cas protein may be from a Streptococcus, (*e.g*., a S. pyogenes, a S. thermophilus) a Crptococcus, a Corynebacterium, a Haemophilus, a Eubacterium, a Pasteurella, a Prevotella, a Veillonella, or a Marinobacter. In some embodiments nucleic acids encoding two or more different Cas proteins, or two or more Cas proteins, may be introduced into a cell, zygote, embryo, or animal, *e.g.,* to allow for recognition and modification of sites comprising the same, similar or different PAM motifs. In some embodiments, the Cas protein is modified to deactivate the nuclease, *e.g.*, nuclease-deficient Cas9, and to recruit transcription activators or repressors, *e.g.*, the co-subunit of the E. coli Pol, VP64, the activation domain of p65, KRAB, or SID4X, to induce epigenetic modifications, *e.g*., histone acetyltransferase, histone methyltransferase and demethylase, DNA methyltransferase and enzyme with a role in DNA demethylation (*e.g.*, the TET family enzymes catalyze oxidation of 5-methylcytosine to 5- hydroxymethylcytosine and higher oxidative derivatives).

For the purposes of gene editing, CRISPR arrays can be designed to contain one or multiple guide RNA sequences corresponding to a desired target DNA sequence; see, for example, Cong et al. (2013) Science, 339:819-823; Ran et al. (2013) Nature Protocols, 8:2281 - 2308. At least about 16 or 17 nucleotides of gRNA sequence are required by Cas9 for DNA cleavage to occur; for Cpfl at least about 16 nucleotides of gRNA sequence is needed to achieve detectable DNA cleavage.

Whereas wild-type Cas9 generates double-strand breaks (DSBs) at specific DNA sequences targeted by a gRNA, a number of CRISPR endonucleases having modified functionalities are available, for example: a "nickase" version of Cas9 generates only a single-strand break; a catalytically inactive Cas9 ("dCas9") does not cut the target DNA but interferes with transcription by steric hindrance. dCas9 can further be fused with a heterologous effector to repress (CRISPRi) or activate (CRISPRa) expression of a target gene. For example, Cas9 can be fused to a transcriptional silencer (*e.g.,* a KRAB domain) or a transcriptional activator (*e.g.,* a dCas9-VP64 fusion). A catalytically inactive Cas9 (dCas9) fused to Fokl nuclease ("dCas9-FokI") can be used to generate DSBs at target sequences homologous to two gRNAs. See, e. g., the numerous CRISPR/Cas9 plasmids disclosed in and publicly available from the Addgene repository (Addgene, 75 Sidney St., Suite 550A, Cambridge, MA 02139; addgene.org/crispr ). A "double nickase" Cas9 that introduces two separate double-strand breaks, each directed by a separate guide RNA, is described as achieving more accurate genome editing by Ran et al. (2013) Cell, 154: 1380 - 1389.

CRISPR technology for editing the genes of eukaryotes is disclosed in US Patent Application Publications 2016/0138008A1 and US2015/0344912A1, and in US Patents 8,697,359, 8,771,945, 8,945,839, 8,999,641, 8,993,233, 8,895,308, 8,865,406, 8,889,418, 8,871,445, 8,889,356, 8,932,814, 8,795,965, and 8,906,616. Cpfl endonuclease and corresponding guide RNAs and PAM sites are disclosed in US Patent Application Publication 2016/0208243 A1 .

In some embodiments, an effector comprises one or more components of a CRISPR system described hereinabove.

In some embodiments, suitable effectors for use in the agents, compositions, and methods of the invention include, for example, nucleases, physical blockers, epigenetic recruiters, *e.g*., a transcriptional enhancer or a transcriptional repressor, and epigenetic CpG modifiers, *e.g*., a DNA methylase, a DNA demethylase, a histone modifying agent, or a histone deacetylase, and combinations of any of the foregoing.

Exemplary effectors include ubiquitin, bicyclic peptides as ubiquitin ligase inhibitors, transcription factors, DNA and protein modification enzymes such as topoisomerases, topoisomerase inhibitors such as topotecan, DNA methyltransferases such as the DNMT family (*e.g*., DNMT3a, DNMT3b, DNMTL), protein methyltransferases (*e.g.*, viral lysine methyltransferase (vSET), protein-lysine N- methyltransferase (SMYD2), deaminases (*e.g*., APOB EC, UG1), histone methyltransferases such as enhancer of zeste homolog 2 (EZH2), PRMT1, histone-lysine-N-methyltransferase (Setdbl), histone methyltransferase (SET2), euchromatic histone-lysine N-methyltransferase 2 (G9a), histone-lysine N- methyltransferase (SUV39H1), and G9a), histone deacetylase (*e.g.,* HDACl, HDAC2, HDAC3), enzymes with a role in DNA demethylation (*e.g.,* the TET family enzymes catalyze oxidation of 5-methylcytosine to 5-hydroxymethylcytosine and higher oxidative derivatives), protein demethylases such as KDMIA and lysine-specific histone demethylase 1 (LSD1), helicases such as DHX9, acetyltransferases, deacetylases (*e.g*., sirtuin 1, 2, 3, 4, 5, 6, or 7), kinases, phosphatases, DNA-intercalating agents such as ethidium bromide, sybr green, and proflavine, efflux pump inhibitors such as peptidomimetics like phenylalanine arginyl-naphthylamide or quinoline derivatives, nuclear receptor activators and inhibitors, proteasome inhibitors, competitive inhibitors for enzymes such as those involved in lysosomal storage diseases, zinc finger proteins, TALENs, specific domains from proteins, such as a KRAB domain, a VP64 domain, a p300 domain (*e.g.,* p300 core domain), an MeCP2 domain, an MQ1 domain, a DNMT3a-3L domain, a TET1 domain, and/or TET2 domain, protein synthesis inhibitors, nucleases (*e.g*., Cpfl, Cas9, zinc finger nuclease), fusions of one or more thereof (*e.g*., dCas9-DNMT, dCas9-APOB EC, dCas9-UGl, dCas9-VP64, dCas9-p300 core, dCas9-KRAB, dCas9-KRAB-MeCP2, dCas9-MQ1, dCas9-DNMT3a-3L, and dCas9-TET1/TET2).

In some embodiments, a suitable nuclease for use in the agent, compositions, and methods of the invention comprises a Cas9 polypeptide, or enzymatically active portion thereof. In one embodiment, the Cas9 polypeptide, or enzymatically active portion thereof, further comprises a catalytically active domain of human exonuclease 1 (hEXO1), *e.g.,* 5' to 3' exonuclease activity and/or an RNase H activity. In other embodiments, a suitable nuclease comprises a transcription activator like effector nucleases (TALEN). In yet other embodiments, a suitable nuclease comprises a zinc finger protein.

The term TALEN, as used herein, is broad and includes a monomeric TALEN that can cleave double stranded DNA without assistance from another TALEN. The term TALEN is also used to refer to one or both members of a pair of TALENs that are engineered to work together to cleave DNA at the same site. TALENs that work together may be referred to as a left-TALEN and a right-TALEN, which references the handedness of DNA. See USSN 12/965,590; USSN 13/426,991 (US 8,450,471); USSN 13/427,040 (US 8,440,431); USSN 13/427,137 (US 8, 440,432); and USSN 13/738,381.

TAL effectors are proteins secreted by Xanthomonas bacteria. The DNA binding domain contains a highly conserved 33-34 amino acid sequence with the exception of the 12th and 13th amino acids. These two locations are highly variable (Repeat Variable Diresidue (RVD)) and show a strong correlation with specific nucleotide recognition. This simple relationship between amino acid sequence and DNA recognition has allowed for the engineering of specific DNA binding domains by selecting a combination of repeat segments containing the appropriate RVDs.

The non-specific DNA cleavage domain from the end of the FokI endonuclease can be used to construct hybrid nucleases that are active in a yeast assay. These reagents are also active in plant cells and in animal cells. Initial TALEN studies used the wild-type FokI cleavage domain, but some subsequent TALEN studies also used FokI cleavage domain variants with mutations designed to improve cleavage specificity and cleavage activity. The FokI domain functions as a dimer, requiring two constructs with unique DNA binding domains for sites in the target genome with proper orientation and spacing. Both the number of amino acid residues between the TALEN DNA binding domain and the FokI cleavage domain and the number of bases between the two individual TALEN binding sites are parameters for achieving high levels of activity. The number of amino acid residues between the TALEN DNA binding domain and the FokI cleavage domain may be modified by introduction of a spacer (distinct from the spacer sequence) between the plurality of TAL effector repeat sequences and the FokI endonuclease domain. The spacer sequence may be 12 to 30 nucleotides.

The relationship between amino acid sequence and DNA recognition of the TALEN binding domain allows for designable proteins. In this case artificial gene synthesis is problematic because of improper annealing of the repetitive sequence found in the TALE binding domain. One solution to this is to use a publicly available software program (DNAWorks) to calculate oligonucleotides suitable for assembly in a two step PCR; oligonucleotide assembly followed by whole gene amplification. A number of modular assembly schemes for generating engineered TALE constructs have also been reported. Both methods offer a systematic approach to engineering DNA binding domains that is conceptually similar to the modular assembly method for generating zinc finger DNA recognition domains.

Once the TALEN genes have been assembled they are inserted into plasmids; the plasmids are then used to transfect the target cell where the gene products are expressed and enter the nucleus to access the genome. TALENs can be used to edit genomes by inducing double-strand breaks (DSB), which cells respond to with repair mechanisms. In this manner, they can be used to correct mutations in the genome which, for example, cause disease.

As used herein, a "zinc finger polypeptide" or "zinc finger protein" is a protein that binds to DNA, RNA and/or protein, in a sequence-specific manner, by virtue of a metal stabilized domain known as a zinc finger. Zinc finger proteins are nucleases having a DNA cleavage domain and a DNA binding zinc finger domain. Zinc finger polypeptides may be made by fusing the nonspecific DNA. cleavage domain of an endonuclease with site-specific DNA binding zinc finger domains. Such nucleases are powerful tools for gene editing and can be assembled to induce double strand breaks (DSBs) site-specifically into genomic DNA. ZFNs allow specific gene disruption as during DNA repair, the targeted genes can be disrupted via mutagenic non-homologous end joint (NHEJ) or modified via homologous recombination (HR) if a closely related DNA template is supplied.

Zinc finger nucleases are chimeric enzymes made by fusing the nonspecific DNA. cleavage domain of the endonuclease FokI with site-specific DNA binding zinc finger domains. Due to the flexible nature of zinc finger proteins (ZFPs), ZFNs can be assembled that induce double strand breaks (DSBs) site-specifically into genomic DNA. ZFNs allow specific gene disruption as during DNA repair, the targeted genes can be disrupted via mutagenic non-homologous end joint (NHEJ) or modified via homologous recombination (HR) if a closely related DNA template is supplied.

In some embodiments, a suitable physical blocker for use in the agent, compositions, and methods of the invention comprises a gRNA, antisense DNA, or triplex forming oligonucleotide (which may target an expression control unit) steric block a transcriptional control element or anchoring sequence. The gRNA recognizes specific DNA sequences and further includes sequences that interfere with, *e.g*., a conjunction nucleating molecule sequence to act as a steric blocker. In some embodiments, the gRNA is combined with one or more peptides, *e.g*., S-adenosyl methionine (SAM), that acts as a steric presence. In other embodiments, a physical blocker comprises an enzymatically inactive Cas9 polypeptide, or fragment thereof (*e.g*., dCas9).

In one embodiment, an epigenetic recruiter activates or enhances transcription of a target gene. In some embodiments, a suitable epigenetic recruiter for use in the agent, compositions, and methods of the invention comprises a VP64 domain or a p300 core domain.

In one embodiment, an epigenetic recruiter silences or represses transcription of a target gene. In some embodiments, a suitable epigenetic recruiter for use in the agent, compositions, and methods of the invention comprises a KRAB domain, or an MeCP2 domain.

In one embodiment, a suitable epigenetic recruiter for use in the agent, compositions, and methods of the invention comprises dCas9-VP64 fusion, a dCas9-p300 core fusion, a dCas9-KRAB fusion, or a dCas9-KRAB-MeCP2 fusion.

As used herein, "VP64" is a transcriptional activator composed of four tandem copies of VP16 (Herpes Simplex Viral Protein 16, amino acids 437-447*: DALDDFDLDML) connected with glycine-serine (GS) linkers. In one embodiment, the VP64 further comprises the transcription factors p65 and Rta at the C terminus.

As used herein, "p300 core domain" refers to the catalytic core of the human acetyltransferase p300.

As used herein, "KRAB" refers to a Krüppel associated box (KRAB) transcriptional repression domain present in human zinc finger protein-based transcription factors (KRAB zinc finger proteins).

As used herein, MeCp2" refers to methyl CpG binding protein 2 which represses transcription, *e.g.,* by binding to a promoter comprising methylated DNA.

In one embodiment, an epigenetic CpG modifier methylates DNA and inactivates or represses transcription. In some embodiments, a suitable epigenetic CpG modifier for use in the agent, compositions, and methods of the invention comprises a MQ1 domain or a DNMT3a-3L domain.

In one embodiment, an epigenetic CpG modifier demethylates DNA and activates or stimulates transcription. In some embodiments, a suitable epigenetic recruiter for use in the agent, compositions, and methods of the invention comprises a TET1 or TET2 domain.

As used herein "MQ1" refers to a prokaryotic DNA methyltransferase.

As used herein "DNMT3a-3L" refers to a fusion of a DNA methyltransferase, Dnmt3a and a Dnmt3L which is catalytically inactive, but directly interacts with the catalytic domains of Dnmt3a.

As used herein "TET1" refers to "ten-eleven translocation methylcytosine dioxygenase 1," a member of the TET family of enzymes, encoded by the TET1 gene. TET1 is a dioxygenase that catalyzes the conversion of the modified DNA base 5-methylcytosine (5-mC) to 5-hydroxymethylcytosine (5-hmC) by oxidation of 5-mC in an iron and alpha-ketoglutarate dependent manner, the initial step of active DNA demethylation in mammals. Methylation at the C5 position of cytosine bases is an epigenetic modification of the mammalian genome which plays an important role in transcriptional regulation. In addition to its role in DNA demethylation, plays a more general role in chromatin regulation. Preferentially binds to CpG-rich sequences at promoters of both transcriptionally active and Polycomb-repressed genes. Involved in the recruitment of the O-GlcNAc transferase OGT to CpG-rich transcription start sites of active genes, thereby promoting histone H2B GlcNAcylation by OGT. Exemplary TET1 nucleotide and amino acid sequence can be found at GenBank Accession Nos.: NM_030625.3, NP_085128.2

As used herein, "TET2" refers to "ten-eleven translocation 2 (TET2)," a member of the TET family of enzymes, encoded by the TET1 gene. Similarly to TET1, TET2 is a dioxygenase that catalyzes the conversion of the modified genomic base 5-methylcytosine (5mC) into 5-hydroxymethylcytosine (5hmC) and plays a key role in active DNA demethylation. TET2 a preference for 5-hydroxymethylcytosine in CpG motifs. TET2 also mediates subsequent conversion of 5hmC into 5-formylcytosine (5fC), and conversion of 5fC to 5-carboxylcytosine (5caC). The conversion of 5mC into 5hmC, 5fC and 5caC probably constitutes the first step in cytosine demethylation. Methylation at the C5 position of cytosine bases is an epigenetic modification of the mammalian genome which plays an important role in transcriptional regulation. In addition to its role in DNA demethylation, also involved in the recruitment of the O-GlcNAc transferase OGT to CpG-rich transcription start sites of active genes, thereby promoting histone H2B GlcNAcylation by OGT. Exemplary nucleotide and amino acid sequence can be found at Genbank Accession No.: NM_001127208.2, NP_001120680.1

In some embodiments, a suitable epigenetic recruiter for use in the agent, compositions, and methods of the invention comprises a MQ1 domain, a DNMT3a-3L, a TET1 or TET2 domain. In one embodiment, a suitable epigenetic recruiter for use in the agent, compositions, and methods of the invention comprises a dCas9-MQ1 fusion, a dCas9-DNMT3a-3L fusion, or a dCas9-TET1 fusion or - dCase9-TET2 fusion.

### III. Delivery of a Site-Specific APOB Disrupting Agent of the Invention and Compositions Comprising a Site-Specific an APOB Disrupting Agents of the Invention

The delivery of the disrupting agents of the invention to a cell *e.g.,* a cell within a subject, such as a human subject (*e.g*., a subject in need thereof, such as a subject having an ApoB-associated disorder, *e.g*., cirrhosis) may be achieved in a number of different ways. For example, delivery may be performed by contacting a cell with a disrupting agent of the invention either *in vitro, ex vivo,* or *in vivo. In vivo* delivery may be performed directly by administering a composition, such as a lipid composition, comprising a disrupting agent to a subject. Alternatively, *in vivo* delivery may be performed indirectly by administering one or more vectors that encode and direct the expression of the disrupting agent. These alternatives are discussed further below.

In some embodiments, the disrupting agent comprises a nucleic acid molecule encoding a fusion protein, the fusion protein comprising a site-specific APOB targeting moiety, such as a polynucleotide encoding a DNA-binding domain of a Transcription activator-like effector (TALE) polypeptide or a zinc finger (ZNF) polypeptide, or fragment thereof, that specifically targets and binds to the APOB expression control region and an effector molecule., such as a VPR.

In other embodiments, the disrupting agent comprises a guide RNA and an mRNA encoding an effector molecule. The ratio of guide RNA to mRNA may be about 100:1 to about 1:100 (wt:wt).

In general, any method of delivery of a site-specific APOB disrupting agent of the invention (*in vitro, ex vivo,* or *in vivo*) may be adapted for use with the disrupting agents of the invention (see *e.g*., Akhtar S. and Julian RL., (1992) Trends Cell. Biol. 2(5):139-144 and WO94/02595). For *in vivo* delivery, factors to be considered for delivering a site-specific APOB disrupting agent of the invention include, for example, biological stability of the disrupting agent, prevention of non-specific effects, and accumulation of the disrupting agent in the target tissue. The non-specific effects of a disrupting agent can be minimized by local administration, for example, by direct injection or implantation into a tissue or topically administering a composition comprising the disrupting agent. Local administration to a treatment site maximizes local concentration of the disrupting agent, limits the exposure of the disrupting agent to systemic tissues that can otherwise be harmed by the disrupting agent or that can degrade the disrupting agent, and permits a lower total dose of the disrupting agent to be administered.

For administering a site-specific APOB disrupting agent systemically for the treatment of a disease, such as an APOB-associate disease, the disrupting agent, *e.g.,* a disrupting agent comprising a site-specific targeting moiety comprising a nucleic acid molecule, can be modified or alternatively delivered using a drug delivery system; both methods act to prevent the rapid degradation of a site-specific targeting moiety comprising a nucleic acid molecule by endo- and exo-nucleases *in vivo.* Modification of a disrupting agent comprising a site-specific targeting moiety comprising a nucleic acid molecule or a pharmaceutical carrier also permits targeting of the disrupting agent to a target tissue and avoidance of undesirable off-target effects. For example, a disrupting agent of the invention may be modified by chemical conjugation to lipophilic groups such as cholesterol to enhance cellular uptake and prevent degradation.

Alternatively, a disrupting agent of the invention may be delivered using a drug delivery system such as a nanoparticle, a dendrimer, a polymer, a liposome, or a cationic delivery system. Positively charged cationic delivery systems facilitate binding of disrupting agent (*e.g*., negatively charged molecule) and also enhance interactions at the negatively charged cell membrane to permit efficient uptake of a disrupting agent by the cell. Cationic lipids, dendrimers, or polymers can either be bound to a disrupting agent, or induced to form a vesicle or micelle (see *e.g.,* Kim SH. et al., (2008) Journal of Controlled Release 129(2):107-116) that encases the disrupting agent. The formation of vesicles or micelles further prevents degradation of the disrupting agent when administered systemically. Methods for making and administering cationic complexes are well within the abilities of one skilled in the art (see *e.g.,* Sorensen, DR., et al. (2003) J. Mol. Biol 327:761-766; Verma, UN. et al., (2003) Clin. Cancer Res. 9:1291-1300; Arnold, AS et al. (2007) J. Hypertens. 25: 197-205). Some non-limiting examples of drug delivery systems useful for systemic delivery of a distrupting agent of the invention include DOTAP (Sorensen, DR., et al (2003), supra; Verma, UN. *et al.,* (2003), supra), Oligofectamine, "solid nucleic acid lipid particles" (Zimmermann, TS. et al., (2006) Nature 441:111-114), cardiolipin (Chien, PY. et al., (2005) Cancer Gene Ther. 12:321-328; Pal, A. et al., (2005) Int J. Oncol. 26:1087-1091), polyethyleneimine (Bonnet ME. et al., (2008) Pharm. Res. Aug 16 Epub ahead of print; Aigner, A. (2006) J. Biomed. Biotechnol. 71659), Arg-Gly-Asp (RGD) peptides (Liu, S. (2006) Mol. Pharm. 3:472-487), and polyamidoamines (Tomalia, DA. et al., (2007) Biochem. Soc. Trans. 35:61-67; Yoo, H. et al., (1999) Pharm. Res. 16:1799-1804). In some embodiments, a disrupting agent (*e.g*., gRNA, or mRNA) forms a complex with cyclodextrin for systemic administration. Methods for administration and pharmaceutical compositions comprising cyclodextrins may be found in U.S. Patent No. 7,427,605.

The disrupting agents of the invention may be incorporated into pharmaceutical compositions suitable for administration. Such compositions typically include one or more species of disrupting agent and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The pharmaceutical compositions of the present invention may be administered in a number of ways depending upon whether local or systemic treatment is desired and upon the area to be treated. Administration may be topical (including ophthalmic, vaginal, rectal, intranasal, transdermal), oral, or parenteral. Parenteral administration includes intravenous drip, subcutaneous, intraperitoneal or intramuscular injection, or intrathecal or intraventricular administration.

The route and site of administration may be chosen to enhance delivery or targeting of the disrupting agent comprising a site-specific targeting moiety to a particular location. For example, to target liver cells, intravenous injection may be used. Lung cells may be targeted by administering the disrupting agent in aerosol form. Jejunum cells may be targeted by anal administration.

Formulations for topical administration may include transdermal patches, ointments, lotions, creams, gels, drops, suppositories, sprays, liquids, and powders. Conventional pharmaceutical carriers, aqueous, powder or oily bases, thickeners and the like may be necessary or desirable. Coated condoms, gloves and the like may also be useful.

Compositions for oral administration include powders or granules, suspensions or solutions in water, syrups, elixirs or non-aqueous media, tablets, capsules, lozenges, or troches. In the case of tablets, carriers that can be used include lactose, sodium citrate and salts of phosphoric acid. Various disintegrants such as starch, and lubricating agents such as magnesium stearate, sodium lauryl sulfate and talc, are commonly used in tablets. For oral administration in capsule form, useful diluents are lactose and high molecular weight polyethylene glycols. When aqueous suspensions are required for oral use, the nucleic acid compositions can be combined with emulsifying and suspending agents. If desired, certain sweetening or flavoring agents can be added.

Compositions for intravenous administration may include sterile aqueous solutions which may also contain buffers, diluents, and other suitable additives.

Formulations for parenteral administration may include sterile aqueous solutions which may also contain buffers, diluents, and other suitable additives. For intravenous use, the total concentration of solutes may be controlled to render the preparation isotonic.

In one embodiment, the administration of a disrupting agent composition of the invention is parenteral, *e.g.,* intravenous (*e.g.,* as a bolus or as a diffusible infusion), intradermal, intraperitoneal, intramuscular, intrathecal, intraventricular, intracranial, subcutaneous, transmucosal, buccal, sublingual, endoscopic, rectal, oral, vaginal, topical, pulmonary, intranasal, urethral, or ocular. Administration can be provided by the subject or by another person, *e.g.,* a health care provider. The composition may be provided in measured doses or in a dispenser which delivers a metered dose. Selected modes of delivery are discussed in more detail below.

In certain embodiments, the disrupting agents of the invention are polynucleotides, such as mRNAs, and are formulated in lipid nanoparticles (LNPs).

### A. Compositions Comprising a Site-Specific an APOB Disrupting Agent of the Invention

The site-specific APOB disrupting agents of the invention may be formulated into compositions, such as pharmaceutical compositions, using one or more excipients to: (1) increase stability; (2) increase cell transfection; (3) permit sustained or delayed release (*e.g*., from a depot formulation); (4) alter the biodistribution (*e.g*., target the disrupting agent to specific tissues or cell types); (5) increase the translation of an encoded protein *in vivo;* and/or (6) alter the release profile of an encoded protein *in vivo.* In addition to traditional excipients, such as any and all solvents, dispersion media, diluents, or other liquid vehicles, dispersion or suspension aids, surface active agents, isotonic agents, thickening or emulsifying agents, preservatives, excipients for use in the compositions of the invention may include, without limitation, lipidoids, liposomes, lipid nanoparticles, polymers, lipoplexes, core-shell nanoparticles, peptides, proteins, cells transfected with nucleic acid molecules, modified nucleic acid molecules, or RNA (*e.g.,* for transplantation into a subject), hyaluronidase, nanoparticle mimics and combinations thereof. Accordingly, the pharmaceutical compositions of the invention can include one or more excipients, each in an amount that together increases the stability of the disrupting agent, increases cell transfection by the disrupting agent, increases the expression of modified nucleic acid, or mRNA encoded protein, and/or alters the release profile of a disrupting agent. Further, the disrupting agents of the present invention may be formulated using self-assembled nucleic acid nanoparticles (see, *e.g.,* U.S. Patent Publication No. 2016/0038612A1.

### i. Lipidoid

The synthesis of lipidoids has been extensively described and formulations containing these compounds are particularly suited for delivery of a disrupting agent of the invention, such as a disrupting agent comprising a site-specific APOB targeting moiety comprising a nucleic acid molecule, *e.g.,* comprising modified nucleic acid molecules or mRNA (see Mahon et al., Bioconjug Chem. 2010 21:1448-1454; Schroeder et al., J Intern Med. 2010 267:9-21; Akinc et al., Nat Biotechnol. 2008 26:561-569; Love et al., Proc Natl Acad Sci USA. 201 0 107: 1864-1869; Siegwart et al., Proc Natl Acad Sci USA. 2011108: 12996-3001).

For example, lipidoids have been used to effectively deliver double stranded small interfering RNA molecules, single stranded nucleic acid molecules, modified nucleic acid molecules or modified mRNA. (*See, e.g*., US Patent Publication 2016/0038612A1). Complexes, micelles, liposomes or particles can be prepared containing these lipidoids and, therefore, provide effective delivery of a site-specific APOB targeting moiety comprising a nucleic acid molecule, as judged by the production of an encoded protein, following the administration of a lipidoid formulation, *e.g*., *via* localized and/or systemic administration. Lipidoid complexes of can be administered by various means including, but not limited to, intravenous, intramuscular, intradermal, intraperitoneal or subcutaneous routes.

*In vivo* delivery of a site-specific APOB targeting moiety comprising, *e.g.,* a nucleic acid molecule, may be affected by many parameters, including, but not limited to, the formulation composition, nature of particle PEGylation, degree of loading, polynucleotide to lipid ratio, and biophysical parameters such as, but not limited to, particle size (Akinc et al., Mol Ther. 2009 17:872-879). As an example, small changes in the anchor chain length of poly(ethylene glycol) (PEG) lipids may result in significant effects on *in vivo* efficacy. Formulations with different lipidoids, including, but not limited to penta[3 -(1-laury laminopropiony I) ]-triethy lenetetramine hydrochloride (TETA-5LAP; aka 98NI2-5, see Murugaiah et al., Analytical Biochemistry, 401:61 (2010), C12-200 (including derivatives and variants), and MDl, may be used.

In one embodiment, a disrupting agent comprising a site-specific APOB targeting moiety comprising, *e.g*., a nucleic acid molecule, is formulated with a lipidoid for systemic intravenous administration to target cells of the liver. For example, a final optimized intravenous formulation comprising a disrupting agent comprising a site-specific APOB targeting moiety comprising a nucleic acid molecule, and a lipid molar composition of 42% 98NI2-5, 48% cholesterol, and 10% PEG-lipid with a final weight ratio of about 7.5 to 1 total lipid to nucleic acid molecule, and a C14 alkyl chain length on the PEG lipid, with a mean particle size of roughly 50-60 nm, can result in the distribution of the formulation to be greater than 90% to the liver (see, Akinc et al., Mol Ther. 2009 17:872-879). In another example, an intravenous formulation using a C12-200 lipidoid (see, *e.g.,* PCT Publication No. WO 2010/129709) having a molar ratio of 50/10/38.5/1.5 of C12-200/disteroylphosphatidyl choline/cholesteroI/PEG-DMG, with a weight ratio of 7 to 1 total lipid to nucleic acid molecule, and a mean particle size of 80 nm may be used to deliver a disrupting agent comprising a site-specific APOB targeting moiety comprising a nucleic acid molecule, to hepatocytes (see, Love et al., Proc Natl Acad Sci USA. 2010 107: 1864-1869). In another embodiment, an MDl lipidoid-containing formulation may be used to effectively deliver a disrupting agent comprising a site-specific APOB targeting moiety comprising a nucleic acid molecule, to hepatocytes *in vivo.* The characteristics of optimized lipidoid formulations for intramuscular or subcutaneous routes may vary significantly depending on the target cell type and the ability of formulations to diffuse through the extracellular matrix into the blood stream. While a particle size of less than 150 nm may be desired for effective hepatocyte delivery due to the size of the endothelial fenestrae (see, Akinc et al., Mol Ther. 2009 17:872-879), use of lipidoid-formulated nucleic acid molecules to deliver the formulation to other cells types including, but not limited to, endothelial cells, myeloid cells, and muscle cells may not be similarly size-limited. Use of lipidoid formulations to deliver siRNA in vivo to other non-hepatocyte cells such as myeloid cells and endothelium has been reported (see Akinc et al., Nat Biotechnol. 200826:561-569; Leuschner et al., Nat Biotechnol. 2011 29: 1005-101 0; Cho et al. Adv. Funct. Mater. 2009 19 :3112-3118; 8th International Judah Folkman Conference, Cambridge, Mass. Oct. 8-9, 2010). For delivery to myeloid cells, such as monocytes, lipidoid formulations may have a similar component molar ratio. Different ratios of lipidoids and other components including, but not limited to, disteroylphosphatidyl choline, cholesterol and PEG-DMG, may be used to optimize the formulation for delivery to different cell types including, but not limited to, hepatocytes, myeloid cells, muscle cells, *etc.* For example, the component molar ratio may include, but is not limited to, 50% CI2-200, 10% disteroylphosphatidyl choline, 38.5% cholesterol, and 1.5% PEG-DMG (see Leuschner et al., Nat Biotechnol 2011 29: 1005-101 0). The use of lipidoid formulations for the localized delivery to cells (such as, but not limited to, adipose cells and muscle cells) via either subcutaneous, intradermal or intramuscular delivery, may not require all of the formulation components desired for systemic delivery and, as such, may comprise only the lipidoid and a disrupting agent comprising comprising a site-specific APOB targeting moiety comprising, *e.g*., a nucleic acid molecule, as described herein.

Combinations of different lipidoids may be used to improve the efficacy of the formualtions by increasing cell transfection and/or increasing the translation of encoded protein contained therein(see Whitehead et al., Mol. Ther. 2011,19:1688-1694).

In one embodiment, the lipidoid may be prepared from the conjugate addition of alklamines to acrylates. As a non-limiting example, a lipidoid may be prepared by the methods described in PCT Patent Publication No. WO 2014/028487. In one embodiment, the lipidoid may comprise a compound having formula (I), formula (II), formula (III), formula (IV) or formula (V) as described in PCT Patent Publication No. WO 2014/028487. In one embodiment, the lipidoid may be biodegradable.

### ii. Liposomes, Lipoplexes, and Lipid Nanoparticles

A disrupting agent of the invention may be formulated using one or more liposomes, lipoplexes, or lipid nanoparticles. In one embodiment, pharmaceutical compositions of the invention include liposomes. Liposomes are artificially-prepared vesicles which are primarily composed of a lipid bilayer and may be used as a delivery vehicle for the administration of nutrients and pharmaceutical formulations. Liposomes may be of different sizes such as, but not limited to, a multilamellar vesicle (MLV) which may be hundreds of nanometers in diameter and may contain a series of concentric bilayers separated by narrow aqueous compartments, a small unicellular vesicle (SUV) which may be smaller than 50 nm in diameter, and a large unilamellar vesicle (LUV) which may be between 50 and 500 nm in diameter. Liposome design may include, but is not limited to, opsonins or ligands in order to improve the attachment of liposomes to unhealthy tissue or to activate events such as, but not limited to, endocytosis. Liposomes may contain a low or a high pH in order to improve the delivery of the pharmaceutical formulations. The formation of liposomes may depend on the physicochemical characteristics such as, but not limited to, the pharmaceutical formulation entrapped and the liposomal ingredients, the nature of the medium in which the lipid vesicles are dispersed, the effective concentration of the entrapped substance and its potential toxicity, any additional processes involved during the application and/or delivery of the vesicles, the optimization size, polydispersity and the shelf-life of the vesicles for the intended application, and the batch-to-batch reproducibility and possibility of large-scale production of safe and efficient liposomal products.

As a non-limiting example, liposomes, such as synthetic membrane vesicles, may be prepared by the methods, apparatus and devices described in U.S. Patent Publication Nos. 2013/0177638, 2013/0177637, 2013/0177636, 201/30177635, 2013/0177634, 2013/0177633, 2013/0183375, 2013/0183373, 2013/0183372 and 2016/0038612) and PCT Patent Publication No WO 2008/042973,

In one embodiment, a pharmaceutical composition described herein may include, without limitation, liposomes such as those formed from 1 ,2-dioleyloxy-N,N -dimethyl ami - nopropane (DODMA) liposomes, DiLa2 liposomes from Marina Biotech (Bothell, Wash.), 1,2-dilinoleyloxy-3-dimethylaminopropane (DLin-DMA), 2,2-dilinoleyl-4-(2-dimethylaminoethyl)-[ 1,3]-dioxolane (DLin-KC2-DMA), and MC3 (US20100324120) and liposomes which may deliver small molecule drugs such as, but not limited to, DOXIL^{®} from Janssen Biotech, Inc. (Horsham, Pa.). In one embodiment, a pharmaceutical composition described herein may include, without limitation, liposomes such as those formed from the synthesis of stabilized plasmid-lipid particles (SPLP) or stabilized nucleic acid lipid particle (SNALP) that have been previously described and shown to be suitable for oligonucleotide delivery *in vitro* and *in vivo* (*see* Wheeler et al. Gene Therapy. 1999 6:271-281; Zhang et al. Gene Therapy. 19996:1438-1447; Jeffs et al. Pharm Res. 2005 22:362-372; Morrissey et al., Nat Biotechnol. 2005 2:1002-1007; Zimmermann et al., Nature. 2006 441:111-114; Heyes et al. J Contr Rel. 2005 107 :276-287; Semple et al. Nature Biotech. 2010 28:172-176; Judge et al. J Clin Invest. 2009 119:661-673; deFougerolles Hum Gene Ther. 2008 19:125-132; U.S. Patent Publication Nos 2013/0122104, 2013/0303587, and 2016/0038612). The original manufacturing method of Wheeler *et al.* was a detergent dialysis method, which was later improved by Jeffs *et al.* and is referred to as the spontaneous vesicle formation method. The liposome formulations of the invention may be composed of 3 to 4 lipid components in addition a disrupting agent comprising a site-specific APOB targeting moiety. As an example a liposome of the invention can contain, but is not limited to, 55% cholesterol, 20% disteroylphosphatidyl choline (DSPC), 10% PEG-SDSG, and 15% 1,2-dioleyloxy-N,N-dimethylaminopropane (DODMA), as described by Jeffs *et al.* As another example, liposome formulationsof the invention may contain, but are not limited to, 48% cholesterol, 20% DSPC, 2% PEG-c-DMA, and 30% cationic lipid, where the cationic lipid can be 1,2-distearloxy-N,N-dimethylaminopropane (DSDMA), DODMA, DLin-DMA, or 1 ,2-dilinolenyloxy -3 -dimethy laminopropane (DLenDMA), as described by Heyes *et al.* In some embodiments, liposome formulations may comprise from about 25.0% cholesterol to about 40.0% cholesterol, from about 30.0% cholesterol to about 45.0% cholesterol, from about 35.0% cholesterol to about 50.0% cholesterol and/or from about 48.5% cholesterol to about 60% cholesterol. In another embodiment, formulations of the invention may comprise a percentage of cholesterol selected from the group consisting of 28.5%, 31.5%, 33.5%, 36.5%, 37.0%, 38.5%, 39.0% and 43.5%. In some embodiments, liposome formulations of the invention may comprise from about 5.0% to about 10.0% DSPC and/or from about 7.0% to about 15.0% DSPC.

In one embodiment, a pharmaceutical composition may include liposomes which may be formed to deliver a disrupting agent of the invention. The disrupting agent comprising a site-specific APOB targeting moiety comprising may be encapsulated by the liposome and/or it may be contained in an aqueous core which may then be encapsulated by the liposome (see, *e.g.,* PCT Patent Publication Nos. WO 2012/031046, WO 2012/031043, WO 2012/030901 and WO 2012/006378 and U.S. Patent Publication Nos. 2013/0189351, 2013/0195969 and 201/30202684).

In another embodiment, liposomes for use in the present invention may be formulated for targeted delivery. As a non-limiting example, the liposome may be formulated for targeted delivery to the liver. Such a liposome may include, but is not limited to, a liposome described in U.S. Patent Publication No. 2013/0195967.

In one embodiment, formulations comprising liposomes and a disrupting agent may be administered intramuscularly, intradermally, or intravenously.

In another embodiment, a lipid formulation of the invention may include at least one cationic lipid, a lipid which enhances transfection and a least one lipid which contains a hydrophilic head group linked to a lipid moiety (International Pub. No. WO2011076807 and U.S. Pub. No. 20110200582). In another embodiment, a lipid formulation of the invention is a lipid vesicle which may have crosslinks between functionalized lipid bilayers (see U.S Patent Publication No. 2012/0177724).

In one embodiment, a formulation comprising a disrupting agent is a lipid nanoparticle (LNP) which may comprise at least one lipid. The lipid may be selected from, but is not limited to, DLin-DMA, DLin-K-DMA, 98NI2-5, CI2-200, DLin-MC3-DMA, DLin-KC2-DMA, DODMA, PLGA, PEG, PEG-DMG, PEGylated lipids and amino alcohol lipids. In another aspect, the lipid may be a cationic lipid such as, but not limited to, DLin-DMA, DLin-D-DMA, DLin-MC3- DMA, DLin-KC2-DMA, DODMA and amino alcohol lipids. The amino alcohol cationic lipid may be the lipids described in and/or made by the methods described in U.S. Patent Publication No. 2013/0150625.

In one embodiment, the cationic lipid may be selected from, but not limited to, a cationic lipid described in PCT Publication Nos. WO 2012/040184, WO 2011/153120, WO 2011/149733, WO 2011/090965, WO 2011/043913, WO 2011/022460, WO 2012/061259, WO 2012/054365, WO 2012/044638, WO 2010/080724, WO 2010/21865, WO 2008/103276, WO 2013/086373 and WO 2013/086354, U.S. Patent Nos. 7,893,302, 7,404,969, 8,283, 333, 8,466,122 and 8,569,256, and U.S. Patent Publication Nos. 2010/0036115, 2012/0202871, 2013/0064894, 2013/0129785, 2013/0150625, 2013/0178541, 2013/0225836 and 2014/0039032. In another embodiment, the cationic lipid may be selected from, but not limited to, formula A described in PCT Publication Nos. WO 2012/040184, WO 0111/53120, WO 2011/149733, WO 2011/090965, WO 2011/043913, WO 2011/022460, WO 2012/061259, WO 2012/054365, WO 2012/044638 and WO 2013/116126 or U.S. Patent Publication Nos. 2013/0178541 and 2013/0225836. In yet another embodiment, the cationic lipid may be selected from, but not limited to, formula CLI-CLXXIX of PCT Publication No. WO 2008/103276, formula CLICLXXIX of U.S Patent No. 7,893,302, formula CLICLXXXXII of U.S. Patent No. 7,404,969 and formula I-VI of us Patent Publication No. 2010/0036115, formula I of U.S. Patent Publication No 2013/0123338.

In one embodiment, the cationic lipid may be synthesized by methods known in the art and/or as described in PCT Publication Nos. WO 2012/040184 WO 2011/153120, WO 2011/149733, WO 2011/090965: WO 2011/043913, WO 2011/022460, WO 2012/061259, WO 2012/054365, WO 2012/044638, WO 2010/080724, WO 2010/21865, WO 2013/126803, WO 2013/086373, and WO 2013/086354.

In one embodiment, the lipids which may be used in the formulations and/or for delivery of the disrupting agents described herein may be a cleavable lipid. As a non-limiting example, a cleavable lipid and/or pharmaceutical compositions comprising cleavable lipids include those described in PCT Patent Publication No. WO 2012/170889. As another non-limiting example, the cleavable lipid may be HGT400l, HGT4002, HGT4003, HGT4004 and/or HGT4005 as described in PCT Patent Publication No. WO 2012/170889.

In one embodiment, polymers which may be used in the formulation and/or delivery of the disrupting agents described herein may include, but is not limited to, poly( ethylene) glycol (PEG), polyethylenimine (PEI), dithiobis(succinimidylpropionate) (DSP), Dimethy 1-3,3' - dithiobispropionimidate (DTBP), poly( ethylene imine) biscarbamate (PEIC), poly(L-lysine) (PLL), histidine modified PLL, poly(N-vinylpyrrohdone) (PVP), poly(propylenimine (PPI), poly(amidoamine) (PAMAM), poly(amido ethylenimine) (SS-PAEI), triehtylenetetramine (TETA), poly(β-aminoester), poly( 4- hydroxy-L-proine ester) (PHP), poly(allylamine), poly( α-[4-aminobutyl]-L-glycolic acid (PAGA), Poly(D,L-lactic-coglycolid acid (PLGA), Poly(N-ethyl-4-vinylpyridinium bromide), poly(phosphazene)s (PPZ), poly(phosphoester)s (PPE), poly(phosphoramidate)s (PPA), poly(N-2-hydroxypropylmethacrylamide) (pHPMA), poly(2-( dimethylamino)ethyl methacrylate) (pDMAEMA), poly(2-aminoethyl propylene phosphate) PPE_EA), Chitosan, galactosylated chitosan, N-dodecylated chitosan, histone, collagen and dextran-spermine. In one embodiment, the polymer may be an inert polymer such as, but not limited to, PEG. In one embodiment, the polymer may be a cationic polymer such as, but not limited to, PEl, PLL, TETA, poly(allylamine), Poly(N -ethyl-4-vinylpyridinium bromide), pHPMA and pDMAEMA. In one embodiment, the polymer may be a biodegradable PEl such as, but not limited to, DSP, DTBP and PEIC. In one embodiment, the polymer may be biodegradable such as, but not limited to, histine modified PLL SSPAEI, poly(β-aminoester), PHP, PAGA, PLGA, PPZ, PPE, PPA and PPE-EA.

In one embodiment, an LNP formulation of the invention may be prepared according to the methods described in PCT Publication Nos. WO 2011/127255 or WO 2008/103276 As a non-limiting example, a disrupting agent comprising a site-specific APOB targeting moiety may be encapsulated in an LNP formulation as described in PCT Publication Nos. WO 2011/127255 and/or WO 2008/103276 As another non-limiting example, a disrupting agent comprising a site-specific APOB targeting moiety as described herein, may be formulated in a nanoparticle to be delivered by a parenteral route as described in U.S. Patent Publication No. 2012/0207845 and PCT Publication No. WO 2014/008334

In one embodiment, LNP formulations described herein may be administered intramusculary. The LNP formulation may comprise a cationic lipid described herein, such as, but not limited to, DLin-DMA, DLin-KC2-DMA, DLin-MC3-DMA, DODMA and C12-200.

In one embodiment, LNP formulations described herein comprising a disrupting agent as described herein, may be administered intradermally. The LNP formulation may comprise a cationic lipid described herein, such as, but not limited to, DLin-DMA, DLin-KC2-DMA, DLin-MC3-DMA, DODMA and C12-200.

The nanoparticle formulations may comprise conjugate, such as a phosphate conjugate, a polymer conjugates, a conjugate that enhances the delivery of nanoparticle as described in US Patent Publication No. US20160038612 A1.

In one embodiment, the lipid nanoparticle formulation comprises DLin-MC3-DMA as described in US Patent Publication No. US20100324120.

In one embodiment, the lipid nanoparticle comprises a lipid compound, or a pharmaceutically acceptable salt, tautomer or stereoisomer thereof, or a lipid nanoparticle formulation, as described in US Patent No.: US10723692B2, US Patent Publication Nos. US20200172472A1, US20200163878A1, US20200046838A1, US20190359556A1, US20190314524A1, US20190274968A1, US20190022247A1, US20180303925A1, US20180185516A1, US20160317676A1, International Patent Publication No.: WO20200146805A1, WO2020081938A1, WO2019089828A1, WO2019036030A1, WO2019036028A1, WO2019036008A1, WO 2018200943A1, WO2018191719A1, WO2018107026A1, WO2018081480A1 (Acuitas Therapeutics, Inc.).

In one embodiment, the lipid nanoparticle comprises an amino lipid, or a pharmaceutically acceptable salt, tautomer or stereoisomer thereof, or a lipid nanoparticle formulation, described by Tekmira Pharmaceuticals Corp. in US9139554B2, US9051567B2, US8883203B2, US Patent Publication US20110117125A1 In one particular example, the compound described in US9139554B2 is DLin-kC2-DMA.

In one embodiment, the lipid nanoparticle comprises an amino lipid, or a pharmaceutically acceptable salt, tautomer or stereoisomer thereof, or a lipid nanoparticle formulation, described by Arbutus Biopharma Corp. in US10561732B2, US9938236B2, US9687550B2, US Patent Publication US20190240354A1, US20170027658A1, WO2020097493A1, WO2020097520A1, WO2020097540A1, WO2020097548A1

Lipid nanoparticles may be engineered to alter the surface properties of particles so the lipid nanoparticles may penetrate the mucosal barrier. Mucus is located on mucosal tissue such as, but not limited to, oral *(e.g.,* the buccal and esophageal membranes and tonsil tissue), ophthalmic, gastrointestinal *(e.g.,* stomach, small intestine, large intestine, colon, rectum), nasal, respiratory *(e.g.,* nasal, pharyngeal, tracheal and bronchial membranes), genital (e.g., vaginal, cervical and urethral membranes). Nanoparticles larger than 10-200 nm which are preferred for higher drug encapsulation efficiency and the ability to provide the sustained delivery of a wide array of drugs have been thought to be too large to rapidly diffuse through mucosal barriers. Mucus is continuously secreted, shed, discarded or digested and recycled so most of the trapped particles may be removed from the mucosla tissue within seconds or within a few hours. Large polymeric nanoparticles (200 nm-500 nm in diameter) which have been coated densely with a low molecular weight polyethylene glycol (PEG) diffused through mucus only 4 to 6-fold lower than the same particles diffusing in water (Lai et al. PNAS 2007 104(5): 1482-487; Lai et al. Adv Drug Deliv Rev. 200961(2): 158-171 ). The transport of nanoparticles may be determined using rates of permeation and/or fluorescent microscopy techniques including, but not limited to, fluorescence recovery after photobleaching (FRAP) and high resolution multiple particle tracking (MPT). As a non-limiting example, compositions which can penetrate a mucosal barrier may be made as described in US Pat. No. 8,241,670 or International Patent Publication No. WO2013110028

In one embodiment, a disrupting agent comprising a site-specific APOB targeting moiety as described herein, is formulated as a lipoplex, such as, without limitation, the ATUPLEX^{™} system, the DACC system, the DBTC system and other siRNAlipoplex technology from Silence Therapeutics (London, United Kingdom), STEMFECFM from STEMGENT^{®} (Cambridge, Mass.), and polyethylenimine (PEl) or protamine- based targeted and non-targeted delivery of nucleic acids acids (Aleku et al. Cancer Res. 2008 68:9788-9798; Strumberg et al. Int J Clin Pharmacol Ther 2012 50:76-78; Santel et al., Gene Ther 2006 13:1222-1234; Santel et al., Gene Ther 200613:1360-1370; Gutbier et al., PulmPharmacol. Ther. 201023:334-344; Kaufmann et al. Microvasc Res 2010 80:286-293; Weide et al. J Immunother. 2009 32:498- 507; Weide et al. J Immnnother. 2008 31:180-188; Pascolo Expert Opin. Biol. Ther. 4:1285-1294; Fotin-Mleczek et al., 2011 J. Immunother. 34: 1-15; Song et al., Nature Biotechnol. 2005,23:709-717; Peer et al., Proc NatlAcad Sci USA. 2007 6; 104:4095-4100; deFougerolles Hum Gene Ther. 2008 19: 125-132

In one embodiment such formulations may also be constructed or compositions altered such that they passively or actively are directed to different cell types *in vivo,* including but not limited to hepatocytes, immune cells, tumor cells, endothelial cells, antigen presenting cells, and leukocytes (Akinc et al. Mol Ther. 2010 18:1357-1364; Song et al., Nat Biotechnol. 2005 23:709-717; Judge et al., J Clinlnvest. 2009 119:661-673; Kaufmann et al., Microvasc Res 2010 80:286- 293; Santel et al., Gene Ther 200613:1222-1234; Santel et al., Gene Ther 2006 13: 1360-1370; Gutbier et al., Pulm Pharmacol. Ther. 2010 23:334-344; Basha et al., Mol. Ther. 2011 19:2186-2200; Fenske and Cullis, Expert Opin Drug Deliv. 20085:25-44; Peer et al., Science. 2008 319:627-630; Peer and Lieberman, Gene Ther. 2011 18: 1127-1133 ). One example of passive targeting of formulations to liver cells includes the DLin-DMA, DLin-KC2-DMA and DLin-MC3-DMA-based lipid nanoparticle formulations which have been shown to bind to apolipoprotein E and promote binding and uptake of these formulations into hepatocytes *in vivo* (Akinc et al. Mol Ther. 2010 18: 1357 -1364 ). Formulations can also be selectively targeted through expression of different ligands on their surface as exemplified by, but not limited by, folate, transferrin, N-acetylgalactosamine (GaINAc), and antibody targeted approaches (Kolhatkar et al., Curr Drug Discov Technol. 2011 8: 197 -206; Musacchio and Torchilin, Front Biosci. 201116: 1388-1412; Yu et al., Mol Membr BioI. 2010 27:286-298; Patil et al., Crit Rev Ther Drug Carrier Syst. 2008 25: 1-61; Benoit et al., Biomacromolecules. 2011 12:2708-2714; Zhao et al., Expert Opin Drug Deliv. 2008 5:309-319;Akinc et al., Mol Ther. 2010 18:1357-1364; Srinivasan et al., Methods Mol BioI. 2012 820: 105-116; Ben-Arie et al., Methods Mol Biol. 2012 757:497-507; Peer 2010 J Control Release. 20:63-68; Peer et al., Proc Natl Acad Sci USA. 2007 104:4095-4100; Kim et al., Methods Mol BioI. 2011 721:339-353; Subramanya et al., Mol Ther. 2010 18:2028-2037; Song et al., Nat Biotechnol. 2005 23:709-717; Peer et al., Science. 2008 319:627 -630; Peer and Lieberman, Gene Ther. 2011 18:1127-1133 ).

In one embodiment, a disrupting agent comprising a site-specific APOB targeting moiety of the invention, may be formulated as a solid lipid nanoparticle. A solid lipid nanoparticle (SLN) may be spherical with an average diameter between 10 to 1000 nm. SLN possess a solid lipid core matrix that can solubilize lipophilic molecules and may be stabilized with surfactants and/or emulsifiers. In a further embodiment, the lipid nanoparticle may be a self-assembly lipid-polymer nanoparticle (see Zhang et al., ACS Nano, 2008, 2 (8), pp 1696-1702). As a non-limiting example, the SLN may be the SLN described in PCT Publication No. WO2013/105101 . As another non-limiting example, the SLN may be made by the methods or processes described in PCT Publication No. WO 2013/105101

Liposomes, lipoplexes, or lipid nanoparticles may be used to improve the efficacy of a disrupting agent comprising a site-specific APOB targeting moiety comprising, *e.g.,* a nucleic acid molecule, to direct protein production as these formulations may be able to increase cell transfection by a nucleic acid molecule; and/or increase the translation of encoded protein *(e.g.,* an effector of the invention). One such example involves the use of lipid encapsulation to enable the effective systemic delivery of polyplex plasmid DNA (Heyes et al., Mol Ther. 2007 15:713- 720 ). The liposomes, lipoplexes, or lipid nanoparticles of the invention may also increase the stability of a a disrupting agent comprising a site-specific APOB targeting moiety comprising, *e.g.,* a nucleic acid molecule. Liposomes, lipoplexes, or lipid nanoparticles are described in U.S. Patent Publication No. 2016/0038612

In one embodiment, a disrupting agent comprising a site-specific APOB targeting moiety comprising may be formulated for controlled release and/or targeted delivery. As used herein, "controlled release" refers to a pharmaceutical composition or compound release profile that conforms to a particular pattern of release to effect a therapeutic outcome. In one embodiment, a disrupting agent comprising a site-specific APOB targeting moiety, as described herein, may be encapsulated into a delivery agent described herein and/or known in the art for controlled release and/or targeted delivery. As used herein, the term "encapsulate" means to enclose, surround or encase. As it relates to the formulation of the compounds of the invention, encapsulation may be substantial, complete or partial. The term "substantially encapsulated" means that at least greater than 50, 60, 70, 80, 85, 90, 95, 96, 97, 98, 99, 99.9, 99.9 or greater than 99.999% of the pharmaceutical composition or disrupting agent of the invention may be enclosed, surrounded or encased within the delivery agent. "Partial encapsulation" or "partially encapsulated" means that less than 10, 10, 20, 30, 40 50 or less of the pharmaceutical composition or disrupting agent of the invention may be enclosed, surrounded or encased within the delivery agent. Advantageously, encapsulation may be determined by measuring the escape or the activity of the pharmaceutical composition or compound of the invention using fluorescence and/or electron micrograph. For example, at least 1, 5, 10, 20, 30, 40, 50, 60, 70, 80, 85, 90, 95, 96, 97, 98, 99, 99.9, 99.99 or greater than 99.99% of the pharmaceutical composition or disrupting agent of the invention are encapsulated in the delivery agent.

In one embodiment, a disrupting agent comprising a site-specific APOB targeting moiety comprising as described herein, may be encapsulated in a therapeutic nanoparticle (e.g., a therapeutic nanoparticle from BIND Therapeutics). Therapeutic nanoparticles may be formulated by methods described herein and known in the art such as, but not limited to, PCT Publication Nos. WO 2010/005740, WO 2010/030763, WO 2010/005721, WO 2010/005723, WO 2012/054923, U.S. Patent Publication Nos. 2201/10262491, 2010/0104645, 2010/0087337, 2010/0068285, 2011/0274759, 2010/0068286, 2012/0288541, 2013/0123351, 2013/0230567, 2013/0236500, 2013/0302433, 2013/0302432, 1013/0280339 and 2013/0251757, and U.S. Patent Nos. 8,206,747, 8,293,276 8,318,208, 8,318,211, 8,623,417, 8,617,608, 8,613,954, 8,613,951, 8,609,142, 8,603,534 and 8,563,041. In another embodiment, therapeutic polymer nanoparticles may be prepared by the methods described in U.S. Patent Publication No. 2012/0140790 As a non-limiting example, the therapeutic nanoparticle may comprise about 4 to about 25 weight percent of a disrupting agent and about 10 to about 99 weight percent of a diblock poly (lactic) acid-poly (ethylene)glycol copolymer comprising poly(lactic) acid as described in US Patent Publication No. 2013/0236500 (Bind) As another non-limiting example, the nanoparticle may comprise about 0.2 to about 35 weight percent of a disrupting agent and about 10 to about 99 weight percent of a diblock poly(lactic) acid-poly( ethylene )glycol copolymer as described in U.S. Patent Publication Nos. 2013/0280339 (Bind) and 2010251757 and U.S. Patent No. 8,652,528

In one embodiment, a disrupting agent formulated in therapeutic nanoparticles may be administered intramuscularly, intrademrally, or intravenously.

In one embodiment, a disrupting agent formulated in ACCURINS^{™} nanoparticles may be administered intramuscularly, intrademrally, or intravenously.

In one embodiment, a disrupting agent may be delivered in therapeutic nanoparticles having a high glass transition temperature such as, but not limited to, the nanoparticles described in US Patent Publication Nos. 2014/0030351 and 2011/0294717

In one embodiment, the therapeutic nanoparticle may be formulated for sustained release. As used herein, "sustained release" refers to a pharmaceutical composition or compound that conforms to a release rate over a specific period of time. The period of time may include, but is not limited to, hours, days, weeks, months and years. As a nonlimiting example, the sustained release nanoparticle may comprise a polymer and a disrupting agent of the present invention (see PCT Publication No. WO2010075072 and U.S. Patent Publication Nos. 2010/0216804, 2011/0217377, 2012/0201859, 2013/0243848 and 2013/0243827

In one embodiment, a disrupting agent of the invention may be encapsulated in, linked to and/or associated with synthetic nanocarriers. Synthetic nanocarriers include, but are not limited to, those described in PCT Publication Nos. WO 2010/005740, WO 2010/030763, WO 2012/13501, WO 2012/149252, WO 2012149255, WO 2012149259, WO 2012149265, WO 2012149268, WO 2012149282, WO 2012149301, WO 2012149393, WO 2012149405, WO 2012149411 and WO 2012149454 and US Patent Publication Nos. 20110262491, 20100104645, 20100087337, 20120244222 and US20130236533, and U.S. Patent No. 8,652,487. The synthetic nanocarriers may be formulated using methods known in the art and/or described herein. As a nonlimiting example, the synthetic nanocarriers may be formulated by the methods described in PCT Publication Nos. WO 2010005740, WO 2010030763 and WO 201213501 and US Patent Publication Nos. 20110262491, 20100104645, 20100087337 and 2012024422. In another embodiment, the synthetic nanocarrier formulations may be lyophilized by methods described in PCT Publication No. WO 2011072218 and U.S. Patent No. 8,211,473. In yet another embodiment, formulations of the present invention, including, but not limited to, synthetic nanocarriers, may be lyophilized or reconstituted by the methods described in US Patent Publication No. 20130230568.

In one embodiment, synthetic nanocarriers comprising a disrupting agent may be administered intramuscularly, intrademrally, or intravenously.

In some embodiments, a disrupting agent may be formulated for delivery using smaller LNPs. Such particles may comprise a diameter from below 0.1 µm up to 1000 µm such as, but not limited to, less than 0.1 µm, less than 1.0 µm, less than 5 µm, less than 10 µm, less than 15 µm, less than 20 µm, less than 25 µm, less than 30 µm, less than 35 µm, less than 40 µm, less than 50 µm, less than 55 µm, less than 60 µm, less than 65 µm, less than 70 µm, less than 75 µm, less than 80 µm, less than 85 µm, less than 90 µm, less than 95 µm, less than 100 µm, less than 125 µm, less than 150 µm, less than 175 µm, less than 200 µm, less than 225 µm, less than 250 µm, less than 275 µm, less than 300 µm, less than 325 µm, less than 350 µm, less than 375 µm, less than 400 µm, less than 425 µm, less than 450 µm, less than 475 µm, less than 500 µm, less than 525 µm, less than 550 µm, less than 575 µm, less than 600 µm, less than 625 µm, less than 650 µm, less than 675 µm, less than 700 µm, less than 725 µm, less than 750 µm, less than 775 µm, less than 800 µm, less than 825 µm, less than 850 µm, less than 875 µm, less than 900 µm, less than 925 µm, less than 950 µm, less than 975 µm.

In another embodiment, a disrupting agent may be formualetd for delivery using smaller LNPs which may comprise a diameter from about 1 nm to about 100 nm, from about 1 nm to about 10 nm, about 1 nm to about 20 nm, from about 1 nm to about 30 nm, from about 1 nm to about 40 nm, from about 1 nm to about 50 nm, from about 1 nm to about 60 nm, from about 1 nm to about 70 nm, from about 1 nm to about 80 nm, from about 1 nm to about 90 nm, from about 5 nm to about from 100 nm, from about 5 nm to about 10 nm, about 5 nm to about 20 nm, from about 5 nm to about 30 nm, from about 5 nm to about 40 nm, from about 5 nm to about 50 nm, from about 5 nm to about 60 nm, from about 5 nm to about 70 nm, from about 5 nm to about 80 nm, from about 5 nm to about 90 nm, about 10 to about 50 nm, from about 20 to about 50 nm, from about 30 to about 50 nm, from about 40 to about 50 nm, from about 20 to about 60 nm, from about 30 to about 60 nm, from about 40 to about 60 nm, from about 20 to about 70 nm, from about 30 to about 70 nm, from about 40 to about 70 nm, from about 50 to about 70 nm, from about 60 to about 70 nm, from about 20 to about 80 nm, from about 30 to about 80 nm, from about 40 to about 80 nm, from about 50 to about 80 nm, from about 60 to about 80 nm, from about 20 to about 90 nm, from about 30 to about 90 nm, from about 40 to about 90 nm, from about 50 to about 90 nm, from about 60 to about 90 nm and/or from about 70 to about 90 nm.

In one embodiment, a disrupting agent may be formulated in smaller LNPs and may be administered intramuscularly, intrademrally, or intravenously.

In one embodiment, a disrupting agent may be formulated for delivery using the drug encapsulating microspheres described in PCT Patent Publication No. WO 2013063468 or U.S. Patent No. 8,440,614 . In another aspect, the amino acid, peptide, polypeptide, lipids (APPL) are useful in delivering the disrupting agents of the invention to cells (see PCT Patent Publication No. WO 2013063468

In one aspect, the lipid nanoparticle may be a limit size lipid nanoparticle described in PCT Patent Publication No. WO 2013059922. The limit size lipid nanoparticle may comprise a lipid bilayer surrounding an aqueous core or a hydrophobic core; where the lipid bilayer may comprise a phospholipid such as, but not limited to, diacylphosphatidylcholine, a diacylphosphatidylethanolamine, a ceramide, a sphingomyelin, a dihydrosphingomyelin, a cephalin, a cerebroside, a C8-C20 fatty acid diacylphophatidylcholine, and I-palmitoyl-2-oIeoyl phosphatidylcholine (POPC). In another aspect the limit size lipid nanoparticle may comprise a polyethylene glycol-lipid such as, but not limited to, DLPEPEG, DMPE-PEG, DPPC-PEG and DSPE-PEG.

In one embodiment, a disrupting agent of the invention may be delivered, localized and/or concentrated in a specific location using the delivery methods described in PCT Patent Publication No. WO 2013063530 . As a non-limiting example, a subject may be administered an empty polymeric particle prior to, simultaneously with or after delivering the disrupting agent to the subject. The empty polymeric particle undergoes a change in volume once in contact with the subject and becomes lodged, embedded, immobilized or entrapped at a specific location in the subject.

In one embodiment, a disrupting agent may be formulated in an active substance release system (see *e.g.,* US Patent Publication No. 20130102545 ). The active substance release system may comprise 1) at least one nanoparticle bonded to an oligonucleotide inhibitor strand which is hybridized with a catalytically active nucleic acid and 2) a compound bonded to at least one substrate molecule bonded to a therapeutically active substance (e.g., a disrupting agent of the invention), where the therapeutically active substance is released by the cleavage of the substrate molecule by the catalytically active nucleic acid.

In one embodiment, the nanoparticles of the present invention may be water soluble nanoparticles such as, but not limited to, those described in PCT Publication No. WO 2013090601. The nanoparticles may be inorganic nanoparticles which have a compact and zwitterionic ligand in order to exhibit good water solubility. The nanoparticles may also have small hydrodynamic diameters (HD), stability with respect to time, pH, and salinity and a low level of non-specific protein binding.

In one embodiment, the nanoparticles of the present invention are stealth nanoparticles or target-specific stealth nanoparticles such as, but not limited to, those described in U.S. Patent Publication Nos. 20130172406 (Bind), US20130251817 (Bind), 2013251816 (Bind) and 20130251766 (Bind). The stealth nanoparticles may comprise a diblock copolymer and a chemotherapeutic agent. These stealth nanoparticles may be made by the methods described in us Patent Publication Nos. 20130172406, 20130251817, 2013251816 and 20130251766. As a non-limiting example, the stealth nanoparticles may target cancer cells such as the nanoparticles described in US Patent Publication Nos. 20130172406, 20130251817, 2013251816 and 20130251766

In one embodiment, stealth nanoparticles comprising a disrupting agent of the invention may be administered intramuscularly, intradermally, or intravenously.

In one embodiment, a disrupting agent of the inventionmay be formulated in and/or delivered in a lipid nanoparticle comprising a plurality of cationic lipids such as, but not limited to, the lipid nanoparticles described in US Patent Publication No. 20130017223. As a non-limiting example, the LNP formulation may comprise a first cationic lipid and a second cationic lipid. As another non-limiting example, the LNP formulation may comprise DLin-MC2-DMA and DLinMC4- DMA. As yet another non-limiting example, the LNP formulation may comprise DLin-MC3-DMA and CI2-200. In one embodiment, the LNP formulations comprising a plurality of cationic lipids (such as, but not limited to, those described in US Patent Publication No. US20130017223) and may be administered intramuscularly, intradermally, or intravenously.

In one embodiment, a disrupting agent as described herein, may be formulated in and/or delivered in a lipid nanoparticle comprising the cationic lipid DLin-MC3-DMA and the neutral lipid DOPE. The lipid nanoparticle may also comprise a PEG based lipid and a cholesterol or antioxidant. These lipid nanoparticle formulations comprising DLin-MC3-DMA and DOPE and a disrupting agent may be administered intramuscularly, intradermally, or intravenously.

In one embodiment, the lipid nanoparticle comprising DLin-MC3-DMA and DOPE may comprise a PEG lipid such as, but not limited to, pentaerythritol PEG ester tetrasuccinimidyl and pentaerythritol PEG ether tetra-thiol, PEGc- DOMG, PEG-DMG (1,2-Dimyristoyl-sn-glycerol, methoxypolyethylene Glycol), PEG-DSG (1,2-Distearoyl-snglycerol, methoxypolyethylene Glycol), PEG-DPG (1,2- Dipalmitoyl-sn-glycerol, methoxypolyethylene glycol), PEG-DSA (PEG coupled to 1,2-distearyloxypropyl-3- amine), PEG-DMA (PEG coupled to 1,2-dimyristyloxypropyl- 3-amine, PEG-c-DNA, PEG-c-DMA, PEG-S-DSG, PEG-c-DMA, PEG-DPG, PEG-DMG 2000 and those described herein and/or known in the art.

In one embodiment, the lipid nanoparticle comprising DLin-MC3-DMA and DOPE may include 0.5% to about 3.0%, from about 1.0% to about 3.5%, from about 1.5% to about 4.0%, from about 2.0% to about 4.5%, from about 2.5% to about 5.0% and/or from about 3.0% to about 6.0% of the lipid molar ratio of a PEG lipid.

In one embodiment, the lipid nanoparticle comprising DLin-MC3-DMA and DOPE may include 25.0% cholesterol to about 50.0% cholesterol, from about 30.0% cholesterol to about 45.0% cholesterol, from about 35.0% cholesterol to about 50.0% cholesterol and/or from about 48.5% cholesterol to about 60% cholesterol. In one embodiment, formulations may comprise a percentage of cholesterol selected from the group consisting of 28.5%, 31.5%, 33.5%, 36.5%, 37.0%, 38.5%, 39.0%, 43.5% and 48.5%.

In one embodiment, the lipid nanoparticle comprising DLin-MC3-DMA and DOPE may include 25.0% antioxidant to about 50.0% antioxidant, from about 30.0% antioxidant to about 45.0% antioxidant, from about 35.0% antioxidant to about 50.0% antioxidant and/or from about 48.5% antioxidant to about 60% antioxidant. In one embodiment, formulations may comprise a percentage of antioxidant selected from the group consisting of 28.5%, 31.5%, 33.5%, 36.5%, 37.0%, 38.5%, 39.0%, 43.5% and 48.5%.

The disrupting agent of the invention can be formulated using natural and/or synthetic polymers. Non-limiting examples of polymers which may be used for delivery include, but are not limited to, DYNAMIC POLYCONJUGATE ^{®} (Arrowhead Research Corp., Pasadena, Calif.) formulations from MIRUS^{®} Bio (Madison, Wis.) and Roche Madison (Madison, Wis.), PHASERXTM polymer formulations such as, without limitation, SMARTT POLYMER TECHNOLOGYTM (Seattle, Wash.), DMRIIDOPE, poloxamer, VAXFECTIN^{®} adjuvant from Vical (San Diego, Calif.), chitosan, cyclodextrin from Calando Pharmaceuticals (Pasadena, Calif.), dendrimers and poly(lactic-co-glycolic acid) (PLGA) polymers, RONDELTM (RNAi/Oligonucleotide Nanoparticle Delivery) polymers (Arrowhead Research Corporation, Pasadena, Calif.) and pH responsive co-block polymers such as, but not limited to, PHASERXTM (Seattle, Wash.).

The polymer formulations may permit the sustained or delayed release of a disrupting agent (e.g., following intramuscular, intradermal or subcutaneous injection). The altered release profile of the disrupting agent can result in, for example, translation of an encoded protein over an extended period of time. The polymer formulation may also be used to increase the stability of the disrupting agent. For example, biodegradable polymers have been previously used to protect nucleic acids other than modified mRNA from degradation and been shown to result in sustained release of payloads in vivo (Rozema et al., Proc Natl Acad Sci USA. 2007 104:12982-12887; Sullivan et al., Expert Opin Drug Deliv. 2010 7:1433-1446; Convertine et al., Biomacromolecules. 2010 Oct. 1; Chu et al., Acc Chern Res. 2012 Jan. 13; Manganiello et alet al., Biomaterials. 2012 33:2301-2309; Benoit et a!., Biomacromolecules. 2011 12:2708-2714; Singha et al., Nucleic Acid Ther. 2011 2: 133- 147; deFougerolles Hnm Gene Ther. 2008 19:125-132; Schaffert and Wagner, Gene Ther. 2008 16:1131-1138; Chaturvedi et al., Expert Opin Drug Deliv. 2011 8: 1455- 1468; Davis, Mol Pharm. 2009 6:659-668; Davis, Nature 201 0464: 1067 -1070

In one embodiment, the pharmaceutical compositions may be sustained release formulations. In a further embodiment, the sustained release formulations may be for subcutaneous delivery. Sustained release formulations may include, but are not limited to, PLGA microspheres, ethylene vinyl acetate (EVAc), poloxamer, GELSITE^{®} (Nanotherapeutics, Inc. Alachua, Fla.), HYLENEX^{®} (Halozyme Therapeutics, San Diego Calif.), surgical sealants such as fibrinogen polymers (Ethic on Inc. Cornelia, Ga.), TISSELL^{®} (Baxter International, Inc Deerfield, Ill.), PEG-based sealants, and COSEAL^{®} (Baxter International, Inc Deerfield, Ill. ).

### B. Vector Encoded Site-Specific APOB Disrupting Agents of the Invention

Disrupting agents comprising a site-specific APOB targeting moiety, e.g., comprising a nucleic acid molecule, may be expressed from transcription units inserted into DNA or RNA vectors (see, *e.g.,* Couture, A, et al., TIG. (1996), 12:5-10; WO 00/22113, WO 00/22114, and US 6,054,299). In some embodiment, expression is sustained (months or longer), depending upon the specific construct used and the target tissue or cell type. These transgenes can be introduced as a linear construct, a circular plasmid, or a viral vector, which can be an integrating or non-integrating vector. The transgene can also be constructed to permit it to be inherited as an extrachromosomal plasmid (Gassmann, et al., (1995) Proc. Natl. Acad. Sci. USA 92:1292). Different components of the disrupting agent, e.g., gRNA and effector, can be located on separate expression vectors that can be co-introduced (e.g., by transfection or infection) into a target cell. Alternatively, each individual component can be transcribed by promoters both of which are located on the same expression plasmid.

Delivery of a disrupting agent expressing vector can be systemic, such as by intravenous or intramuscular administration, by administration to target cells ex-planted from the patient followed by reintroduction into the patient, or by any other means that allows for introduction into a desired target cell.

In certain embodiment, the nucleic acids described herein or the nucleic acids encoding a protein described herein, *e.g.,* an effector, are incorporated into a vector, *e.g.,* a viral vector.

The individual strand or strands of a disrupting agent comprising a site-specific APOB targeting moiety comprising a nucleic acid molecule can be transcribed from a promoter in an expression vector. Where two separate strands are to be expressed to generate, for example, a dsRNA, two separate expression vectors can be co-introduced *(e.g.,* by transfection or infection) into a target cell. Alternatively, each individual strand of a nucleic acid molecule can be transcribed by promoters both of which are located on the same expression plasmid. In one embodiment, a nucleic acid molecule is expressed as inverted repeat polynucleotides joined by a linker polynucleotide sequence such that the nucleic acid molecule has a stem and loop structure.

Expression vectors are generally DNA plasmids or viral vectors. Expression vectors compatible with eukaryotic cells, preferably those compatible with vertebrate cells, can be used to produce recombinant constructs for the expression of a disrupting agent as described herein.

Constructs for the recombinant expression of a disrupting agent will generally require regulatory elements, *e.g.,* promoters, enhancers, *etc.,* to ensure the expression of the disrupting agent in target cells.

Expression of natural or synthetic nucleic acids is typically achieved by operably linking a nucleic acid encoding the nucleic acid of interest to a regulatory region, such as a promoter, and incorporating the construct into an expression vector. The vectors can be suitable for replication and integration in eukaryotes.

Regulatory regions, such as a promoter, suitable for operable linking to a nucleic acid molecules can be operably linked to a regulatory region such as a promoter. can be from any species. Any type of promoter can be operably linked to a nucleic acid sequence. Examples of promoters include, without limitation, tissue-specific promoters, constitutive promoters, and promoters responsive or unresponsive to a particular stimulus (e.g., inducible promoters). Additional promoter elements, e.g., enhancing sequences, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, individual elements can function either cooperatively or independently to activate transcription.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor- la (EF-la). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter.

Further, the present invention should not be limited to the use of constitutive promoters. Inducible promoters are also contemplated as part of the invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

Additional regulatory regions that may be useful in nucleic acid constructs, include, but are not limited to, transcription and translation terminators, initiation sequences, polyadenylation sequences, translation control sequences (e.g., an internal ribosome entry segment, IRES), enhancers, inducible elements, or introns. Such regulatory regions may not be necessary, although they may increase expression by affecting transcription, stability of the mRNA, translational efficiency, or the like. Such regulatory regions can be included in a nucleic acid construct as desired to obtain optimal expression of the nucleic acids in the cell(s). Sufficient expression, however, can sometimes be obtained without such additional elements.

The expression vector to be introduced can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate transcriptional control sequences to enable expression in the host cells. Useful selectable markers include, for example, antibiotic -resistance genes, such as neo and the like. Non-limiting examples of selectable markers include puromycin, ganciclovir, adenosine deaminase (ADA), aminoglycoside phosphotransferase (neo, G418, APH), dihydrofolate reductase (DHFR), hygromycin-B-phosphtransferase, thymidine kinase (TK), and xanthin-guanine phosphoribosyltransferase (XGPRT). Such markers are useful for selecting stable transformants in culture. Other selectable markers include fluorescent polypeptides, such as green fluorescent protein or yellow fluorescent protein.

Signal peptides may also be included and can be used such that an encoded polypeptide is directed to a particular cellular location *(e.g.,* the cell surface).

Reporter genes may be used for identifying potentially transfected cells and for evaluating the functionality of transcriptional control sequences. In general, a reporter gene is a gene that is not present in or expressed by the recipient source and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene *(e.g.,* Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

Other aspects to consider for vectors and constructs are known in the art.

In some embodiments, a vector, *e.g.,* a viral vector comprises a disrupting agent comprising a site-specific APOB targeting moiety comprising a nucleic acid molecule.

Viral vector systems which can be utilized with the methods and compositions described herein include, but are not limited to, (a) adenovirus vectors (e.g., an Ad5/F35 vector); (b) retrovirus vectors, including but not limited to lentiviral vectors (including integration competent or integration-defective lentiviral vectors), moloney murine leukemia virus, *etc.*; (c) adeno-associated virus vectors; (d) herpes simplex virus vectors; (e) SV 40 vectors; (f) polyoma virus vectors; (g) papilloma virus vectors; (h) picornavirus vectors; (i) pox virus vectors such as an orthopox, e.g., vaccinia virus vectors or avipox, e.g. canary pox or fowl pox; and (j) a helper-dependent or gutless adenovirus. Replication-defective viruses can also be advantageous. Different vectors will or will not become incorporated into the cells' genome. The constructs can include viral sequences for transfection, if desired. Alternatively, the construct can be incorporated into vectors capable of episomal replication, e.g. EPV and EBV vectors. See, *e.g.*, U.S. Patent Nos. 6,534,261; 6,607,882; 6,824,978; 6,933,113; 6,979,539; 7,013,219; and 7,163,824.

Vectors, including those derived from retroviruses such as adenoviruses and adeno-associated viruses and lentiviruses, are suitable tools to achieve long- term gene transfer since they allow long-term, stable integration of a transgene and its propagation in daughter cells. Examples of vectors include expression vectors, replication vectors, probe generation vectors, and sequencing vectors. The expression vector may be provided to a cell in the form of a viral vector. Viral vector technology is well known in the art, and described in a variety of virology and molecular biology manuals.

In one embodiment, a suitable viral vector for use in the present invention is an adeno-associated viral vector, such as a recombinant adeno-associate viral vector.

Recombinant adeno-associated virus vectors (rAAV) are gene delivery systems based on the defective and nonpathogenic parvovirus adeno-associated type 2 virus. All vectors are derived from a plasmid that retains only the AAV 145 bp inverted terminal repeats flanking the transgene expression cassette. Efficient gene transfer and stable transgene delivery due to integration into the genomes of the transduced cell are key features for this vector system. (Wagner et al., Lancet 351:9117 1702-3 (1998), Kearns et al., Gene Ther. 9:748-55 (1996)). AAV serotypes, including AAV1, AAV2, AAV3, AAV4, AAV5, AAV6, AAV7, AAV8 and AAV9, can be used in accordance with the present invention.

Replication-deficient recombinant adenoviral vectors (Ad) can be produced at high titer and readily infect a number of different cell types. Most adenovirus vectors are engineered such that a transgene replaces the Ad E1a, E1b, and/or E3 genes; subsequently the replication defective vector is propagated in human 293 cells that supply deleted gene function in trans. Ad vectors can transduce multiple types of tissues in vivo, including nondividing, differentiated cells such as those found in liver, kidney and muscle. Conventional Ad vectors have a large carrying capacity. An example of the use of an Ad vector in a clinical trial involved polynucleotide therapy for antitumor immunization with intramuscular injection (Sterman et al., Hum. Gene Ther. 7:1083-9 (1998)). Additional examples of the use of adenovirus vectors for gene transfer in clinical trials include Rosenecker et al., Infection 24:15-10 (1996); Sterman et al., Hum. Gene Ther. 9:7 1083-1089 (1998); Welsh et al., Hum. Gene Ther. 2:205-18 (1995); Alvarez et al., Hum. Gene Ther. 5:597-613 (1997); Topf et al., Gene Ther. 5:507-513 (1998); Sterman et al., Hum. Gene Ther. 7:1083-1089 (1998).

Packaging cells are used to form virus particles that are capable of infecting a host cell. Such cells include 293 cells, which package adenovirus, and ψ2 cells or PA317 cells, which package retrovirus. Viral vectors used in gene therapy are usually generated by a producer cell line that packages a nucleic acid vector into a viral particle. The vectors typically contain the minimal viral sequences required for packaging and subsequent integration into a host (if applicable), other viral sequences being replaced by an expression cassette encoding the protein to be expressed. The missing viral functions are supplied in trans by the packaging cell line. For example, AAV vectors used in gene therapy typically only possess inverted terminal repeat (ITR) sequences from the AAV genome which are required for packaging and integration into the host genome. Viral DNA is packaged in a cell line, which contains a helper plasmid encoding the other AAV genes, namely rep and cap, but lacking ITR sequences. The cell line is also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, e.g., heat treatment to which adenovirus is more sensitive than AAV.

### IV. Methods of the Disclosure

The presen disclosure also provides methods of use of the agents and compositions described herein to modulate expression of apolipoprotein B (APOB ) in a cell. The methods include contacting the cell with a site-specific APOB disrupting agent, the disrupting agent comprising a site-specific APOB targeting moiety which targets an APOB expression control region, and an effector molecule, thereby modulating expression of APOB in the cell. The site-specific disrupting agent, the effector, or both the site-specific disrupting agent and the effector may be present in a composition, such as a composition described above. In some embodiments, the site-specific disrupting agent and the effector are present in the same compositions. In other embodiments, the site-specific disrupting agent and the effector are present in different compositions.

Expression of APOB may be enhanced or reduced as compared to, for example, a cell that was not contacted with the site-specific APOB disrupting agent. Modulation in gene expression can be assessed by any methods known in the art. For example, a modulation in the expression may be determined by determining the mRNA expression level of a gene, *e.g.,* in a cell, a plurality of cells, and/or a tissue sample, using methods routine to one of ordinary skill in the art, e.g., northern blotting, qRT-PCR; by determining the protein level of a gene using methods routine to one of ordinary skill in the art, such as western blotting, immunological techniques.

The term "reduced" in the context of the level of APOB gene expression or APOB protein production in a subject, or a disease marker or symptom refers to a statistically significant decrease in such level. The decrease can be, for example, at least 20%, 25%, 30%, 35%, 40%, 45%, 50%, 55%, 60%, 65%, 70%, 75%, 80%, 85%, 90%, or 95%, or below the level of detection for the detection method. In certain embodiments, the expression of the target is normalized, *i.e.,* decreased towards or to a level accepted as within the range of normal for an individual without such disorder. As used here, "lower" in a subject can refer to lowering of gene expression or protein production in a cell in a subject does not require lowering of expression in all cells or tissues of a subject. For example, as used herein, lowering in a subject can include lowering of gene expression or protein production in the liver of a subject.

The term "reduced" can also be used in association with normalizing a symptom of a disease or condition, *i.e.* decreasing the difference between a level in a subject suffering from an APOB-associated disease towards or to a level in a normal subject not suffering from an APOB-associated disease. As used herein, if a disease is associated with an elevated value for a symptom, "normal" is considered to be the upper limit of normal. If a disease is associated with a decreased value for a symptom, "normal" is considered to be the lower limit of normal.

The term "enhanced" in the context of the level of APOB gene expression or APOB protein production in a subject, or a disease marker or symptom refers to a statistically significant increase in such level. The increase can be, for example, at least 20%, 25%, 30%, 35%, 40%, 45%, 50%, 55%, 60%, 65%, 70%, 75%, 80%, 85%, 90%, or 95%, or above the level of detection for the detection method. In certain embodiments, the expression of the target is normalized, *i.e.,* increase towards or to a level accepted as within the range of normal for an individual without such disorder. As used here, "higher" in a subject can refer to increasing gene expression or protein production in a cell in a subject does not require increasing expression in all cells or tissues of a subject. For example, as used herein, increasing in a subject can include increasing gene expression or protein production in the liver of a subject.

The term "enhanced" can also be used in association with normalizing a symptom of a disease or condition, *i.e.* increasing the difference between a level in a subject suffering from an APOB-associated disease towards or to a level in a normal subject not suffering from an APOB-associated disease. As used herein, if a disease is associated with an elevated value for a symptom, "normal" is considered to be the upper limit of normal. If a disease is associated with a decreased value for a symptom, "normal" is considered to be the lower limit of normal.

In some embodiments, a suitable cell for use in the methods of the disclosure a mammalian cell. In some embodiments, the cell is a somatic cell. In some embodiments, the cell is a primary cell. For example, in some embodiments, the cell is a mammalian somatic cell. In some embodiments, the mammalian somatic cell is a primary cell. In some embodiments, the mammalian somatic cell is a non-embryonic cell.

The step of contacting may be performed *in vitro, in vivo (i.e.,* the cell may be within a subject), or *ex vivo.* In some embodiments, contacting a cell is performed *ex vivo* and the methods further include, prior to the step of contacting, a step of removing the cell *(e.g.,* a mammalian cell) from a subject. In some embodiments, the methods further comprise, after the step of contacting, a step of (b) administering the cell (*e.g.*, mammalian cells) to a subject.

The *in vivo* methods may include administering to a subject an agent or composition of the invention.

The term "subject," as used herein refers to an organism, for example, a mammal (*e.g.*, a human, a non-human mammal, a non-human primate, a primate, a laboratory animal, a mouse, a rat, a hamster, a gerbil, a cat, or a a dog). In some embodiments a human subject is an adult, adolescent, or pediatric subject. In some embodiments, a subject had a disease or a condition. In some embodiments, the subject is suffering from a disease, disorder or condition, *e.g.,* a disease, disorder or condition that can be treated as provided herein. In some embodiments, a subject is susceptible to a disease, disorder, or condition; in some embodiments, a susceptible subject is predisposed to and/or shows an increased risk (as compared to the average risk observed in a reference subject or population) of developing the disease, disorder or condition. In some embodiments, a subject displays one or more symptoms of a disease, disorder or condition. In some embodiments, a subject does not display a particular symptom (e.g,. clinical manifestation of disease) or characteristic of a disease, disorder, or condition. In some embodiments, a subject does not display any symptom or characteristic of a disease, disorder, or condition. In some embodiments, a subject is a patient. In some embodiments, a subject is an individual to whom diagnosis and/or therapy is and/or has been administered.

Subjects that would benefit from the invention include subjects having an "APOB-associated disease" or a subject at risk of an "APOB-associated disease."

Thus, the present invention further provides APOB disrupting agenst for use in methods of treatment of a subject in need thereof. The treatments include administering an agent or composition of the invention to a subject, *e.g.*, a subject that would benefit from a modulation of APOB expression, such as a subject having an APOB-associated disease, in a therapeutically effective amount.

In addition, the present invention provides APOB distrupting agent for use in ethods for preventing at least one symptom in a subject that would benefit from a modulation of APOB expression, such as a subject having an APOB-associated disease, by administering to the subject an agent or composition of the invention in a prophylactically effective amount.

"Therapeutically effective amount," as used herein, is intended to include the amount of an agent or composition that, when administered to a patient for treating a subject having a APOB-associated disease, is sufficient to effect treatment of the disease (*e.g.*, by diminishing, ameliorating, or maintaining the existing disease or one or more symptoms of disease or its related comorbidities). The "therapeutically effective amount" may vary depending on the agent or composition, how it is administered, the disease and its severity and the history, age, weight, family history, genetic makeup, stage of pathological processes mediated by APOB gene expression, the types of preceding or concomitant treatments, if any, and other individual characteristics of the patient to be treated.

"Prophylactically effective amount," as used herein, is intended to include the amount of an agent or composition that, when administered to a subject who does not yet experience or display symptoms of an APOB-associated disease, but who may be predisposed to an APOB-associated disease, is sufficient to prevent or delay the development or progression of the disease or one or more symptoms of the disease for a clinically significant period of time. The "prophylactically effective amount" may vary depending on the agent or composition, how it is administered, the degree of risk of disease, and the history, age, weight, family history, genetic makeup, the types of preceding or concomitant treatments, if any, and other individual characteristics of the patient to be treated.

As used herein, "prevention" or "preventing," when used in reference to a disease, disorder or condition thereof, that would benefit from a reduction in expression of an APOB gene or production of APOB protein, refers to a reduction in the likelihood that a subject will develop a symptom associated with such a disease, disorder, or condition, e.g., a sign or symptom of APOB gene expression or APOB activity.

A "therapeutically-effective amount" or "prophylactically effective amount" also includes an amount of an agent or composition that produces some desired local or systemic effect at a reasonable benefit/risk ratio applicable to any treatment. Agents and compositions employed in the the present invention may be administered in a sufficient amount to produce a reasonable benefit/risk ratio applicable to such treatment. In some embodiments, a therapeutically effective amount or prophylactically effect amount tis administered in a single dose; in some embodiments, multiple unit doses are required to deliver a therapeutically or prophylactically effective amount.

As used herein, the phrase "symptoms are reduced" may be used when one or more symptoms of a particular disease, disorder or condition is reduced in magnitude (*e.g*., intensity, severity, *etc.*) and/or frequency. In some embodiments, a delay in the onset of a particular symptom is considered one form of reducing the frequency of that symptom.

When the subject to be treated is a mammal such as a human, the composition can be administered by any means known in the art including, but not limited to oral, intraperitoneal, or parenteral routes, including intracranial (*e.g.*, intraventricular, intraparenchymal, and intrathecal), intravenous, intramuscular, subcutaneous, transdermal, airway (aerosol), nasal, rectal, and topical (including buccal and sublingual) administration. In certain embodiments, the compositions are administered by intravenous infusion or injection. In certain embodiments, the compositions are administered by subcutaneous injection.

As used herein, the term " APOB-associated disease," is a disease or disorder that is caused by, or associated with APOB gene expression or APOB protein production. The term "APOB-associated disease" includes a disease, disorder or condition that would benefit from a decrease in APOB gene expression, replication, or protein activity. Non-limiting examples of APOB-associated diseases include, for example, dyslipidemia *(e.g.,* hyperlipidemia, high LDL cholesterol, low HDL cholesterol, hypertriglyceridemia, postprandial hypertriglyceridemia), disorders of glycemic control *(e.g.,* insulin resistance not related to immune response to insulin, type 2 diabetes), cardiovascular disease *(e.g.,* hypertension, endothelial cell dysfunction), metabolic syndrome, disease of lipid deposition or dysfunction *(e.g.,* adipocyte dysfunction, visceral adipose deposition, obesity), disease of elevated uric acid *(e.g.,* hyperuricemia, gout), and eating disorders such as excessive sugar craving. Further details regarding signs and symptoms of the various diseases or conditions are provided herein and are well known in the art.

Administration of the agents or composition for use according to the invention may result in a reduction of the severity, signs, symptoms, or markers of an APOB-associated disease or disorder in a patient with an APOB-associated disease or disorder. By "reduction" in this context is meant a statistically significant decrease in such level. The reduction (absolute reduction or reduction of the difference between the elevated level in the subject and a normal level) can be, for example, at least about 20%, 25%, 30%, 35%, 40%, 45%, 50%, 55%, 60%, 65%, 70%, 75%, 80%, 85%, 90%, or 95%, or to below the level of detection of the assay used.

Administration of the agents or compositions according to the invention may stably or transiently modulating expression of a target gene. In some embodiments, a modulation of expression persists for at least about 1 hr to about 30 days, or at least about 2 hrs, 6 hrs, 12 hrs, 18 hrs, 24 hrs, 2 days, 3, days, 4 days, 5 days, 6 days, 7 days, 8 days, 9 days, 10 days, 1 1 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, or longer or any time therebetween. In some other embodiments, a modulation of expression persists for no more than about 30 mins to about 7 days, or no more than about 1 hr, 2 hrs, 3 hrs, 4 hrs, 5 hrs, 6 hrs, 7 hrs, 8 hrs, 9 hrs, 10 hrs, 11 hrs, 12 hrs, 13 hrs, 14 hrs, 15 hrs, 16 hrs, 17 hrs, 18 hrs, 19 hrs, 20 hrs, 21 hrs, 22 hrs, 24 hrs, 36 hrs, 48 hrs, 60 hrs, 72 hrs, 4 days, 5 days, 6 days, 7 days, or any time therebetween.

The agents or compositions may be administered once to the subject or, alternatively, multiple administrations may be performed over a period of time. For example, two, three, four, five, or more administrations may be given to the subject during one treatment or over a period of time. In some embodiments, six, eight, ten, 12, 15 or 20 or more administrations may be given to the subject during one treatment or over a period of time as a treatment regimen.

In some embodiments, administrations may be given as needed, *e.g.,* for as long as symptoms associated with the disease, disorder or condition persist. In some embodiments, repeated administrations may be indicated for the remainder of the subject's life. Treatment periods may vary and could be, *e.g.,* one day, two days, three days, one week, two weeks, one month, two months, three months, six months, a year, or longer.

Efficacy of treatment or prevention of disease can be assessed, for example by measuring disease progression, disease remission, symptom severity, reduction in pain, quality of life, dose of a medication required to sustain a treatment effect, level of a disease marker, or any other measurable parameter appropriate for a given disease being treated or targeted for prevention. It is well within the ability of one skilled in the art to monitor efficacy of treatment or prevention by measuring any one of such parameters, or any combination of parameters. As discussed herein, the specific parameters to be measured depend on the APOB-associated disease that the subject is suffering from.

Comparisons of the later readings with the initial readings provide a physician an indication of whether the treatment is effective. It is well within the ability of one skilled in the art to monitor efficacy of treatment or prevention by measuring any one of such parameters, or any combination of parameters. In connection with the administration of an agent or composition, "effective against" a APOB-associated disorder indicates that administration in a clinically appropriate manner results in a beneficial effect for at least a statistically significant fraction of patients, such as a improvement of symptoms, a cure, a reduction in disease, extension of life, improvement in quality of life, or other effect generally recognized as positive by medical doctors familiar with treating APOB-associated disorders.

A treatment or preventive effect is evident when there is a statistically significant improvement in one or more parameters of disease status, or by a failure to worsen or to develop symptoms where they would otherwise be anticipated. As an example, a favorable change of at least 10% in a measurable parameter of disease, and preferably at least 20%, 30%, 40%, 50% or more can be indicative of effective treatment. Efficacy for a given agent or composition can also be judged using an experimental animal model for the given disease as known in the art. When using an experimental animal model, efficacy of treatment is evidenced when a statistically significant reduction in a marker or symptom is observed.

Alternatively, the efficacy can be measured by a reduction in the severity of disease as determined by one skilled in the art of diagnosis based on a clinically accepted disease severity grading scale. Any positive change resulting in *e.g.,* lessening of severity of disease measured using the appropriate scale, represents adequate treatment using an agent or composition as described herein.

As used herein, the terms "treating" or "treatment" refer to a beneficial or desired result including, but not limited to, alleviation or amelioration of one or more signs or symptoms associated with APOB gene expression or APOB protein production. "Treatment" can also mean prolonging survival as compared to expected survival in the absence of treatment.

The present invention is next described by means of the following examples. However, the use of these and other examples anywhere in the specification is illustrative only, and in no way limits the scope and meaning of the invention or of any exemplified form. The invention is not limited to any particular preferred embodiments described herein.

### EXAMPLES

### Example 1. Materials and Methods

The materials and methods described herein are used in Examples 2-4.

### Primary mouse hepatocyte culturing and treatments

Primary female mouse (CD-1) cryopreserved hepatocytes (PMH) were purchased from Gibco (Lot#: 770). Cells were thawed in specially formulated thawing medium (Gibco^{™}, CM7500). PMH were centrifuged at 60 g for 6 minutes and supernatant was discarded by decanting. The PMH pellet was re-suspended in specially formulated hepatocyte plating medium (Williams' E medium supplemented with 5% fetal bovine serum, 1 µM dexamethasone, 10,000 U/mL penicillin, 10,000 µg/mL streptomycin, 4 µg/mL human recombinant insulin, 2 mM GlutaMAX^{™} and 15 mM HEPES). Alive PMH were counted with trypan blue staining and cells were seeded into rat tail collagen-I coated 96-well plates at 30,000 alive PMH/well concentration. PMH were allowed to attach to the wells for 4 hours at 37 °C in a humidity-controlled incubator with 5% CO₂. The he medium was subsequently replaced with hepatocyte incubation medium (Williams' E medium supplemented with 0.1 µM dexamethasone, 10,000 U/mL penicillin, 10,000 µg/mL streptomycin, 1% ITS+, 2 mM GlutaMAX^{™} and 15 mM HEPES). Cells were incubated 24 hours before the treatments at 37 °C in humidity-controlled incubator under 5% CO₂ atmosphere.

For the treatments, PMH medium in the wells was replaced with hepatocyte incubation medium containing LNP formulations at various concentrations of total RNA (*i.e.,* the combination of sgRNA and Cas9 coding mRNA). Each concentration, starting from 20 µg/ml total RNA, was assayed in triplicate. Briefly, the LNPs were diluted in the incubation medium at the required concentrations in a separate 96-well plate. The LNPs were serially diluted in-plate two-fold using incubation medium. Then, 100 µl medium that contains LNPs from the preparation plate was transferred to the PMH plate. Cells were incubated with the formulations continuously for 72 hours. At the end of the treatment time, the medium in the wells was collected for ApoB ELISA and PMH were washed with PBS three times to remove the cell debris and medium. Genomic DNA and mRNA from the PMH were extracted using AllPrep DNA/RNA kit (Qiagen) and samples were processed further.

### Example 2. Editing of APOB Expression Control Region

This example describes the editing of an APOB expression control region with a site-specific APOB disrupting agent, comprising a site-specific APOB targeting moiety which targets an APOB expression control region, *i.e.,* a single guide RNA (sgRNA)=, and an effector comprising a Cas9. Without wishing to be bound by any theory, it was hypothesized that the targeting moiety guides the effector to the target site and effector comprising Cas9 cleaves the genomic DNA at the APOB gene expression control region. Non-homologous end joining (NHEJ) repair of Cas9-induced breaks leaves a variety of different mutations, which can be detected by sequencing or other methods. Thus, the site specific disrupting agent edits the genomic DNA at the target expression control region. Some of the edits are productive, that is, these edits (*i.e.*, mutations) disrupt the function of expression regulatory sequences, *e.g.,* disrupts the formation of a topological configuration *(e.g.,* a loop) mediated by CTCF sites.

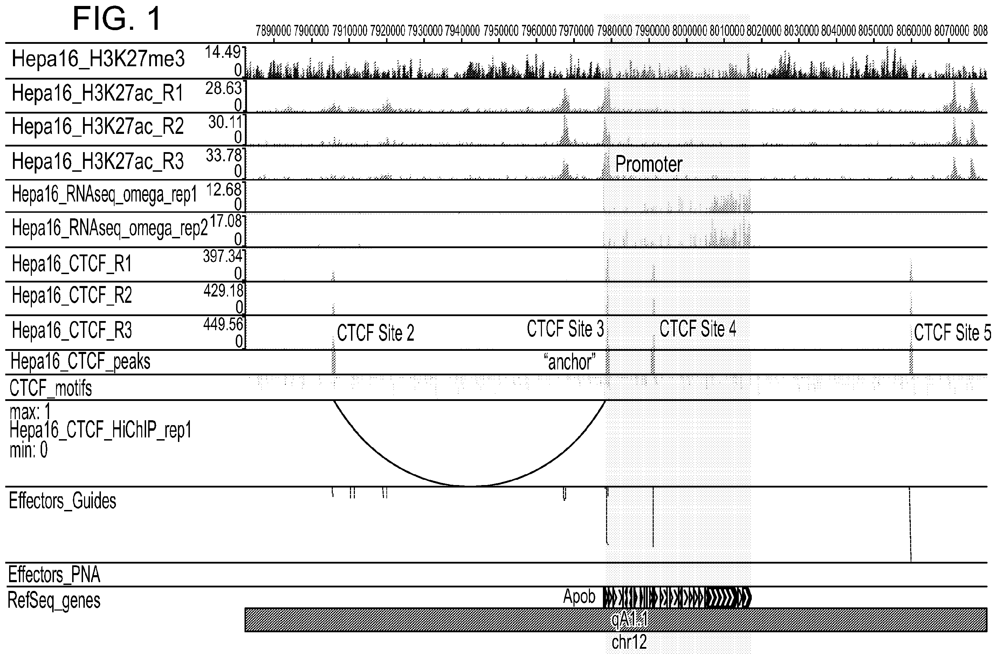

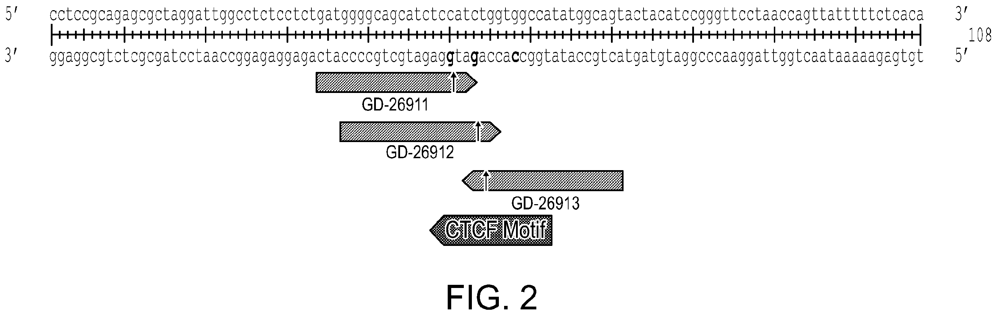

Guide RNAs were designed to site-specifically target the transcriptional control region comprising CTCF anchor site 3 (near the transcriptional start site) of the APOB gene (see, *e.g.*, Figures 1 and 2) and synthesized according to standard methods for oligonucleotide synthesis. The nucleotide sequences of the single guide RNAs are provided in Table 2.

Cell culture and transfection were conducted as described in Example 1.

LNP formulations were prepared using individual sgRNA (see, Figure 2) and Cas9 coding mRNA. The LNP formulations were added to the cells for a final concentration of total RNA (*i.e.,* sgRNA and mRNA) of 20.00, 10.00, 5.00, 2.50, 1.25, or 0.630 µg/ml. The experiment was ended after 72 hours. The supernatant of the cell cultures were collected for protein assay. The cells were lysed for downstream mRNA and/or DNA purification. A non target sgRNA, GD-23150, was used as negative control. GD-23150 has the sequence as follows:

GTATTACTGATATTGGTGGGGTTTTAGAGCTAGAAATAGCAAGTTAAAATAAGGCTAGTC
CGTTATCAACTTGAAAAAGTGGCACCGAGTCGGTGCTTTT

The editing of the APOB expression control region was analyzed using a T7E1 assay and a sequencing-based assay. The assays were performed according to well-known protocols . Briefly, genomic DNA from the PMH (above) was amplified using primers for the CTCF site 3 region and sequenced by amplicon sequencing (MiSeq, Illumina). The primers used have the sequences below:
Forward: CCCCTCCACCCCTTCCTATT
Reverse: CAAAGGGACAGGGCTAAGTGT
Forward adapter sequence: CACTCTTTCCCTACACGACGCTCTTCCGATCT
Reverse adapter sequence: GGAGTTCAGACGTGTGCTCTTCCGATCT

The wild type amplicon, *i.e.,* the amplicon that does not contain mutations generated via Cas9 editing, has the sequence as shown below:

CCCCTCCACCCCTTCCTATTTACTCTACCCTAGGGGTCAGAGGATCAGGCTTTGCC
GCAATACCCAGCTTCCTCCGCAGAGCGCTAGGATTGGCCTCTCCTCTGATGGGGCAGCAT
CTCCATCTGGTGGCCATATGGCAGTACTACATCCGGGTTCCTAACCAGTTATTTTCTCAC
AATCAAAAAATTCCTGTCATGGACAGTGGAATCTGCCAGAAGTTTCTTTTATGGAGGGG
GTTTCTAGAAGTCAACCAGACAGCCTTAACAGCTAACTTTGTGTCCGGTACTTCATAGGT
TCTAGTCACGATAGCATTTTCAAACTGAGAAAACACTTAGCCCTGTCCCTTTG.

The PCR products were purified and subjected to T7 endonuclease digestion or amplicon sequencing. T7 endonuclease cleaves double-stranded DNA at positions of mismatches, which were caused by the mutation generated by NHEJ repair of Cas9-induced breaks at the target site.

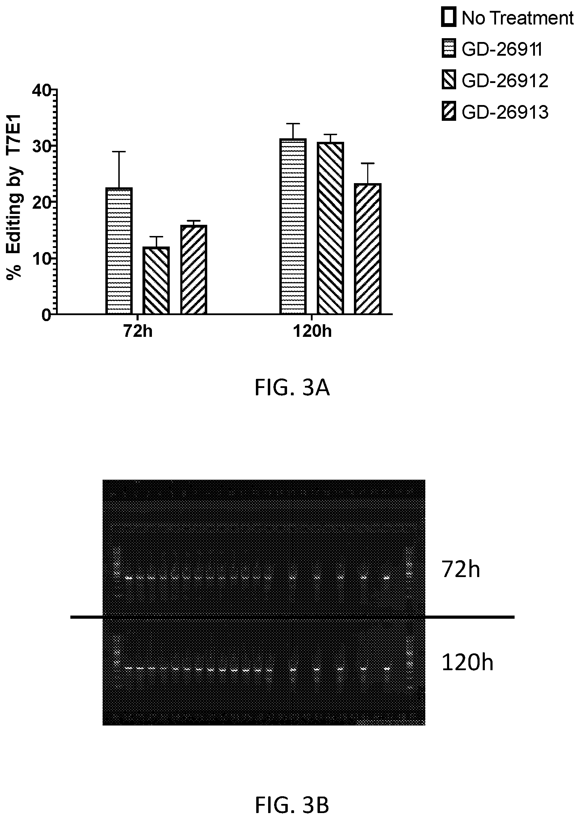

As shown in Figures 3A - 3D, the sgRNAs in combination with Cas9 edited the expression control region of APOB gene. The materials and methods for the experiments in Figures 3A to 3D were slightly different than those described in Example 1. Briefly, after the thawing of the PMH cells, alive PMH were counted with trypan blue staining and cells were seeded into rat tail collagen-I coated 24-well plates at 180,000 alive PMH/well concentration. PMH were allowed to attach to the wells for 4 hours at 37 °C in a humidity-controlled incubator with 5% CO₂. Then, the medium was replaced with hepatocyte incubation medium (Williams' E medium supplemented with 0.1 µM dexamethasone, 10,000 U/mL penicillin, 10,000 µg/mL streptomycin, 1% ITS+, 2 mM GlutaMAX^{™} and 15 mM HEPES). Cells were incubated for 24 hours before the treatments were conducted at 37 °C in humidity-controlled incubator under 5% CO₂ atmosphere.

For the treatments, PMH medium in the wells was replaced with hepatocyte incubation medium containing LNP formulations at 5 µg/ml total RNA concentration based on the results explained elsewhere herein. PMH treatments were done in triplicate. Briefly, the LNPs were diluted in the incubation medium at the required concentrations in a separate 24-well plate. The medium from the wells of PMH plates were collected. Then, 500 µl medium that contained LNPs from the preparation plate was transferred to the PMH plate. Cells were incubated with the formulations for 24 hours. Then, the medium from the wells were collected for ApoB ELISA and fresh incubation medium without LNPs were added on to cells. Forty-eight hours and 96 hours later (corresponding to 72 hours and 120 hours after the treatments, respectively), the medium in the wells were collected PMH were washed with PBS three times to remove the cell debris and medium. Then, the genomic DNA and mRNA from the PMH were extracted using AllPrep DNA/RNA kit (Qiagen) and samples were processed further. The PCR primers and the expected wild type amplicon sequence were the same as described above.

As shown in Figures 3A and 3B, the sgRNA in combination with Cas9 coding mRNA edited the expression control region of the APOB gene. Figure 3A shows the percentage of Cas9-induced editing of the expression control region for the indicated sgRNA and Cas9 coding mRNA at a concentration of 5 µg/ml as determined by T7E1 assay. Figure 3B is a representative electrophoresis gel image showing that PCR products cleaveable by T7 endonuclease were not present on the gel.

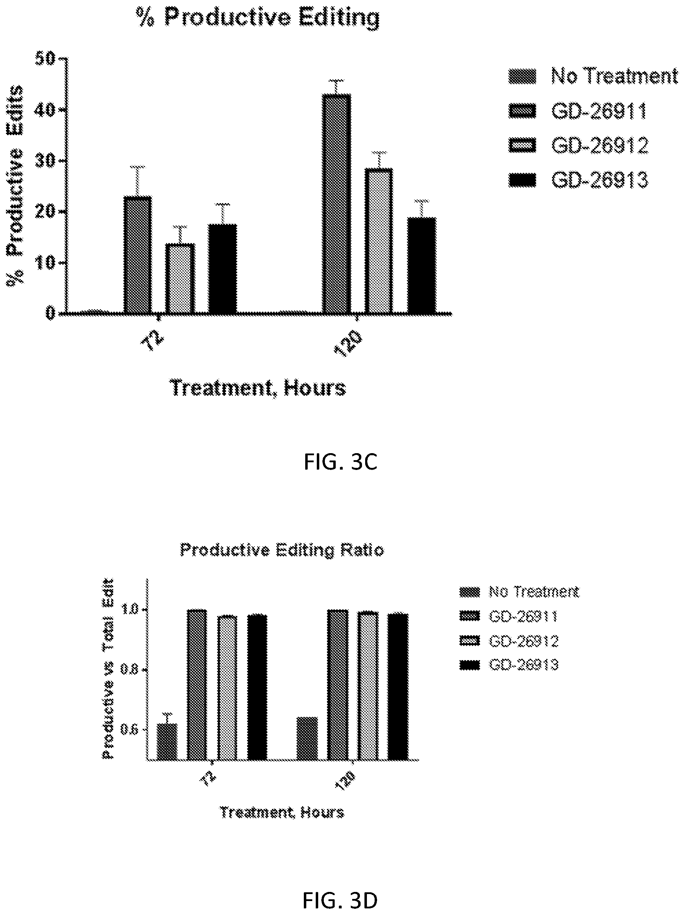

As shown in Figures 3C and 3D, the sgRNA in combination with Cas9 edited expression control region of APOB gene. Figure 3C shows the percentage of Cas9-induced productive editing of the expression control region of the indicated sgRNA and Cas9 coding mRNA at a concentration of 5 µg/ml of. Figure 3D shows the ratio of productive editing of the indicated sgRNA.

### Example 3. Modulation of APOB Expression - mRNA

This example describes repression of APOB expression, as measured by the reduction of APOB mRNA level, using a site-specific APOB disrupting agent, comprising a site-specific APOB targeting moiety which targets an APOB expression control region, *i.e.,* a sgRNA, and an effector comprising a Cas9.

Cells were cultured and transfected with sgRNA and Cas9 coding mRNA as described in Example 1. An APOB specific siRNA was used as a positive control and added to the cell culture at 0.63 ng/ml. The siRNA has the sequences as follows.

| | |
|---|---|
| Guide Strand: | 5'-AUUUfCfaggaaUfUfgUfUfaaaguu-3' |
| Passenger Strand: | 5'-iBCfUfUfUfdAdACfdAdAUfUfCfCfUfdGdAdAdAUfdTdTiB-3' |

N: RNA residues n: 2'-O-methyl residues Nf: 2'-F residues dT: desoxy-T iB: inverted abasic residue

The ApoB mRNA from the PMH treatment was analyzed by qPCR using the RNA extract obtained from the treated cells. β-actin was used as a housekeeping positive control for calculating relative ApoB mRNA levels.

In a preliminary study, data from all concentrations in the treatment groups were used for determining that the treatments did not have a negative effect on the β-actin levels as an indicator of the overall cell health (data not shown). According to those results, it was determined that total RNA concentrations (*i.e.*, the combination of sgRNA and Cas9 coding mRNA) above 5 µg/ml had negative impact on the housekeeping gene levels and thus, deemed to cause cell stress and discarded from further analysis.

Accordingly, Figures 4A-4D depict the relative ApoB levels of the treatment groups within the total RNA concentrations that were accepted as safe based on the preliminary study. As shown in Figures 4A-4D, sgRNA in combination with Cas9 coding mRNA reduced the expression of APOB gene in cultured PMHs as measured by reduction in mRNA levels. Figures 4A-4D are graphs depicting that certain site-specific APOB target moieties (GD-26911 and GD-26912) in combination with Cas9 coding mRNA resulted in a reduction of over 50% in ApoB expression with total RNA (*i.e.,* combination of sgRNA and Cas9 coding mRNA) concentration at 5µg/ml. The graphs show APOB mRNA level at 72 hours after contacting the cells with the indicated sgRNA and an effector comprising Cas9. Results are from experimental triplicates. The level of siRNA targeting APOB gene (siApoB) is relative to NTX control. In NTX control, cultured cells were transfected with the LNP only, *i.e*., the LNP formulation did not contain RNA.

Figures 4A and 4B depict relative APOB mRNA level. The ApoB mRNA levels were graphed versus the non-targeting guide RNA formulation (GD-23150 group). Figure 4A is a graph depicting within well results. Delta cycle threshold (dCT) values werewas calculated using the individual APOB result relative to actin within the sample's respective well. Figure 4B is a graph depicting across plate results. dCT value was calculated using the individual APOB results relative to the average of actin in all samples at each dilution point.

Figures 4C and 4D depict relative APOB mRNA levels as compared to NTC with normalized mRNA input. Figure 4C is a graph depicting within well result. dCT values werecalculated using the individual APOB results relative to actin within the sample's respective well. Figure 4D is a graph depicting across plate results. dCT value were calculated using the individual APOB result relative to the average of actin in all samples.

Figures 4E and 4F further confirm the downregulation of APOB expression with a site-specific APOB disrupting agent, comprising a site-specific APOB targeting moiety which targets an APOB expression control region, *i.e.,* a sgRNA, and an effector comprising a Cas9. In Figures 4E and 4F, 5 µg/ml of total RNA, *i.e.,* the combination of sgRNA and Cas9 coding mRNA, was transfected to the cells. In addition, a sgRNA, GD-27723, which targets an exon of the APOB gene, was used as a positive control, while a sgRNA, GD-23149, which targets a different, unrelated gene, was used as a negative control. GD-27723 and GD23149 have the sequences as follows:

| | |
|---|---|
| GD-27723: | TCAAGCTGGCCATTCCTGAAGTTTTAGAGCTAGAAATAGCAAGTTAAAATAAG GCTAGTCCGTTATCAACTTGAAAAAGTGGCACCGAGTCGGTGCTTTT |
| GD-23149: | TTACAGCCACGTCTACAGCAGTTTTAGAGCTAGAAATAGCAAGTTAAAATAAG GCTAGTCCGTTATCAACTTGAAAAAGTGGCACCGAGTCGGTGCTTTT |

As shown in Figures 4E and 4F, sgRNA targeting of the APOB expression control region, in combination with an effector comprising Cas9, specifically reduced the mRNA level of APOB. While the APOB specific sgRNA reduced the mRNA level of APOB in PMH cells, sgRNA targeting an unrelated gene failed to downregulate the expression of APOB.

### Example 4. Modulation of APOB Expression - Protein

This example describes repression of APOB expression, as measured by the reduction of APOB protein level, using a site-specific APOB disrupting agent, comprising a site-specific APOB targeting moiety which targets an APOB expression control region, *i.e.,* a sgRNA, and an effector comprising a Cas9, .

Cells were cultured and treated with a site-specific sgRNA and Cas9 coding mRNA as described in Example 1 with slight modification. Five ((5) µg/ml of total RNA, *i.e.,* the combination of sgRNA and Cas9 coding mRNA, were transfected into the cells. At 72 hours after the transfection, the supernatants from the cell cultures were collected and ELISA assays were performed to determine the concentration of APOB protein in the supernatants.

As shown in Figures 5A and 5B, sgRNA targeting an APOB expression control region in combination with Cas9 reduced the expression of the APOB gene in cultured PMHs as measured by a reduction in protein level. Figure 5A depicts the concentration of ApoB protein in the supernatant of cultured cells. Figure 5B depicts the percentage of reduction using a combination of the indicated sgRNA and Cas9 coding mRNA.

**Table 2. Site-Specific APOB Targeting Moieties - The first 20 nucleotides in each moiety below comprise the targeting portion of the moiety.**

| **Identifier** | **Nucleotide Sequence 5'-3'** |
|---|---|
| GD-26911 (unmodified) | GATGGGGCAGCATCTCCATCGTTTTAGAGCTAGAAATAGCAAGTTAAAATAA GGCTAGTCCGTTATCAACTTGAAAAAGTGGCACCGAGTCGGTGCTTTT |
| GD-26911 | mGs;mAs;mUs;rG;rG;rG;rG;rC;rA;rG;rC;rA;rU;rC;rU;rC;rC;rA;rU;rC;rG;rU;rU;rU;rU;r A;rG;mA;mG;mC;mU;mA;mG;mA;mA;mA;mU;mA;mG;rC;rA;rA;rG;rU;rU;rA;rA;rA;r A;rU;rA;rA;rG;rG;rC;rU;rA;rG;rU;rC;rC;rG;rU;rU;rA;rU;rC;mA;mA;mC;mU;mU;mG; mA;mA;mA;mA;mA;mG;mU;mG;mG;mC;mA;mC;mC;mG;mA;mG;mU;mC;mG;mG; mU;mG;mC;mU;mUs;mUs;mUs |
| GD-26912 (unmodified) | GGGGCAGCATCTCCATCTGGGTTTTAGAGCTAGAAATAGCAAGTTAAAATAA GGCTAGTCCGTTATCAACTTGAAAAAGTGGCACCGAGTCGGTGCTTTT |
| GD-26912 | mGs;mGs;mGs;rG;rC;rA;rG;rC;rA;rU;rC;rU;rC;rC;rA;rU;rC;rU;rG;rG;rG;rU;rU;rU;rU;r A;rG;mA;mG;mC;mU;mA;mG;mA;mA;mA;mU;mA;mG;rC;rA;rA;rG;rU;rU;rA;rA;rA;r A;rU;rA;rA;rG;rG;rC;rU;rA;rG;rU;rC;rC;rG;rU;rU;rA;rU;rC;mA;mA;mC;mU;mU;mG; mA;mA;mA;mA;mA;mG;mU;mG;mG;mC;mA;mC;mC;mG;mA;mG;mU;mC;mG;mG; mU;mG;mC;mU;mUs;mUs;mUs |
| GD-26913 (unmodified) | ACTGCCATATGGCCACCAGAGTTTTAGAGCTAGAAATAGCAAGTTAAAATAA GGCTAGTCCGTTATCAACTTGAAAAAGTGGCACCGAGTCGGTGCTTTT |
| GD-26913 | mAs;mCs;mUs;rG;rC;rC;rA;rU;rA;rU;rG;rG;rC;rC;rA;rC;rC;rA;rG;rA;rG;rU;rU;rU;rU;r A;rG;mA;mG;mC;mU;mA;mG;mA;mA;mA;mU;mA;mG;rC;rA;rA;rG;rU;rU;rA;rA;rA;r A;rU;rA;rA;rG;rG;rC;rU;rA;rG;rU;rC;rC;rG;rU;rU;rA;rU;rC;mA;mA;mC;mU;mU;mG; mA;mA;mA;mA;mA;mG;mU;mG;mG;mC;mA;mC;mC;mG;mA;mG;mU;mC;mG;mG; mU;mG;mC;mU;mUs;mUs;mUs |

**Table A. Abbreviations of nucleotide monomers used in nucleic acid sequence representation. It will be understood that these monomers, when present in an oligonucleotide, are mutually linked by 5'-3'-phosphodiester bonds.**

| **Abbreviation** | **Nucleotide(s)** |
|---|---|
| A | Adenosine-3'-phosphate |
| As | adenosine-3'-phosphorothioate |
| C | cytidine-3'-phosphate |
| Cs | cytidine-3'-phosphorothioate |
| G | guanosine-3'-phosphate |
| Gs | guanosine-3'-phosphorothioate |
| U | Uridine-3'-phosphate |
| Us | uridine -3'-phosphorothioate |
| N | any nucleotide, modified or unmodified |
| mA | 2'-O-methyladenosine-3'-phosphate |
| mAs | 2'-O-methyladenosine-3'- phosphorothioate |
| mC | 2'-O-methylcytidine-3'-phosphate |
| mCs | 2'-O-methylcytidine-3'- phosphorothioate |
| mG | 2'-O-methylguanosine-3'-phosphate |
| mGs | 2'-O-methylguanosine-3'- phosphorothioate |
| mU | 2'-O-methyluridine-3'-phosphate |
| mUs | 2'-O-methyluridine-3'-phosphorothioate |
| s | phosphorothioate linkage |
| r | ribonucleotide |

## Claims

1. A site-specific apolipoprotein B (APOB) disrupting agent comprising a site-specific APOB targeting moiety which targets a CCCTC-binding factor (CTCF) binding motif in an APOB expression control region, wherein the site-specific APOB targeting moiety comprises:
(a) a DNA binding domain, or fragment thereof, of a zinc finger (ZNF) polypeptide, or a transcription activator-like effector (TALE) polypeptide that specifically binds to the APOB expression control region; or
(b) a CRISPR associated protein (Cas) polypeptide.

2. The site-specific APOB disrupting agent of claim 1, wherein the site specific APOB targeting moiety comprises a DNA-binding domain, or fragment thereof, of a ZNF polypeptide that specifically binds to the APOB expression control region.

3. The site-specific APOB disrupting agent of claim 1, wherein the site specific APOB targeting moiety comprises a DNA-binding domain, or fragment thereof, of a TALE polypeptide that specifically binds to the APOB expression control region.

4. The site-specific APOB disrupting agent of claim 1, wherein the site specific APOB targeting moiety comprises:
(i) a CRISPR associated protein (Cas) polypeptide, optionally wherein the Cas polypeptide is an enzymatically inactive Cas polypeptide, and
(ii) a guide RNA, optionally wherein the guide RNA comprises a nucleotide sequence having at least 85% nucleotide identity to the entire nucleotide sequence of any of the nucleotide sequences in Table 2.

5. The site specific APOB disrupting agent of any of claims 2-3, wherein the APOB disrupting agent further comprises an effector molecule.

6. The site specific APOB disrupting agent of claim 5, wherein the APOB disrupting agent is a fusion protein comprising the site-specific APOB targeting moiety and the effector molecule.

7. The site specific APOB disrupting agent of claim 5 or 6, wherein the effector molecule is selected from the group consisting of a physical blocker, an epigenetic recruiter, and an epigenetic CpG modifier.

8. The site-specific APOB disrupting agent of claim 7, wherein the epigenetic recruiter is selected from the group consisting of a transcriptional enhancer and a transcriptional repressor.

9. The site-specific APOB disrupting agent of claim 7, wherein the CpG modifier is selected from the group consisting of a DNA methylase, a DNA demethylase, a histone modifying agent, and a histone deacetylase.

10. A nucleic acid encoding the APOB disrupting agent of any one of claims 1-9.

11. A vector comprising the nucleic acid encoding the APOB disrupting agent of any one of claims 1-9, optionally wherein the vector is a viral expression vector.

12. A pharmaceutical composition comprising the nucleic acid encoding the APOB disrupting agent of any one of claims 1-9, wherein the nucleic acid is formulated in a lipid nanoparticle.

13. The pharmaceutical composition of claim 12 for use in treating an APOB-associated disease selected from the group consisting of a hyperlipidemia, a hypercholesterolemia, high LDL cholesterol, low HDL cholesterol, hypertriglyceridemia, postprandial hypertriglyceridemia, insulin resistance not related to an immune response to insulin, type 2 diabetes, hypertension, endothelial cell dysfunction, heart disease, and atherosclerosis.

## Patentansprüche

1. Ortsspezifischer Wirkstoff zur Störung von Apolipoprotein B (APOB), der eine ortsspezifische APOB-Zieleinheit umfasst, die auf ein Bindungsmotiv des CCCTC-Bindungsfaktors (CTCF) in einer APOB-Expressionskontrollregion abzielt, wobei die ortsspezifische APOB-Zieleinheit Folgendes umfasst:
(a) eine DNA-Bindungsdomäne, oder ein Fragment davon, eines Zinkfinger-(ZNF)-Polypeptids oder eines Transkriptionsaktivator-ähnlichen Effektor-(TALE)-Polypeptids, das spezifisch an die APOB-Expressionskontrollregion bindet; oder
(b) ein CRISPR-assoziiertes Protein (Cas)-Polypeptid.

2. Ortsspezifischer Wirkstoff zur Störung von APOB nach Anspruch 1, wobei die ortsspezifische APOB-Zieleinheit eine DNA-Bindungsdomäne, oder ein Fragment davon, eines ZNF-Polypeptids umfasst, das spezifisch an die APOB-Expressionskontrollregion bindet.

3. Ortsspezifischer Wirkstoff zur Störung von APOB nach Anspruch 1, wobei die ortsspezifische APOB-Zieleinheit eine DNA-Bindungsdomäne, oder ein Fragment davon, eines TALE-Polypeptids umfasst, das spezifisch an die APOB-Expressionskontrollregion bindet.

4. Ortsspezifischer Wirkstoff zur Störung von APOB nach Anspruch 1, wobei die ortsspezifische APOB-Zieleinheit Folgendes umfasst:
(i) ein CRISPR-assoziiertes Protein (Cas)-Polypeptid, wobei das Cas-Polypeptid gegebenenfalls ein enzymatisch inaktives Cas-Polypeptid ist; und
(ii) eine Führungs-RNA, wobei die Führungs-RNA gegebenenfalls eine Nukleotidsequenz umfasst, die eine Nukleotididentität von mindestens 85 % mit der gesamten Nukleotidsequenz einer der in Tabelle 2 angegebenen Nukleotidsequenzen aufweist.

5. Ortsspezifischer Wirkstoff zur Störung von APOB nach einem der Ansprüche 2 bis 3, wobei der Wirkstoff zur Störung von APOB weiter ein Effektormolekül umfasst.

6. Ortsspezifischer Wirkstoff zur Störung von APOB nach Anspruch 5, wobei der Wirkstoff zur Störung von APOB ein Fusionsprotein ist, das die ortsspezifische APOB-Zieleinheit und das Effektormolekül umfasst.

7. Ortsspezifischer Wirkstoff zur Störung von APOB nach Anspruch 5 oder 6, wobei das Effektormolekül aus der Gruppe ausgewählt ist, die aus einem physikalischen Blocker, einem epigenetischen Rekrutierer und einem epigenetischen CpG-Modifikator besteht.

8. Ortsspezifischer Wirkstoff zur Störung von APOB nach Anspruch 7, wobei der epigenetische Rekrutierer aus der Gruppe ausgewählt ist, die aus einem Transkriptionsverstärker und einem Transkriptionsrepressor besteht.

9. Ortsspezifischer Wirkstoff zur Störung von APOB nach Anspruch 7, wobei der CpG-Modifikator aus der Gruppe ausgewählt ist, die aus einer DNA-Methylase, einer DNA-Demethylase, einem Histon-Modifikationsmittel und einer Histon-Deacetylase besteht.

10. Nukleinsäure, die für den Wirkstoff zur Störung von APOB nach einem der Ansprüche 1 bis 9 kodiert.

11. Vektor, der die Nukleinsäure umfasst, die für den Wirkstoff zur Störung von APOB nach einem der Ansprüche 1 bis 9 kodiert, gegebenenfalls wobei es sich bei dem Vektor um einen viralen Expressionsvektor handelt.

12. Pharmazeutische Zusammensetzung, die die Nukleinsäure umfasst, die für den Wirkstoff zur Störung von APOB nach einem der Ansprüche 1 bis 9 kodiert, wobei die Nukleinsäure in einem Lipidnanopartikel formuliert ist.

13. Pharmazeutische Zusammensetzung nach Anspruch 12 zur Verwendung bei der Behandlung einer APOB-assoziierten Erkrankung, ausgewählt aus der Gruppe bestehend aus Hyperlipidämie, Hypercholesterinämie, erhöhtem LDL-Cholesterinspiegel, verringertem HDL-Cholesterinspiegel, Hypertriglyceridämie, postprandialer Hypertriglyceridämie, Insulinresistenz, die nicht mit einer Immunreaktion auf Insulin zusammenhängt, Typ-2-Diabetes, Bluthochdruck, Endothelzelldysfunktion, Herzerkrankungen und Atherosklerose.

## Revendications

1. Agent perturbateur de l'apolipotrotéine B (APOB) spécifique au site comprenant un fragment de ciblage de l'APOB spécifique au site qui cible un motif de liaison de facteur de liaison à CCCTC (CTCF) dans une région de régulation de l'expression de l'APOB, dans lequel le fragment de ciblage de l'APOB spécifique au site comprend :
(a) un domaine de liaison à l'ADN, ou un fragment de celui-ci, d'un polypeptide à doigts de zinc (ZNF), ou d'un polypeptide effecteur de type activateur de transcription (TALE) qui se lie spécifiquement à la région de régulation de l'expression de l'APOB ; ou
(b) un polypeptide de la protéine associée à CRISPR (Cas).

2. Agent perturbateur de l'APOB spécifique au site selon la revendication 1, dans lequel le fragment de ciblage de l'APOB spécifique au site comprend un domaine de liaison à l'ADN, ou un fragment de celui-ci, d'un polypeptide ZNF qui se lie spécifiquement à la région de régulation de l'expression de l'APOB.

3. Agent perturbateur de l'APOB spécifique au site selon la revendication 1, dans lequel le fragment de ciblage de l'APOB spécifique au site comprend un domaine de liaison à l'ADN, ou un fragment de celui-ci, d'un polypeptide TALE qui se lie spécifiquement à la région de régulation de l'expression de l'APOB.

4. Agent perturbateur de l'APOB spécifique au site selon la revendication 1, dans lequel le fragment de ciblage de l'APOB spécifique au site comprend :
(i) un polypeptide de la protéine associée à CRISPR (Cas), dans lequel facultativement le polypeptide Cas est un polypeptide Cas enzymatiquement inactif ; et
(ii) un ARN guide, dans lequel facultativement l'ARN guide comprend une séquence nucléotidique présentant au moins 85 % d'identité de nucléotides avec la séquence nucléotidique entière de l'une quelconque des séquences nucléotidiques dans le tableau 2.

5. Agent perturbateur de l'APOB spécifique au site selon l'une quelconque des revendications 2-3, dans lequel l'agent perturbateur de l'APOB comprend en outre une molécule effectrice.

6. Agent perturbateur de l'APOB spécifique au site selon la revendication 5, dans lequel l'agent perturbateur de l'APOB est une protéine de fusion comprenant le fragment de ciblage de l'APOB spécifique au site et la molécule effectrice.

7. Agent perturbateur de l'APOB spécifique au site selon la revendication 5 ou 6, dans lequel la molécule effectrice est sélectionnée à partir du groupe constitué d'un inhibiteur physique, d'un recruteur épigénétique et d'un modificateur épigénétique de CpG.

8. Agent perturbateur de l'APOB spécifique au site selon la revendication 7, dans lequel le recruteur épigénétique est sélectionné à partir du groupe constitué d'un activateur de transcription et d'un répresseur de transcription.

9. Agent perturbateur de l'APOB spécifique au site selon la revendication 7, dans lequel le modificateur de CpG est sélectionné à partir du groupe constitué d'une méthylase d'ADN, d'une déméthylase d'ADN, d'un agent de modification des histones et d'une histone désacétylase.

10. Acide nucléique codant pour l'agent perturbateur de l'APOB selon l'une quelconque des revendications 1-9.

11. Vecteur comprenant l'acide nucléique codant pour l'agent perturbateur de l'APOB selon l'une quelconque des revendications 1-9, dans lequel facultativement le vecteur est un vecteur d'expression viral.

12. Composition pharmaceutique comprenant l'acide nucléique codant pour l'agent perturbateur de l'APOB selon l'une quelconque des revendications 1-9, dans laquelle l'acide nucléique est formulé dans une nanoparticule lipidique.

13. Composition pharmaceutique selon la revendication 12 destinée à une utilisation dans le traitement d'une maladie associée à l'APOB sélectionnée à partir du groupe constitué d'une hyperlipidémie, d'une hypercholestérolémie, d'un cholestérol LDL élevé, d'un cholestérol HDL bas, d'une hypertriglycéridémie, d'une hypertriglycéridémie postprandiale, d'une résistance à l'insuline non liée à une réponse immunitaire à l'insuline, d'un diabète de type 2, d'une hypertension, d'un dysfonctionnement des cellules endothéliales, d'une maladie cardiaque et d'une athérosclérose.
